# DEMANDE DE BREVET EUROPEEN

(11) **EP 3 838 891 A1**
(43) Date de publication de la demande: **23.06.2021**
(21) Numéro de dépôt: 19306694.1
(22) Date de dépôt: 19.12.2019
(51) Int. Cl.: C07C 251/08, C08G 18/32, C09D 175/04

(54) **ALDIMINES ET LEURS UTILISATIONS**

(71) Demandeur: BOSTIK SA, 92700 Colombes (FR)
(72) Inventeur: FOUQUAY, Stéphane, 60280 VENETTE (FR); SANZ, Federico, 60280 VENETTE (FR)
(74) Mandataire: Arkema Patent

(57) **Abrégé**

La présente invention concerne des composé ayant l'une des formules (I) ou (II) suivantes : 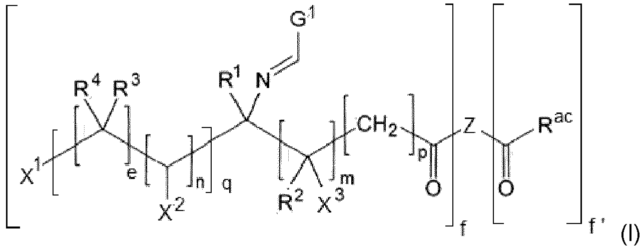 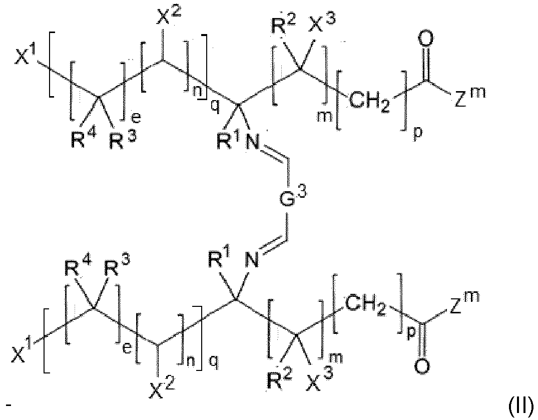 ainsi que leurs utilisations.

## Description

### DOMAINE DE L'INVENTION

La présente invention concerne une nouvelle famille de (poly)aldimines, ainsi que les compositions les comprenant.

La présente invention concerne également l'utilisation de ces (poly)aldimines dans des compositions adhésives, des mastics ou des revêtements.

### ARRIERE-PLAN TECHNIQUE

Les (poly)aldimines sont des produits de condensation d'amines primaires et d'aldéhydes, et constituent une classe de composés bien connue. Par contact avec l'eau, les (poly)aldimines peuvent s'hydrolyser en amines et aldéhydes correspondants. En raison de leurs propriétés, les aldimines peuvent également être utilisées comme une forme protégée d'amines.

Les aldimines sont notamment utiles dans les compositions adhésives à base de polyuréthanes réticulables à l'humidité, notamment dans les mastics ainsi que dans les compositions adhésives à base d'époxydes. Leur utilisation en tant que durcisseur latent activables à l'humidité dans des systèmes à base de polyuréthane permet avantageusement d'éviter la formation de bulles, dans la mesure où la réticulation avec les aldimines (amines bloquées) ne conduit pas à la libération de CO₂ (au contraire de la réticulation directe des groupes isocyanates avec l'humidité).

Il existe un besoin pour de nouvelles (poly)aldimines.

### DESCRIPTION DE L'INVENTION

### A - Aldimines de formule (I) ou (II)

La présente invention concerne un composé ayant l'une des formules (I) ou (II) suivantes : 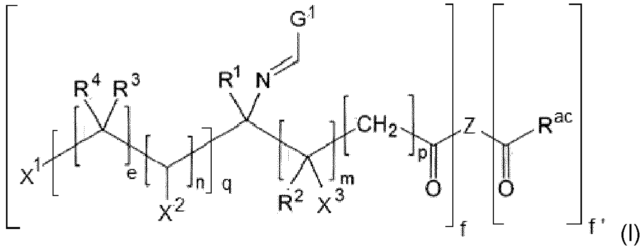 dans laquelle:
- X¹ représente F¹ ou -N=C(H)-G¹ ou -Ph-XH, ou -XH, avec X représentant O ou S ou Se et Ph représentant un groupe phényle;
- X² représente F² ou -N=C(H)-G¹ ou -Ph-XH, ou -XH, avec X représentant O ou S ou Se et Ph représentant un groupe phényle;
- X³ représente F³ ou -N=C(H)-G¹ ou -Ph-XH, ou -XH, avec X représentant O ou S ou Se et Ph représentant un groupe phényle;
   à condition qu'au maximum un seul radical parmi les radicaux X¹, X² et X³ représente -N=C(H)-G¹ ;
   à condition qu'au maximum un seul radical parmi les radicaux X¹, X² et X³ représente -XH ou -Ph-XH;
   à condition que quand X¹ = -N=C(H)-G¹ alors q = 1 ;
   à condition que quand X¹ = -N=C(H)-G¹ ou -Ph-XH ou -XH, alors X² = F² et X³ = F³ ;
   à condition que quand X² = -N=C(H)-G¹ ou -Ph-XH ou -XH, alors X¹ = F¹ et X³ = F³ ;
   à condition que quand X³ = -N=C(H)-G¹ ou -Ph-XH ou -XH, alors X² = F² et X¹ = F¹ ;
- F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- R¹, R², R³ et R⁴ représente chacun, indépendamment les uns des autres, un radical choisi parmi un atome d'hydrogène, un groupe alkyle linéaire ou ramifié, comprenant de 1 à 20 atomes de carbone, ledit groupe alkyle comprenant éventuellement un ou plusieurs hétéroatomes, un groupe (hétéro)cycloalkyle comprenant de 3 à 20 atomes de carbone, un groupe arylalkyle comprenant de 7 à 20 atomes de carbone ou un groupe (hétéro)aryle comprenant de 4 à 12 atomes de carbone ;
- n est un nombre entier allant de 0 à 28 ;
- p est un nombre entier allant de 0 à 10 ;
- m représente 0 ou 1 ;
- q représente 0 ou 1 ;
- e est un nombre entier allant de 1 à 3 ;
- f est un nombre entier ou non entier allant de 1 à 3, f étant de préférence un nombre entier égal à 1, 2 ou 3 ;
- f' est un nombre entier ou non entier allant de 0 à 2, f' étant de préférence un nombre entier égal à 0, 1 ou 2 ;
- la somme f + f' représente un entier variant de 1 à 3 ;
- Z représente un radical -organique monovalent Z^{m}, divalent Z^{d} ou trivalent Z^{t}, ayant une masse molaire ou une masse moléculaire moyenne en nombre (Mn) allant de 16 à 22 000 g/mol, de préférence de 16 à 12 000 g/mol, de préférence encore de 16 à 8 000 g/mol, encore plus préférentiellement de 16 à 4 000 g/mol ;
- R^{ac} représente un atome d'hydrogène ou un radical hydrocarboné monovalent comprenant de 1 à 60 atomes de carbone, ledit radical pouvant éventuellement comprendre un ou plusieurs hétéroatomes ;
- G¹ représente un radical hydrocarboné monovalent de masse molaire ou de masse moléculaire moyenne en nombre (Mn) allant de 15 à 4000 g/mol, de préférence de 60 à 2 000 g/mol, préférentiellement de 60 à 1 000 g/mol et encore plus préférentiellement de 60 à 500 g/mol, ledit radical pouvant éventuellement comprendre un ou plusieurs hétéroatomes, à condition que G¹ ne représente pas un radical aryle substitué ou non substitué ; 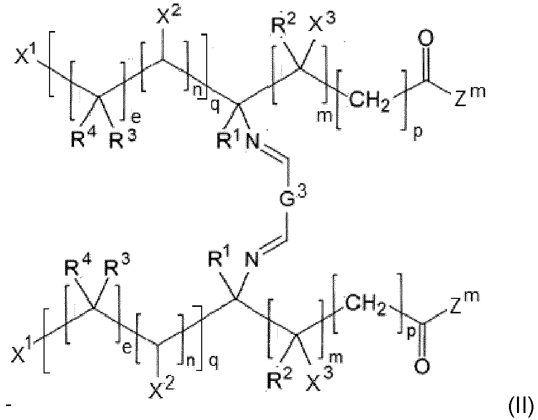
dans laquelle :
- X¹ représente F¹ ou -Ph-XH, ou -XH;
- X² représente F² ou -Ph-XH, ou -XH;
- X³ représente F³ ou -Ph-XH, ou -XH;
- F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- X, R¹, R², R³, R⁴, n, q, m, p, e et Z^{m} étant tels que définis pour la formule (I) ci-dessus ;
- G³ représente un radical hydrocarboné monovalent de masse molaire ou de masse moléculaire moyenne en nombre (Mn) allant de 14 à 4000 g/mol, de préférence de 42 à 2 000 g/mol, préférentiellement de 42 à 1 000 g/mol et encore plus préférentiellement de 42 à 500 g/mol, ledit radical pouvant éventuellement comprendre un ou plusieurs hétéroatomes, à condition que G³ ne représente pas un radical arylène substitué ou non;
à condition qu'au maximum un seul radical parmi les radicaux X¹, X² et X³ représente -XH ou -Ph-XH;
à condition que quand X¹ = -Ph-XH ou -XH, alors X² = F² et X³ = F³ ;
à condition que quand X² = -Ph-XH ou -XH, alors X¹ = F¹ et X³ = F³ ;
à condition que quand X³ = -Ph-XH ou -XH, alors X² = F² et X¹ = F¹.

Dans la présente demande, la condition selon laquelle « au maximum un seul radical parmi les radicaux X¹, X² et X³ représente -N=C(H)-G¹ » signifie que soit aucun des radicaux X¹, X² et X³ ne représente -N=C(H)-G¹, soit un seul radical parmi les radicaux X¹, X² et X³ représente -N=C(H)-G¹.

Dans la présente demande, la condition selon laquelle « au maximum un seul radical parmi les radicaux X¹, X² et X³ représente -XH ou -Ph-XH» signifie que soit aucun des radicaux X¹, X² et X³ ne représente -XH ou -Ph-XH, soit un seul radical parmi les radicaux X¹, X² et X³ représente -XH ou -Ph-XH.

Dans le cadre de l'invention, on entend par « -Ph-XH », un groupe phényle substitué par un groupe -XH, avec X représentant O ou S ou Se. Le groupement -XH se trouve avantageusement en para.

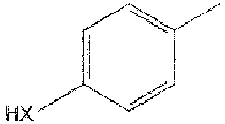

Dans le cadre de l'invention, on entend par « arylalkyle », un groupe alkyle substitué par un groupe aryle, le groupe arylalkyle comprenant de 7 à 20 atomes de carbone. A titre de groupe arylalkyle, on peut par exemple citer le benzyle.

Dans le cadre de l'invention, on entend par « (hétéro)aryle », un groupe hétéroaryle ou un groupe aryle, ledit groupe (hétéro)aryle comprenant de 4 à 12 atomes de carbone.

Dans le cadre de l'invention, on entend par « aryle », un radical aromatique monocyclique ou bicyclique comprenant de 6 à 12 atomes de carbone. On peut par exemple citer le phényle.

Dans le cadre de l'invention, on entend par « hétéroaryle », un radical aromatique monocyclique ou bicyclique comprenant au moins un hétéroatome tel que par exemple O, S ou N, et de 4 à 12 atomes de carbone. On peut par exemple citer les radicaux furanyle, thiophényle, pyrrolyle, pyridinyle, indolyle ou imidazolyle.

Dans le cadre de l'invention, on entend par « (hétéro)cycloalkyle », un groupe hétérocycloalkyle ou un groupe cycloalkyle.

Dans le cadre de l'invention, on entend par « cycloalkyle », un système monocyclique ou polycyclique, de préférence mono ou bicyclique, saturé, comportant de 3 à 12 atomes de carbone, les cycles pouvant être deux à deux fusionnés ou pontés, tel que les groupes cyclopropyle, cyclopentyle, cyclohexyle ou encore norbornyle.

Dans le cadre de l'invention, on entend par « hétérocycloalkyle », un système monocyclique ou polycyclique, de préférence mono ou bicyclique, saturé, comportant de 3 à 12 atomes de carbone et au moins un hétéroatome tel que par exemple O ou N, les cycles pouvant être deux à deux fusionnés ou pontés.

Dans la présente demande, les radicaux alkyle, arylalkyle, (hétéro)aryle, (hétéro)cycloalkyle peuvent être éventuellement substitués.

Dans le cadre de l'invention, l'expression « radical hydrocarboné pouvant contenir un ou plusieurs hétéroatomes » signifie que le radical peut contenir un hétéroatome soit sous la forme d'une fonction telle que par exemple une fonction carbonyle -C(=O), une fonction amide -C(=O)-NH₂- ou ester -C(=O)-O- / -O-C(=O)-, ou encore -O-C(=O)-NH-, -OR ou - N(R')(R"), ou alors sous forme d'un hétéroatome tel que par exemple -O-, -S- ou -NH-.

Dans le cadre de l'invention, un « alkyle pouvant éventuellement comprendre un ou plusieurs hétéroatomes » est un groupe alkyle pouvant éventuellement comprendre un hétéroatome choisi parmi -O-, S ou -NH-.

Dans la formule (I) ci-dessus, lorsque f' = 0 et f = 1, alors Z est un radical monovalent Z^{m}.

Dans la formule (I) ci-dessus, lorsque f' = 0 et f = 2, alors Z est un radical divalent Z^{d}.

Dans la formule (I) ci-dessus, lorsque f' = 0 et f = 3, alors Z est un radical trivalent Z^{t}.

Dans la formule (I) ci-dessus, lorsque f' = 1 et f = 1, alors Z est un radical divalent Z^{d}.

Dans la formule (I) ci-dessus, lorsque f' = 2 et f =1, alors Z est un radical trivalent Z^{t}.

Dans la formule (I) ci-dessus, lorsque f + f' = 1, alors Z est un radical monovalent Z^{m}.

Dans la formule (I) ci-dessus, lorsque f + f' = 2, alors Z est un radical divalent Z^{d}.

Dans la formule (I) ci-dessus, lorsque f + f' = 3, alors Z est un radical trivalent Z^{t}.

Selon un mode de réalisation, Z représente un radical -organique monovalent Z^{m}, divalent Z^{d} ou trivalent Z^{t}, ayant une masse molaire ou une masse moléculaire moyenne en nombre (Mn) allant de 16 à 2 000 g/mol, préférentiellement de 16 à 1 000 g/mol, et encore plus préférentiellement de 30 à 500 g/mol.

Selon un mode de réalisation, dans l'une quelconque des formules (I) ou (II), Z^{m} représente un radical choisi parmi -OR⁵, -NH₂, -NH-R' ou -N(R')(R") dans lesquels :
- R⁵ représente un groupe alkyle linéaire ou ramifié comprenant de 1 à 1 000 atomes de carbone pouvant éventuellement comprendre un ou plusieurs hétéroatomes, un groupe (hétéro)cycloalkyle comprenant de 3 à 20 atomes de carbone, un groupe arylalkyle comprenant de 7 à 20 atomes de carbone, ou un groupe (hétéro)aryle comprenant de 4 à 12 atomes de carbone;
- R' représente un groupe alkyle linéaire ou ramifié, comprenant de 1 à 300 atomes de carbone pouvant éventuellement comprendre un ou plusieurs hétéroatomes, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone ;
- R" représente un groupe alkyle linéaire ou ramifié, comprenant de 1 à 300 atomes de carbone pouvant éventuellement comprendre un ou plusieurs hétéroatomes, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone.

De préférence, dans l'une quelconque des formules (I) ou (II), Z^{m} représente un radical choisi parmi -OR⁵, -NH₂, -NH-R' ou -N(R')(R") dans lesquels :
- R⁵ représente un groupe alkyle linéaire ou ramifié comprenant de 1 à 60 atomes de carbone pouvant éventuellement comprendre un ou plusieurs hétéroatomes, un groupe (hétéro)cycloalkyle comprenant de 3 à 20 atomes de carbone, un groupe arylalkyle comprenant de 7 à 20 atomes de carbone, ou un groupe (hétéro)aryle comprenant de 4 à 12 atomes de carbone;
- R' représente un groupe alkyle linéaire ou ramifié, comprenant de 1 à 60 atomes de carbone pouvant éventuellement comprendre un ou plusieurs hétéroatomes, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone ;
- R" représente un groupe alkyle linéaire ou ramifié, comprenant de 1 à 60 atomes de carbone pouvant éventuellement comprendre un ou plusieurs hétéroatomes, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone.

Le radical -G¹ peut représenter un radical -C(R⁶)(R⁷)(R⁸) ou un radical -G² avec :
- R⁶ et R⁷ représentant chacun, indépendamment l'un de l'autre, un radical hydrocarboné monovalent comprenant de 1 à 12 atomes de carbone, ledit radical étant éventuellement substitué par un groupe -OH ;
   ou R⁶ et R⁷ forment ensemble un cycle aliphatique comprenant de 4 à 12 atomes de carbone, ledit cycle étant éventuellement substitué ;
- R⁸ représente un radical hydrocarboné monovalent comprenant de 1 à 60 atomes de carbone, ledit radical comprenant éventuellement un hétéroatome ;
- G² représente un radical hétéroaryle éventuellement substitué, ou un radical -C(O)-R¹² avec R¹² représentant un radical alcoxy, un radical alkényle, ou un radical arylalkényle comprenant au moins 6 atomes de carbone,
   ledit radical G² ayant une masse molaire ou une masse moléculaire moyenne en nombre (Mn) allant de 15 à 4 000 g/mol, de préférence allant de 60 à 2 000 g/mol, préférentiellement allant de 60 à 1 000 g/mol et encore plus préférentiellement allant de 60 à 500 g/mol.

Le radical -G³ peut représenter :
- un radical alkylène linéaire ou ramifié, cyclique ou non, saturé ou insaturé, ou
- un radical -G⁵-(G⁶-G⁵)ᵣ-,
   de masse molaire ou de masse moléculaire moyenne en nombre (Mn) allant de 14 à 4 000 g/mol, de préférence allant de 60 à 2 000 g/mol, préférentiellement allant de 60 à 1 000 g/mol et encore plus préférentiellement allant de 60 à 500 g/mol, dans lequel :
   - G⁵ représente un radical (hétéro)arylène éventuellement substitué ;
   - G⁶ représente un atome d'oxygène, un atome de soufre ou un radical choisi parmi l'un des radicaux suivants : -O-R²⁷-O-, -CH₂-O-R²⁸-O-CH₂-, -CH₂-O-C(=O)-R²⁹-C(=O)-O-CH₂-, -CH₂-O-C(=O)-NH-R³⁰-NH-C(=O)-O-CH₂-, -O-C(=O)-NH-R³¹-NH-C(=O)-O-, -O-C(=O)-R³²-C(=O)-O-, et avec R²⁷, R²⁸, R²⁹, R³⁰, R³¹ et R³² représentant indépendamment les uns des autres un radical hydrocarboné comprenant éventuellement au moins un hétéroatome ;
   - r représente 0 ou 1 ;
   - à condition que quand r = 0, alors G⁵ représente un hétéroarylène éventuellement substitué ;
- un radical -CH(R²⁵)(R²⁶)-[G⁴-CH(R²⁵)(R²⁶)]_{w}-, de masse molaire ou de masse moléculaire moyenne en nombre (Mn) allant de 42 à 4 000 g/mol, de préférence allant de 42 à 2 000 g/mol, préférentiellement allant de 42 à 1 000 g/mol et encore plus préférentiellement allant de 42 à 500 g/mol, dans lequel:
   - R²⁵ et R²⁶ représente chacun, indépendamment l'un de l'autre, un radical hydrocarboné monovalent comprenant de 1 à 12 atomes de carbone, ledit radical étant éventuellement substitué par un groupe -OH ;
      ou R²⁵ et R²⁶ forment ensemble un cycle aliphatique comprenant de 4 à 12 atomes de carbone, ledit cycle étant éventuellement substitué ;
   - G⁴ représente une liaison carbone-carbone ou un radical hydrocarboné divalent, ledit radical comprenant éventuellement au moins un hétéroatome ;
   - w représente un nombre entier égal à 0 ou 1.

Les modes préférés pour G¹, R⁶, R⁷, R⁸ et G² sont notamment ceux décrits ci-après pour les composés de formule (IV).

Les modes préférés pour G³ sont notamment ceux décrits ci-après pour les composés de formule (VIII).

De préférence, les composés de formule (I) et (II), sont ceux pour lesquels :
- n représente 0, 1, 2, 3, 4, 9 ou 28 ;
- m représente 0 ou 1 ;
- p représente 0 ou 1 ou 9 ou 10;
- q représente 0 ou 1 ;
- F¹ représente un radical choisi parmi un atome d'hydrogène, un groupe (hétéro)aryle comprenant de 4 à 12 atomes de carbone, un groupe -COOZ (formule (I) avec Z étant tel que défini précédemment dans la formules (I), ou respectivement -COOZ^{m} avec Z^{m} étant tel que défini dans la formule (II)), un groupe -C(O)NH₂, un groupe -SMe, ou un groupe guanidyl de formule -NH-C(=NH)-NH₂,
- F² représente un radical choisi parmi un atome d'hydrogène ou un groupe alkyle linéaire ou ramifié comprenant de 1 à 10 atomes de carbone,
- F³ représente un radical choisi parmi un atome d'hydrogène ou un groupe alkyle linéaire ou ramifié comprenant de 1 à 10 atomes de carbone ;
- R¹, R², R³ et R⁴ représente chacun, indépendamment les uns des autres, un atome d'hydrogène ou un groupe alkyle linéaire ou ramifié comprenant de 1 à 10 atomes de carbone, encore plus préférentiellement de 1 à 5 atomes de carbone, ou un groupe arylalkyle comprenant de 7 à 20 atomes de carbone, ou un groupe aryle comprenant de 6 à 12 atomes de carbone.

De façon encore plus préférée, les composés de formule (I) et (II), sont ceux pour lesquels :
- n représente 0, 1, 2, 3 ou 4 ;
- m représente 0 ou 1 ;
- p représente 0 ou 1;
- q représente 0 ou 1 ;
- F¹ représente un radical choisi parmi un atome d'hydrogène, un groupe hétéroaryle comprenant de 4 à 12 atomes de carbone, un groupe -COOZ (Z étant tel que défini précédemment dans la formule (I)), un groupe -C(O)NH₂, un groupe -SMe, ou un groupe guanidyl de formule -NH-C(=NH)-NH₂,
- F² représente un radical choisi parmi un atome d'hydrogène ou un groupe alkyle linéaire ou ramifié comprenant de 1 à 5 atomes de carbone,
- F³ représente un radical choisi parmi un atome d'hydrogène;
- R¹, R², R³ et R⁴ représente chacun, indépendamment les uns des autres, un atome d'hydrogène ou un groupe alkyle linéaire ou ramifié comprenant de 1 à 5 atomes de carbone, ou un groupe benzyle, ou un groupe phényle.

De préférence, dans les composés de formule (I) :
- f représente 1 ou 2 ; et
- f' représente 0 (zéro).

Les composés de formule (I) peuvent avoir l'une des formules (I-A), (I-B), (I-C) ou (I-D) suivantes : 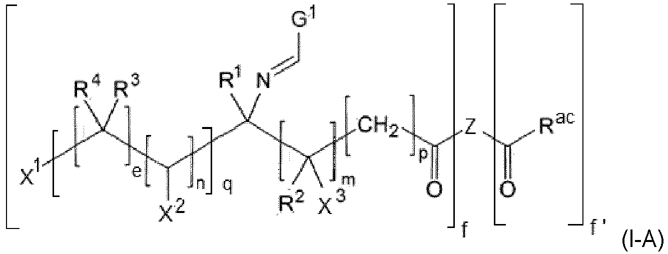 dans laquelle :
- R¹, R², R³, R⁴, n, m, p, q, e, f, f', Z, G¹ et R^{ac} sont tels que définis précédemment dans la formule (I),
- X¹ représente F¹ tel que défini précédemment ou -Ph-XH, ou -XH avec X représentant O ou S ou Se et Ph représentant un groupe phényle;
- X² représente F² tel que défini précédemment ou -Ph-XH, ou -XH avec X représentant O ou S ou Se et Ph représentant un groupe phényle;
- X³ représente F³ tel que défini précédemment ou -Ph-XH, ou -XH avec X représentant O ou S ou Se et Ph représentant un groupe phényle;
   à condition qu'au plus un des radicaux X¹, X² ou X³ représente un radical -Ph-XH ou -XH avec X représentant O ou S ou Se et Ph représentant un radical phényle ; 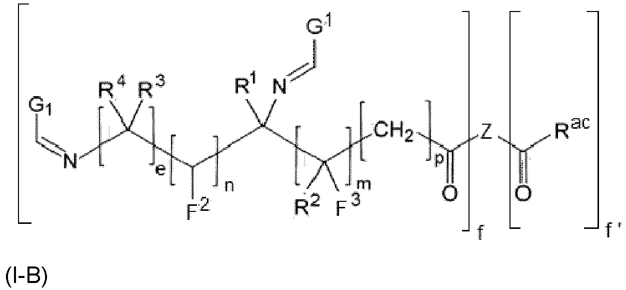 dans laquelle F², F³, R¹, R², R³, R⁴, n, m, p, e, f, f', Z, G¹ et R^{ac} sont tels que définis précédemment pour la formule (I) ; 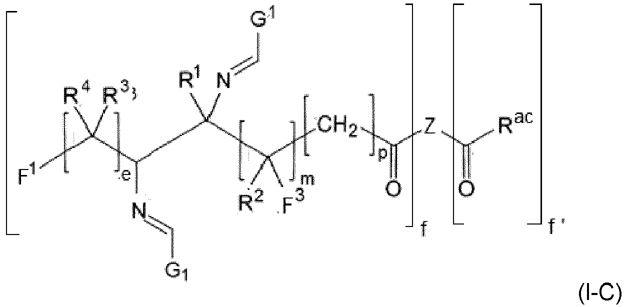 dans laquelle F¹, F³, R¹, R², R³, R⁴, F², m, p, q, e, f, f', G¹, Z et R^{ac} sont tels que définis précédemment pour la formule (I) ; 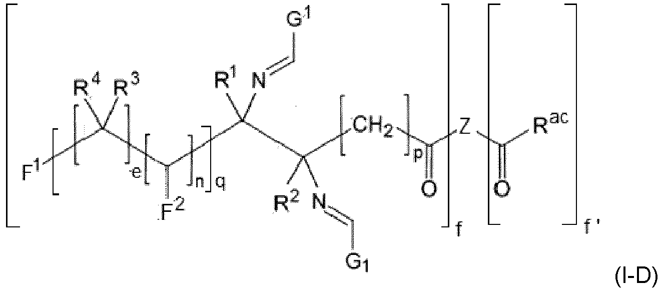
dans laquelle F¹, F², R¹, R², R³, R⁴, F², n, m, p, q, e, f, f', G¹, Z et R^{ac} sont tels que définis précédemment pour la formule (I).

De façon encore plus préférée, les composés de formule (I) sont des composés de formule (I-A) susmentionnée.

### Aldimines de formule (I-A)

De préférence, les composés de formule (I-A) sont ceux dans laquelle :
- n représente 0, 1, 2, 3 ou 4 ;
- m représente 0 ou 1 ;
- p représente 0 ou 1 ;
- q représente 0 ou 1 ;
- e est un nombre entier allant de 1 à 3 ;
- F¹ représente un radical choisi parmi un atome d'hydrogène, un groupe (hétéro)aryle comprenant de 4 à 12 atomes de carbone, un groupe -COOZ, un groupe -C(O)NH₂, un groupe -SMe, ou un groupe guanidyl de formule -NH-C(=NH)-NH₂,
- F² représente un radical choisi parmi un atome d'hydrogène ou un groupe alkyle linéaire ou ramifié comprenant de 1 à 10 atomes de carbone,
- F³ représente un radical choisi parmi un atome d'hydrogène;
- R¹, R², R³ et R⁴ représente chacun, indépendamment les uns des autres, un atome d'hydrogène ou un groupe alkyle linéaire ou ramifié comprenant de 1 à 10 atomes de carbone, encore plus préférentiellement de 1 à 5 atomes de carbone ou un groupe benzyle, ou un groupe phényle.

Les composés de formule (I-A) peuvent avoir l'une des formules (I-A-1), (I-A-2) ou (I-A-3) suivantes : 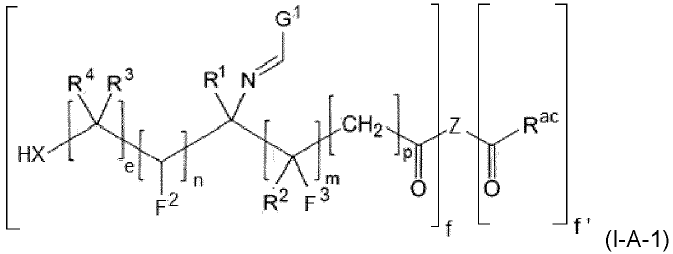 dans laquelle X, R¹, R², R³, R⁴, n, m, p, e, f, f', Z, F², F³, G¹ et R^{ac} sont tels que définis précédemment dans la formule (I-A) ; 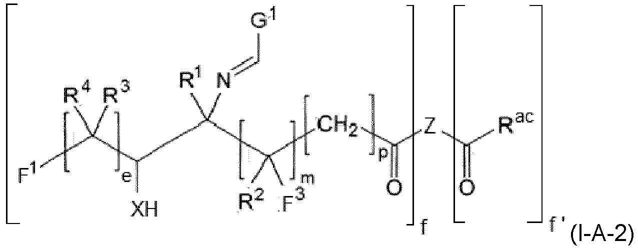 dans laquelle R¹, R², R³, R⁴, m, p, e, f, f', Z, X, G¹, F¹, F³, G¹ et R^{ac} sont tels que définis précédemment dans la formule (I-A), 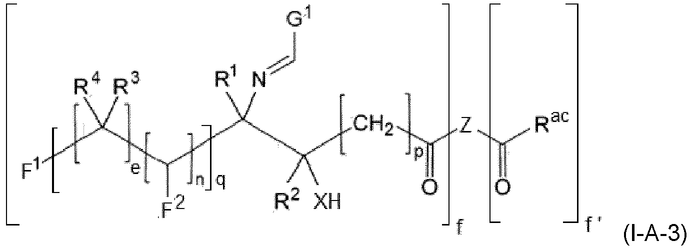 dans laquelle R¹, R², R³, R⁴, n, p, q, e, f, f', Z, X, G¹, F¹, F², R^{ac} sont tels que définis précédemment dans la formule (I-A).

De préférence, dans les composés de formule (I-A), (I-A-1), (I-A-2) et (I-A-3) :
- f représente 1 ou 2, et
- f' représente 0 (zéro).

### Aldimides de formule (I-B)

De préférence, les composés de formule (I-B) sont ceux dans laquelle :
- n représente 0, 3, ou 4 ;
- m représente 0 ;
- p représente 0 ;
- e est un nombre entier allant de 1 à 3 ;
- F² représente un atome d'hydrogène ;
- F³ représente un radical choisi parmi un atome d'hydrogène ou un groupe alkyle linéaire ou ramifié comprenant de 1 à 10 atomes de carbone ;
- R¹, R², R³ et R⁴ représente chacun, indépendamment les uns des autres, un atome d'hydrogène ou un groupe alkyle linéaire ou ramifié comprenant de 1 à 10 atomes de carbone, encore plus préférentiellement de 1 à 5 atomes de carbone.

Les composés de formule (I-B) peuvent avoir l'une des formules (I-B-1), (I-B-2) ou (I-B-3) suivantes : 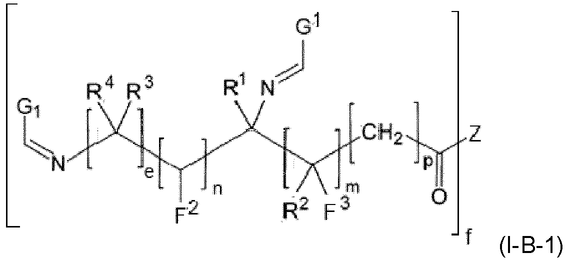 dans laquelle F², F³, R¹, R², R³, R⁴, n, m, p, e, f, G¹ et Z sont tels que définis précédemment dans la formule (I-B), 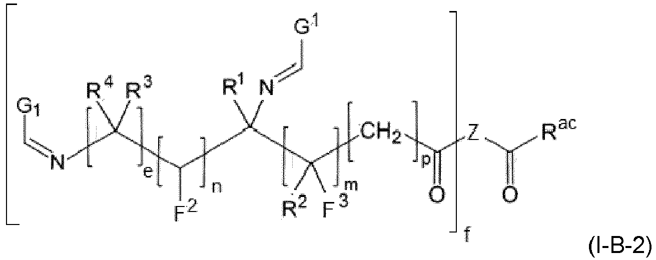 dans laquelle F², F³, R¹, R², R³, R⁴, n, m, p, e, f, G¹, Z et R^{ac} sont tels que définis précédemment dans la formule (I-B), 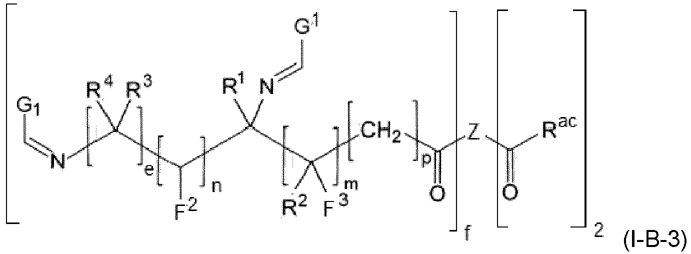 dans laquelle F², F³, R¹, R², R³, R⁴, n, m, p, e, f, G¹, Z et R^{ac} sont tels que définis précédemment dans la formule (I-B).

### Aldimides de formule (I-C)

De préférence, les composés de formule (I-C) sont ceux dans laquelle :
- m représente 0 ;
- p représente 0 ;
- e est un nombre entier allant de 1 à 3 ;
- F¹ représente un radical choisi parmi un atome d'hydrogène ou un groupe alkyle linéaire ou ramifié comprenant de 1 à 10 atomes de carbone,
- F³ représente un radical choisi parmi un atome d'hydrogène ou un groupe alkyle linéaire ou ramifié comprenant de 1 à 10 atomes de carbone ;
- R¹, R², R³ et R⁴ représente chacun, indépendamment les uns des autres, un atome d'hydrogène ou un groupe alkyle linéaire ou ramifié comprenant de 1 à 10 atomes de carbone, encore plus préférentiellement de 1 à 5 atomes de carbone.

Les composés de formule (I-C) peuvent avoir l'une des formules (I-C-1), (I-C-2) ou (I-C-3) suivantes : 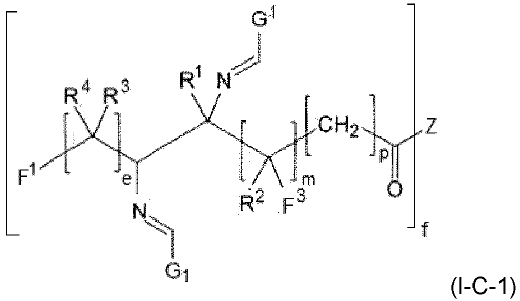 dans laquelle F¹, F³, R¹, R², R³, R⁴, F², m, p, q, e, f, G¹ et Z sont tels que définis précédemment dans la formule (I-C), 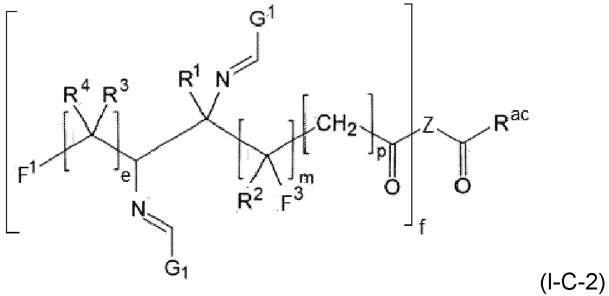 dans laquelle F¹, F³, R¹, R², R³, R⁴, F², m, p, q, e, f, G¹, Z et R^{ac} sont tels que définis précédemment dans la formule (I-C), 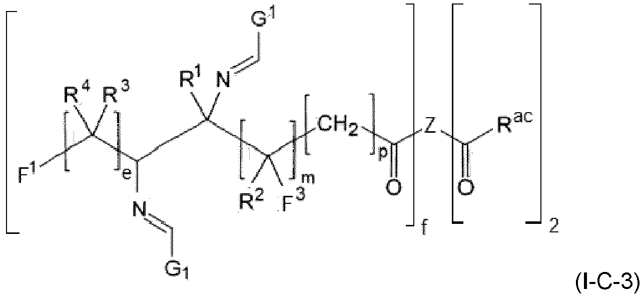 dans laquelle F¹, F³, R¹, R², R³, R⁴, F², m, p, q, e, f, G¹, Z et R^{ac} sont tels que définis précédemment dans la formule (I-C).

### Aldimides de formule (I-D)

De préférence, les composés de formule (I-D) sont ceux dans laquelle :
- n représente 0 ou 3 ;
- p représente 0 ;
- q représente 0 ou 1 ;
- e est un nombre entier allant de 1 à 3 ;
- F¹ représente un radical choisi parmi un atome d'hydrogène ou un groupe alkyle linéaire ou ramifié comprenant de 1 à 10 atomes de carbone,
- F³ représente un radical choisi parmi un atome d'hydrogène ou un groupe alkyle linéaire ou ramifié comprenant de 1 à 10 atomes de carbone ;
- R¹, R², R³ et R⁴ représente chacun, indépendamment les uns des autres, un atome d'hydrogène ou un groupe alkyle linéaire ou ramifié comprenant de 1 à 10 atomes de carbone, encore plus préférentiellement de 1 à 5 atomes de carbone.

Les composés de formule (I-D) peuvent avoir l'une des formules (I-D-1), (I-D-2) ou (I-D-3) suivantes : 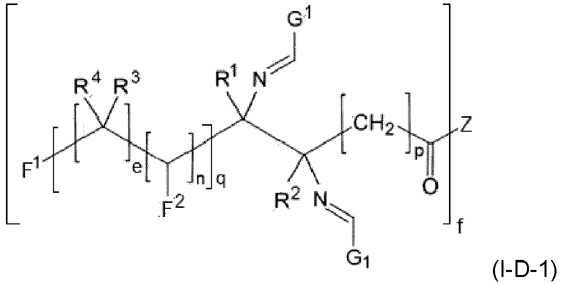 dans laquelle F¹, F², R¹, R², R³, R⁴, F², n, m, p, q, e, f, G¹ et Z sont tels que définis précédemment dans la formule (I-D) ; 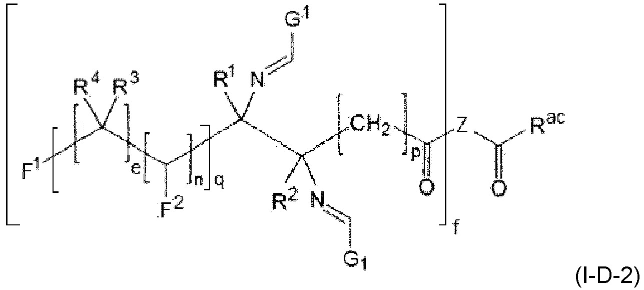 dans laquelle F¹, F², R¹, R², R³, R⁴, F², n, m, p, q, e, f, G¹, Z et R^{ac} sont tels que définis précédemment dans la formule (I-D), 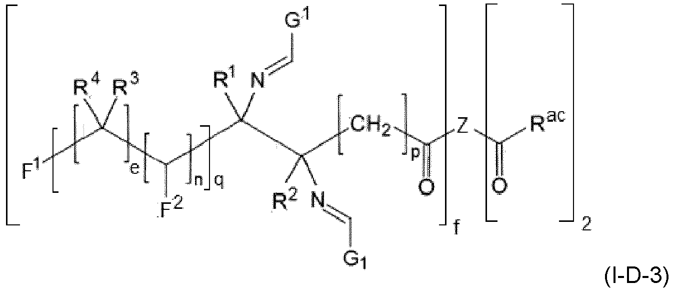 dans laquelle F¹, F², R¹, R², R³, R⁴, F², n, m, p, q, e, f, G¹, Z et R^{ac} sont tels que définis précédemment dans la formule (I-D).

### B. Précurseurs aldimines de formule (III) et (III')

### B.1. Précurseurs aldimines de formule (III)

Les composés de formule (I) susmentionnée peuvent être obtenus par réaction entre :
- un composé de formule (III) suivante : 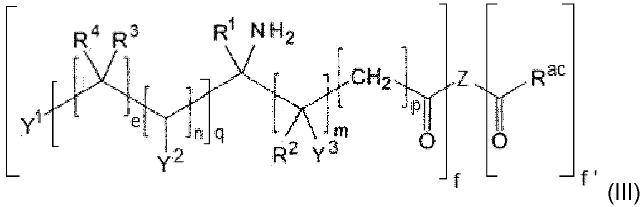 dans laquelle :
   - Y¹ représente F¹ ou -NH₂ ou -Ph-XH ou -XH avec X représentant O ou S ou Se et Ph représentant un groupe phényle;
   - Y² représente F² ou -NH₂ ou -Ph-XH ou -XH avec X représentant O ou S ou Se et Ph représentant un groupe phényle;
   - Y³ représente F³ ou -NH₂ ou -Ph-XH ou -XH avec X représentant O ou S ou Se et Ph représentant un groupe phényle;
      à condition qu'au maximum un seul radical parmi les radicaux Y¹, Y² et Y³ représente -NH₂,
      à condition qu'au maximum un seul radical parmi les radicaux Y¹, Y² et Y³ représente -Ph-XH ou -XH ;
      à condition que quand Y¹ = -NH₂ alors q = 1 ;
      à condition que quand Y¹ = -NH₂ ou -XH ou -Ph-XH, alors Y² = F² et Y³ = F³ ;
      à condition que quand Y²= -NH₂ ou -XH ou -Ph-XH, alors Y¹ = F¹ et Y₃ = F³ ;
      à condition que quand Y³= -NH₂ ou -XH ou -Ph-XH, alors Y² = F² et Y¹ = F¹ ;
   - F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
   - F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
   - F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
   - R¹, R², R³ et R⁴ représente chacun, indépendamment les uns des autres, un radical choisi parmi un atome d'hydrogène, un groupe alkyle linéaire ou ramifié, comprenant de 1 à 20 atomes de carbone, ledit groupe alkyle comprenant éventuellement un ou plusieurs hétéroatomes, un groupe (hétéro)cycloalkyle comprenant de 3 à 20 atomes de carbone, un groupe arylalkyle comprenant de 7 à 20 atomes de carbone ou un groupe (hétéro)aryle comprenant de 4 à 12 atomes de carbone ;
   - n est un nombre entier allant de 0 à 28 ;
   - p est un nombre entier allant de 0 à 10 ;
   - m représente 0 ou 1 ;
   - q représente 0 ou 1 ;
   - e est un nombre entier allant de 1 à 3 ;
   - f est un nombre entier ou non entier allant de 1 à 3, f étant de préférence un nombre entier égal à 1, 2 ou 3 ;
   - f' est un nombre entier ou non entier allant de 0 à 2, f' étant de préférence un nombre entier égal à 0, 1 ou 2 ;
   - la somme f + f' représente un entier variant de 1 à 3 ;
   - Z représente un radical organique monovalent Z^{m}, divalent Z^{d} ou trivalent Z^{t}, ayant une masse moléculaire moyenne en nombre (Mn) allant de 16 à 22 000 g/mol ;
   - R^{ac} représente un atome d'hydrogène ou un radical hydrocarboné monovalent comprenant de 1 à 60 atomes de carbone, ledit radical pouvant éventuellement comprendre un ou plusieurs hétéroatomes;
- et un composé de formule (IV) suivante : 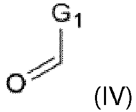 dans laquelle G¹ est tel que défini précédemment dans la formule (I) ci-dessus.

Dans la présente demande, la condition selon laquelle « au maximum un seul radical parmi les radicaux Y¹, Y² et Y³ représente -NH₂ » signifie que soit aucun des radicaux Y¹, Y² et Y³ ne représente -NH₂, soit un seul radical parmi les radicaux Y¹, Y² et Y³ représente -NH₂.

Dans la formule (III) ci-dessus, lorsque f' = 0 et f = 1, alors Z est un radical monovalent Z^{m}.

Dans la formule (III) ci-dessus, lorsque f' = 0 et f = 2, alors Z est un radical divalent Z^{d}.

Dans la formule (III) ci-dessus, lorsque f' = 0 et f = 3, alors Z est un radical trivalent Z^{t}.

Dans la formule (III) ci-dessus, lorsque f' = 1 et f = 1, alors Z est un radical divalent Z^{d}.

Dans la formule (III) ci-dessus, lorsque f' = 2 et f =1, alors Z est un radical trivalent Z^{t}.

Dans la formule (III) ci-dessus, lorsque f + f' = 1, alors Z est un radical monovalent Z^{m}.

Dans la formule (III) ci-dessus, lorsque f + f' = 2, alors Z est un radical divalent Z^{d}.

Dans la formule (III) ci-dessus, lorsque f + f' = 3, alors Z est un radical trivalent Z^{t}.

La réaction entre un composé de formule (III) susmentionnée et un composé de formule (IV) susmentionnée peut être réalisée selon un mode opératoire similaire à celui utilisé (exemple 15) dans US 5,087,661, soit par entrainement azéotropique de l'eau formée à environ 90°C en présence de toluène comme solvant de réaction.

Le composé de formule (IV) peut être utilisé en quantité stœchiométrique ou en excès par rapport au composé de formule (III) selon qu'il est plus ou moins facile d'éliminer l'aldéhyde non réagit de formule (IV) sous pression réduite (environ 1 mm Hg) à 150°C en même temps que le toluène.

Les composés de formule **(III)** peuvent avoir l'une des formules (III-A), (III-B), (III-C) ou (III-D) suivantes : 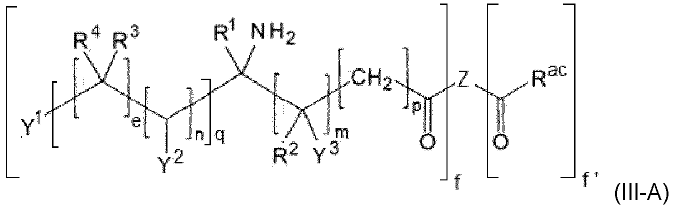 dans laquelle :
- R¹, R², R³, R⁴, n, m, p, q, e, f, f', Z et R^{ac}, sont tels que définis précédemment,
- Y¹ représente F¹ tel que défini précédemment dans la formule (III) ou -Ph-XH, ou -XH avec X représentant O ou S ou Se et Ph représentant un groupe phényle;
- Y² représente F² tel que défini précédemment dans la formule (III) ou -Ph-XH, ou -XH avec X représentant O ou S ou Se et Ph représentant un groupe phényle;
- Y³ représente F³ tel que défini précédemment dans la formule (III) ou -Ph-XH, ou -XH avec X représentant O ou S ou Se et Ph représentant un groupe phényle;
   à condition qu'au plus un des radicaux Y¹, Y² ou Y³ représente un radical -Ph-XH ou -XH avec X représentant O ou S ou Se et Ph représentant un radical phényle; 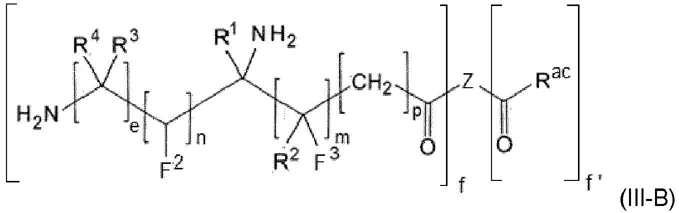 dans laquelle R¹, R², R³, R⁴, F², F³, n, m, p, e, f, f', Z et R^{ac} sont tels que définis précédemment dans la formule (III), 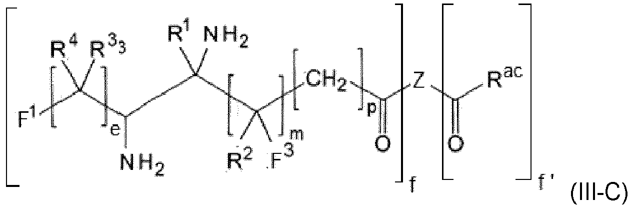 dans laquelle R¹, R², R³, R⁴, F¹, F³, m, p, e, f, f', Z et R^{ac} sont tels que définis précédemment dans la formule (III), 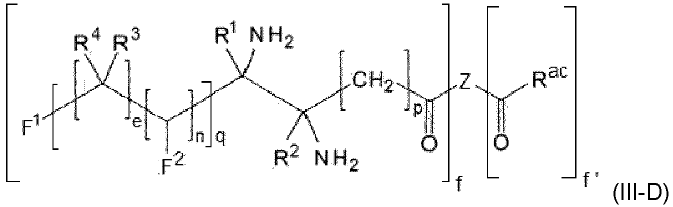 dans laquelle R¹, R², R³, R⁴, F¹, F², n, q, p, e, f, f', Z et R^{ac} sont tels que définis précédemment dans la formule (III).

De façon encore plus préférée, les composés de formule (III) sont des composés de formule (III-A) susmentionnée.

### Composés de formule (III-A)

De préférence, les composés de formule (III-A) sont ceux dans laquelle :
- m représente 0 ou 1 ;
- p représente 0 ou 1 ;
- n représente 0, 1, 2, 3 ou 4;
- q représente 0 ou 1 ;
- e est un nombre entier allant de 1 à 3 ;
- F¹ représente un radical choisi parmi un atome d'hydrogène, un groupe (hétéro)aryle comprenant de 4 à 12 atomes de carbone, un groupe -COOZ, un groupe -C(O)NH₂, un groupe -SMe, ou un groupe guanidyl de formule -NH-C(=NH)-NH₂,
- F² représente un radical choisi parmi un atome d'hydrogène ou un groupe alkyle linéaire ou ramifié comprenant de 1 à 10 atomes de carbone,
- F³ représente un atome d'hydrogène;
- R¹, R², R³ et R⁴ représente chacun, indépendamment les uns des autres, un atome d'hydrogène ou un groupe alkyle linéaire ou ramifié comprenant de 1 à 10 atomes de carbone, encore plus préférentiellement de 1 à 5 atomes de carbone ou un groupe benzyle, ou un groupe phényle.

Les composés de formule (III-A) peuvent avoir l'une des formules (III-A-1), (III-A-2) ou (III-A-3) suivantes : 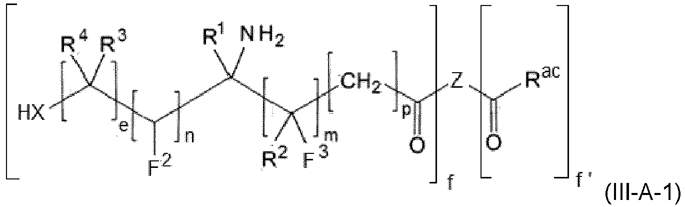 dans laquelle X, R¹, R², R³, R⁴, X, F², F³, n, m, p, e, f, f', Z et R^{ac} sont tels que définis précédemment dans la formule (III-A) ; 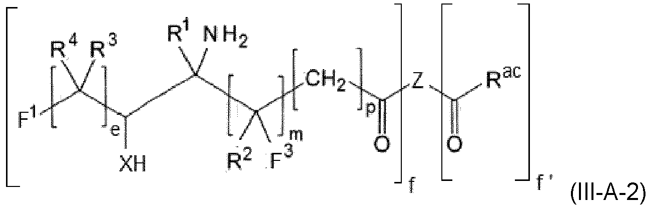 dans laquelle X, R¹, R², R³, R⁴, X, F¹, F³, m, p, e, f, f', Z et R^{ac} sont tels que définis précédemment dans la formule (III-A), 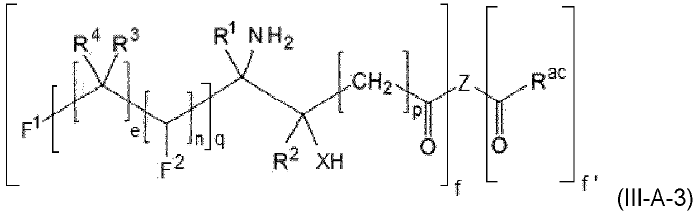 dans laquelle X, R¹, R², R³, R⁴, X, F¹, F², n, p, q, e, f, f', Z, et R^{ac} sont tels que définis précédemment dans la formule (III-A).

De préférence, dans les composés de formule (III-A), (III-A-1), (III-A-2) et (III-A-3) :
- f représente 1 ou 2, et
- f' représente 0 (zéro).

### Composés de formule (III-B)

De préférence, les composés de formule (III-B) sont ceux dans laquelle :
- n représente 0, 3, ou 4;
- m représente 0 ;
- p représente 0 ;
- e est un nombre entier allant de 1 à 3 ;
- F² représente un atome d'hydrogène ;
- F³ représente un radical choisi parmi un atome d'hydrogène ou un groupe alkyle linéaire ou ramifié comprenant de 1 à 10 atomes de carbone ;
- R¹, R², R³ et R⁴ représente chacun, indépendamment les uns des autres, un atome d'hydrogène ou un groupe alkyle linéaire ou ramifié comprenant de 1 à 10 atomes de carbone, encore plus préférentiellement de 1 à 5 atomes de carbone.

### Composés de formule (III-C)

De préférence, les composés de formule (III-C) sont ceux dans laquelle :
- m représente 0 ;
- p représente 0 ;
- e est un nombre entier allant de 1 à 3 ;
- F¹ représente un radical choisi parmi un atome d'hydrogène ou un groupe alkyle linéaire ou ramifié comprenant de 1 à 10 atomes de carbone,
- F³ représente un radical choisi parmi un atome d'hydrogène ou un groupe alkyle linéaire ou ramifié comprenant de 1 à 10 atomes de carbone ;
- R¹, R², R³ et R⁴ représente chacun, indépendamment les uns des autres, un atome d'hydrogène ou un groupe alkyle linéaire ou ramifié comprenant de 1 à 10 atomes de carbone, encore plus préférentiellement de 1 à 5 atomes de carbone.

### Composés de formule (III-D)

De préférence, les composés de formule (III-D) sont ceux dans laquelle :
- n représente 0 ou 3 ;
- p représente 0 ;
- q représente 0 ou 1 ;
- e est un nombre entier allant de 1 à 3 ;
- F¹ représente un radical choisi parmi un atome d'hydrogène ou un groupe alkyle linéaire ou ramifié comprenant de 1 à 10 atomes de carbone,
- F³ représente un radical choisi parmi un atome d'hydrogène ou un groupe alkyle linéaire ou ramifié comprenant de 1 à 10 atomes de carbone ;
- R¹, R², R³ et R⁴ représente chacun, indépendamment les uns des autres, un atome d'hydrogène ou un groupe alkyle linéaire ou ramifié comprenant de 1 à 10 atomes de carbone, encore plus préférentiellement de 1 à 5 atomes de carbone.

### Composés de formule (IV)

Les composés de formule (IV) peuvent avoir l'une des formules (IV-A) ou (IV-B) suivantes : 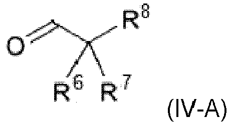 dans laquelle :
- R⁶ et R⁷ représente chacun, indépendamment l'un de l'autre, un radical hydrocarboné monovalent comprenant de 1 à 12 atomes de carbone, ledit radical étant éventuellement substitué par un groupe -OH ;
ou R⁶ et R⁷ forment ensemble un cycle aliphatique comprenant de 4 à 12 atomes de carbone, ledit cycle étant éventuellement substitué ;
- R⁸ représente un radical hydrocarboné monovalent comprenant de 1 à 60 atomes de carbone, ledit radical comprenant éventuellement un hétéroatome ; 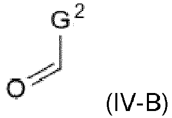 dans laquelle G² représente un radical hétéroaryle éventuellement substitué, ou un radical - C(O)-R¹² avec R¹² représentant un radical alcoxy, un radical alkényle, ou un radical arylalkényle comprenant au moins 6 atomes de carbone,
   ledit radical G² ayant une masse molaire ou une masse moléculaire moyenne en nombre (Mn) allant de 15 à 4 000 g/mol, de préférence allant de 60 à 2 000 g/mol, préférentiellement allant de 60 à 1 000 g/mol et encore plus préférentiellement allant de 60 à 500 g/mol.

Selon un mode de réalisation, G² représente un radical hétéroaryle substitué par au moins un radical choisi parmi -OR¹⁹, O-C(=O)-NHR²³, -O-C(=O)R²⁴, -CH₂-OR²⁰, -CH₂-OC(=O)-R²¹, -COOR^{a}, -C(=O)-NR¹⁶R¹⁷ et -CH₂-O-C(=O)-NHR²², avec R¹⁹, R²⁰, R²¹, R²², R²³ et R²⁴ représentant indépendamment les uns des autres un groupe alkyle linéaire ou ramifié, cyclique ou non, saturé ou insaturé, un atome d'hydrogène, ou un groupe aryle ; R^{a} représentant un radical alkyle linéaire ou ramifié ou un radical (hétéro)aryle, ou un radical arylalkyle ; R¹⁶ et R¹⁷ représentant chacun, indépendamment l'un de l'autre, un hydrogène, un radical alkyle linéaire ou ramifié, un radical cycloalkyle, ou un radical arylalkyle ou R¹⁶ et R¹⁷ forment ensemble un cycle aliphatique comprenant de 4 à 12 atomes de carbone, ledit cycle étant éventuellement substitué et comprenant éventuellement un ou plusieurs hétéroatomes.

De préférence, les composés de formule (IV-A) sont ceux pour lesquels :
- R⁶ et R⁷ représente chacun, indépendamment l'un de l'autre, un radical alkyle linéaire ou ramifié comprenant de 1 à 12 atomes de carbone, ledit radical étant éventuellement substitué par un groupe -OH ; et/ou
- R⁸ représente l'un des radicaux suivants :
   - un radical -CH₂-W dans lequel W représente un groupe aryle éventuellement substitué ou un hétérocycloalkyle éventuellement substitué, ou
   - un radical -CH(R⁹)-O-R¹⁰ dans lequel R⁹ représente un hydrogène, un radical alkyle linéaire ou ramifié, un radical cycloalkyle, un radical arylalkyle, ou un radical alcoxycarbonyle comprenant de 1 à 12 atomes de carbone ; et R¹⁰ représente un hydrogène ou un radical hydrocarboné comprenant de 1 à 30 atomes de carbone, ledit radical comprenant éventuellement un ou plusieurs atomes d'oxygène ; ou
   - un radical -CH(R⁹)-O-C(O)-R¹¹ dans lequel R⁹ représente un hydrogène, un radical alkyle linéaire ou ramifié, un radical cycloalkyle, un radical arylalkyle, ou un radical alcoxycarbonyle comprenant de 1 à 12 atomes de carbone ; et R¹¹ représente un hydrogène, ou un radical alkyle linéaire ou ramifié comprenant de 1 à 30 atomes de carbone, ledit radical alkyle comprenant éventuellement des portions cycliques et éventuellement au moins un hétéroatome ; ou
   - un radical -CH(R¹⁵)-NR¹⁶R¹⁷ dans lequel R¹⁵ représente un hydrogène, un radical alkyle linéaire ou ramifié, un radical cycloalkyle, un radical arylalkyle, ou un radical alcoxycarbonyle comprenant de 1 à 12 atomes de carbone ; et R¹⁶ et R¹⁷ représentent indépendamment l'un de l'autre un hydrogène, un radical alkyle linéaire ou ramifié, un radical cycloalkyle, ou un radical arylalkyle ;
      ou R¹⁶ et R¹⁷ forment ensemble un cycle aliphatique comprenant de 4 à 12 atomes de carbone, ledit cycle étant éventuellement substitué et comprenant éventuellement un ou plusieurs hétéroatomes ; ou
   - un radical -CH(R⁹)-O-C(O)-NHR¹⁸ dans lequel R⁹ représente un hydrogène, un radical alkyle linéaire ou ramifié, un radical cycloalkyle, un radical arylalkyle, ou un radical alcoxycarbonyle comprenant de 1 à 12 atomes de carbone ; et R¹⁸ représente un hydrogène, ou un radical alkyle linéaire ou ramifié comprenant de 1 à 30 atomes de carbone; ou
   - un radical -C(=O)-O-R^{a} avec R^{a} représentant un radical alkyle linéaire ou ramifié ou un radical (hétéro)aryle, ou un radical arylalkyle ; ou
   - un radical -C(=O)-N(R¹⁶)(R¹⁷) avec R¹⁶ et R¹⁷ représentant chacun, indépendamment l'un de l'autre, un hydrogène, un radical alkyle linéaire ou ramifié, un radical cycloalkyle, ou un radical arylalkyle ou R¹⁶ et R¹⁷ forment ensemble un cycle aliphatique comprenant de 4 à 12 atomes de carbone, ledit cycle étant éventuellement substitué et comprenant éventuellement un ou plusieurs hétéroatomes.

De préférence, les composés de formule (IV-B) sont ceux pour lesquels G² représente un radical (hétéro)aryle comprenant de 5 à 12 atomes de carbone, de préférence substitué par au moins deux groupes alkyles ou par au moins un groupe -CH₂OH ou par au moins un groupe -OH.

Les composés de formule (IV-A) ont de préférence l'une des formules (IV-A-1), (IV-A-2), (IV-A-3), (IV-A-4) ou (IV-A-5) suivantes : 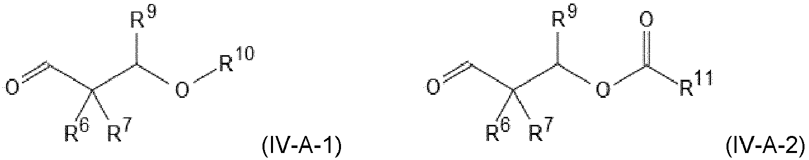 ou 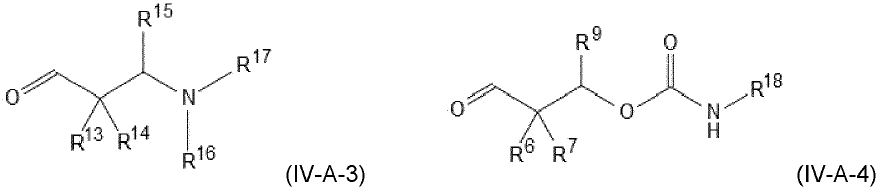 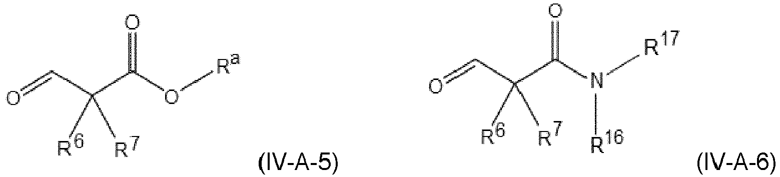 dans lesquelles :
- R⁶ et R⁷ représente chacun, indépendamment l'un de l'autre, un radical hydrocarboné monovalent comprenant de 1 à 12 atomes de carbone, ledit radical étant éventuellement substitué par un groupe -OH ;
   ou R⁶ et R⁷ forment ensemble un cycle aliphatique comprenant de 4 à 12 atomes de carbone, ledit cycle étant éventuellement substitué ;
- R⁹ représente un hydrogène, un radical alkyle linéaire ou ramifié, un radical cycloalkyle, un radical arylalkyle, ou un radical alcoxycarbonyle comprenant de 1 à 12 atomes de carbone ;
- R¹⁰ représente un hydrogène ou un radical hydrocarboné comprenant de 1 à 30 atomes de carbone, ledit radical comprenant éventuellement un ou plusieurs atomes d'oxygène ;
- R¹¹ représente un hydrogène, ou un radical alkyle linéaire ou ramifié comprenant de 1 à 30 atomes de carbone, ledit radical alkyle comprenant éventuellement des portions cycliques et éventuellement au moins un hétéroatome ;
- R¹³ et R¹⁴ représente chacun, indépendamment l'un de l'autre, un radical hydrocarboné monovalent comprenant de 1 à 12 atomes de carbone, ledit radical étant éventuellement substitué par un groupe -OH ;
   ou R¹⁴ et R¹³ forment ensemble un cycle aliphatique comprenant de 4 à 12 atomes de carbone, ledit cycle étant éventuellement substitué ;
- R¹⁵ représente un hydrogène, un radical alkyle linéaire ou ramifié, un radical cycloalkyle, un radical arylalkyle, ou un radical alcoxycarbonyle comprenant de 1 à 12 atomes de carbone ;
- R¹⁶ et R¹⁷ représente chacun, indépendamment l'un de l'autre, un hydrogène, un radical alkyle linéaire ou ramifié, un radical cycloalkyle, ou un radical arylalkyle ;
   ou R¹⁶ et R¹⁷ forment ensemble un cycle aliphatique comprenant de 4 à 12 atomes de carbone, ledit cycle étant éventuellement substitué et comprenant éventuellement un ou plusieurs hétéroatomes ;
- R¹⁸ représente un hydrogène, ou un radical alkyle linéaire ou ramifié comprenant de 1 à 30 atomes de carbone;
- R^{a} représentant un radical alkyle linéaire ou ramifié ou un radical (hétéro)aryle, ou un radical arylalkyle.

Parmi les aldéhydes de formule (IV-A), on peut citer par exemple, le pivalaldéhyde (= 2,2-diméthyl-propanal), le 2,2-diméthyl-butanal, le 2,2-diéthyl-butanal, le 1-méthyl-cyclopentanecarboxaldehyde, le 1-méthyl-cyclohexane-carboxaldehyde; les éthers dérivés du 2-hydroxy-2-méthylpropanal et des alcools tels que le propanol, l'isopropanol, le butanol et le 2-éthylhexanol; les esters dérivés de l'acide 2-formyl-2-méthylpropionique ou de l'acide 3-formyl-3-méthylbutyrique et des alcools tels que le propanol, l'isopropanol, le butanol et le 2-éthylhexanol; les esters dérivés du 2-hydroxy-2-méthylpropanal et d'acides carboxyliques tel que l'acide butyrique, l'acide isobutyrique, et l'acide 2-éthylhexanoique; ainsi que les éthers et esters dérivés du 3-hydroxypropanal disubstitué en position 2,2, les hydroxybutanals ou les aldéhydes homologues supérieurs, comme par exemple le 2,2-diméthyl-3-hydroxypropanal comme décrit ci-après.

Les aldéhydes de formule (IV-A-1) représentent notamment des éthers aliphatiques, cycloaliphatiques ou arylaliphatiques obtenus à partir de 3-hydroxyaldéhydes substitués en position 2,2 et d'alcools, comme par exemple avec des alcools gras, ou de phénols de formule R¹⁰-OH.

Les 3-hydroxyaldéhydes substitués en position 2,2 peuvent être obtenus via une réaction croisée entre des aldéhydes aliphatiques primaires ou secondaire, comme par exemple le formaldéhyde, et des aldéhydes aliphatiques, cycloaliphatiques ou arylaliphatiques secondaires, comme par exemple, l'isobutyraldehyde, le 2-méthylbutyraldéhyde, le 2-éthylbutyraldéhyde, le 2-méthylvaleraldéhyde, le 2-éthylcaproaldéhyde, le cyclopentanecarboxaldéhyde, le cyclohexanecarboxaldéhyde, le 1,2,3,6-tétrahydrobenzaldéhyde, le 2-méthyl-3-phénylpropionaldéhyde, le 2-phénylpropionaldéhyde (hydratropaldéhyde) ou le diphénylacetaldéhyde. Parmi les exemples de 3-hydroxyaldéhydes substitués en position 2,2, on peut citer le 2,2-diméthyl-3-hydroxypropanal, le 2-hydroxyméthyl-2-méthyl-butanal, le 2-hydroxyméthyl-2-éthyl-butanal, le 2-hydroxyméthyl-2-méthyl-pentanal, le 2-hydroxyméthyl-2-éthyl-hexanal, le 1-hydroxyméthyl-cyclopentanecarboxaldéhyde, le 1-hydroxyméthyl-cyclohexanecarboxaldéhyde, le 1-hydroxyméthyl-cyclohex-3-ènecarboxaldehyde, le 2-hydroxyméthyl-2-méthyl-3-phényl-propanal, le 3-hydroxy-2-méthyl-2-phénylpropanal et le 3-hydroxy-2,2-diphényl-propanal.

Les aldéhydes de formule (IV-A-1) particulièrement préférés sont le 2,2-diméthyl-3-phénoxy-propanal, le 3-cyclohexyloxy-2,2-diméthyl-propanal, le 2,2-diméthyl-3-(2-éthyl-hexyloxy)-propanal, le 2,2-diméthyl-3-lauroxy-propanal, le 2,2-diméthyl-3-stéaroxy-propanal et le 3-hydroxy-2,2-diméthyl-propanal, le 3-hydroxy-2-(hydroxyméthyl)-2-méthylpropionaldéhyde.

Selon un mode de réalisation, les composés de formule (IV-A-1) sont ceux pour lesquels :
- R⁶ et R⁷ représente chacun, indépendamment l'un de l'autre, un radical alkyle linéaire ou ramifié comprenant de 1 à 12 atomes de carbone, ledit radical étant éventuellement substitué par un groupe -OH ;
- R⁹ représente un hydrogène ;
- R¹⁰ représente un hydrogène.

Les aldéhydes de formule (IV-A-2) représentent notamment des esters de 3-hydroxyaldéhydes substitués en position 2,2, comme par exemple le 2,2-diméthyl-3-hydroxypropanal, le 2-hydroxyméthyl- 2-méthyl-butanal, le 2-hydroxyméthyl-2-éthylbutanal, le 2-hydroxyméthyl-2-méthyl-pentanal, le 2-hydroxyméthyl-2-éthyl-hexanal, le 1-hydroxyméthylcyclopentanecarboxaldéhyde, le 1-hydroxyméthylcyclohexanecarboxaldéhyde, le 1-hydroxyméthyl-cyclohex-3-ènecarboxaldéhyde, le 2-hydroxyméthyl-2-méthyl-3-phénylpropanal, le 3-hydroxy-2-méthyl-2-phényl-propanal et le 3-hydroxy-2,2-diphényl-propanal, avec des acides carboxyliques.

Les acides carboxyliques utilisables pour cette réaction sont par exemple des acides carboxyliques aliphatiques saturés tel que l'acide formique, l'acide acétique, l'acide propionique, l'acide butyrique, l'acide isobutyrique, l'acide valérique, l'acide caproique, l'acide 2-éthyl-caproique, l'acide énanthique, l'acide caprylique, l'acide pélargonique, l'acide caprique, l'acide undécanoique, l'acide laurique, l'acide tridécanoique, l'acide myristique, l'acide pentadecanoique, l'acide palmitique, l'acide margarique, l'acide stéarique, l'acide nonadécanoique, l'acide arachidique; les acides carboxyliques aliphatiques insaturés tel que l'acide palmitoléique, l'acide oléique, l'acide érucique; les acides carboxyliques aliphatiques polyinsaturés tel que l'acide linoléique, l'acide linolénique, l'acide éléostéarique, l'acide arachidonique; les acides carboxyliques cycloaliphatiques tels que les acides acides cyclohexanecarboxyliques; les acides arylaliphatiques tel que l'acide phénylacétique; les acides carboxyliques aromatiques tel que l'acide benzoique, l'acide naphthoique, l'acide toluoylique, l'acide anisique; les isomères de ces acides; les mélanges d'acides gras issus de la saponification des graisses et des huiles naturelles telle l'huile de colza, l'huile de tournesol, l'huile de lin, l'huile d'olive, l'huile de coprah, l'huile de palmiste, et l'huile de palme; ainsi que les acides dicarboxyliques monoalkyl et monoaryl esters, obtenus par simple estérification des acides diacides carboxyliques tel que l'acide succinique, l'acide glutarique, l'acide adipique adipique, l'acide pimélique, l'acide subérique, l'acide azélaique, l'acide sébacique, l'acide 1,12-dodecanedioique, l'acide maléique, l'acide fumarique, l'acide hexahydrophthalique, l'acide hexahydroisophthalique, l'acide hexahydrotéréphthalique, l'acide 3,6,9-trioxaundécanedioique et les dérivés similaires sur base polyéthylène glycol, avec les alcools tel que le méthanol, l'éthanol, le propanol, le butanol, les homologues supérieur et les isomères de ces alcools.

Parmi les aldéhydes de formule (IV-A-2), on peut par exemple citer le 2,2-diméthyl-oxopropylacétate, ou le 2,2-diméthyl-3-oxopropyl dodécanoate.

Selon un mode de réalisation, les composés de formule (IV-A-2) sont ceux pour lesquels :
- R⁶ et R⁷ représente chacun, indépendamment l'un de l'autre, un radical alkyle linéaire ou ramifié comprenant de 1 à 12 atomes de carbone, ledit radical étant éventuellement substitué par un groupe -OH ;
- R⁹ représente un hydrogène ;
- R¹¹ représente un hydrogène, ou un radical alkyle linéaire ou ramifié comprenant de 1 à 30 atomes de carbone, préférentiellement de 1 à 11 atomes de carbone.

Selon un mode de réalisation, les composés de formule (IV-A-3) sont ceux pour lesquels :
- R¹³ et R¹⁴ représente chacun, indépendamment l'un de l'autre, un radical alkyle linéaire ou ramifié comprenant de 1 à 12 atomes de carbone;
- R¹⁵ représente un hydrogène ;
- R¹⁶ et R¹⁷ forment ensemble un cycle aliphatique comprenant de 4 à 12 atomes de carbone, ledit cycle comprenant un hétéroatome.

Parmi les aldéhydes de formule (IV-A-3), on peut par exemple citer le 2,2-diméthyl-3-(morpholin-4-yl)propanal (CAS Number : 23588-51-4).

Parmi les aldéhydes de formule (IV-A-5), on peut par exemple citer les composés suivants: 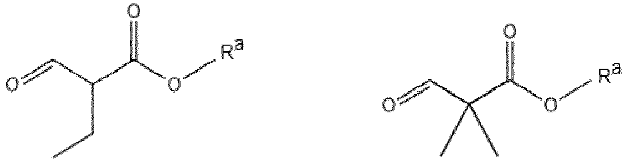 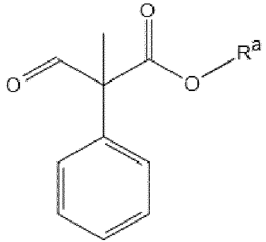 avec R^{a} étant tel que définis ci-dessus.

Parmi les aldéhydes de formule (IV-B), on peut par exemple citer les aldéhydes de formules (IV-B-1), (IV-B-2), (IV-B-3), (IV-B-4), (IV-B-5), (IV-B-6), (IV-B-7) ou (IV-B-8) suivantes : 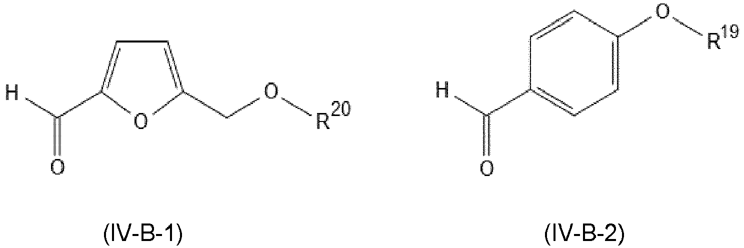 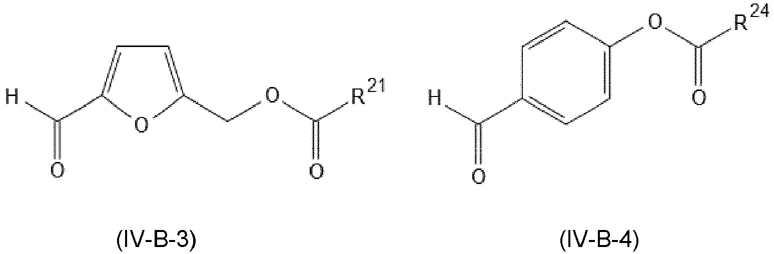 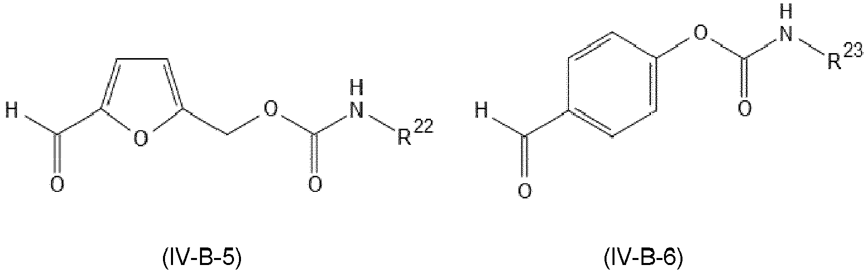 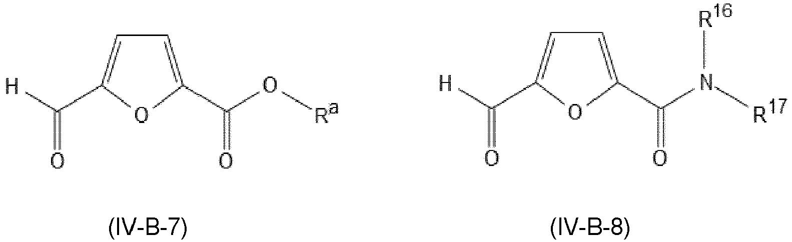 dans lesquelles R^{a}, R¹⁶, R¹⁷, R¹⁹, R²⁰, R²¹, R²², R²³, et R²⁴ sont tels que définis précédemment.

Parmi les aldéhyde de formule (IV-B), on peut par exemple citer les 2- et 3- et 4-formylpyridine, le 2-furfuraldéhyde, le 2-thiophènecarbaldéhyde, le quinoline-2-carbaldéhyde et ces isomères de position en 3-, 4-, 5-, 6-, 7- et 8-, ; ainsi que le glyoxal, les acide-esters glyoxaliques tel que l'ester méthylique de l'acide glyoxalique, le cinnamaldéhyde, et les cinnamaldéhydes substitués ; et le diméthylcyclohex-3-ène-1-carbaldéhyde.

Selon un mode de réalisation, les aldéhydes de formule (IV) sont choisis dans le groupe constitué du 2,2-diméthyl-3-phenoxypropanal, du 2,2-diméthyl-3-cyclohexyloxypropanal, du 2,2-diméthyl-3-(2-éthylhexyloxy)propanal, du 2,2-diméthyl-3-lauryloxypropanal, du 2,2-diméthyl-3-stearyloxypropanal, du 2,2-diméthyl-3-(morpholin-4-yl)propanal, du 2,2-diméthyl-3-oxopropylacétate, du 2,2-diméthyl-3-oxopropyl dodécanoate, du 2,2-diméthyl-3(m-phenyl)propanal, du 2,2-diméthyl-3(m-tolyl)propanal ou Majantal, du diméthylcyclohex-3-ène-1-carbaldéhyde, du 3-hydroxy-2-(hydroxyméthyl)-2-méthylpropionaldéhyde, et de leurs mélanges.

### Composés de formule (III)

Les composés de formule (III) peuvent être obtenus par un procédé comprenant les étapes suivantes :
i) étape de réaction entre :
   - un composé de formule (V) suivante ou l'un de ses dérivés: 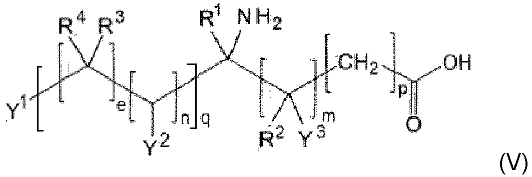 dans laquelle :
      - Y¹ représente F¹ ou -NH₂ ou -Ph-XH ou -XH avec X représentant O ou S ou Se et Ph représentant un groupe phényle;
      - Y² représente F² ou -NH₂ ou -Ph-XH ou -XH avec X représentant O ou S ou Se et Ph représentant un groupe phényle;
      - Y³ représente F³ ou -NH₂ ou -Ph-XH ou -XH avec X représentant O ou S ou Se et Ph représentant un groupe phényle ;
         à condition qu'au maximum un seul radical parmi les radicaux Y¹, Y² et Y³ représente -NH₂,
         à condition qu'au maximum un seul radical parmi les radicaux Y¹, Y² et Y³ représente -Ph-XH ou -XH ;
         à condition que quand Y¹ = -NH₂ alors q = 1 ;
         à condition que quand Y¹ = -NH₂ ou -Ph-XH ou -XH, alors Y² = F² et Y³ = F³ ;
         à condition que quand Y² = -NH₂ ou -Ph-XH ou -XH, alors Y¹ = F¹ et Y³ = F³ ;
         à condition que quand Y³ = -NH₂ ou -Ph-XH ou -XH, alors Y¹ = F¹ et Y² = F² ;
      - F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe -COOH, un groupe -C(O)NH₂, un groupe -SMe, ou un groupe guanidyl de formule -NH-C(=NH)-NH₂,
      - F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe -COOH, un groupe -C(O)NH₂, un groupe -SMe, ou un groupe guanidyl de formule -NH-C(=NH)-NH₂,
      - F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe -COOH, un groupe -C(O)NH₂, un groupe -SMe, ou un groupe guanidyl de formule -NH-C(=NH)-NH₂,
      - R¹, R², R³ et R⁴ représente chacun, indépendamment les uns des autres, un radical choisi parmi un atome d'hydrogène, un groupe alkyle linéaire ou ramifié, comprenant de 1 à 20 atomes de carbone, ledit groupe alkyle comprenant éventuellement un ou plusieurs hétéroatomes, un groupe (hétéro)cycloalkyle comprenant de 3 à 20 atomes de carbone, un groupe arylalkyle comprenant de 7 à 20 atomes de carbone ou un groupe (hétéro)aryle comprenant de 5 à 12 atomes de carbone ;
      - n est un nombre entier allant de 0 à 28 ;
      - m représente 0 ou 1 ;
      - p est un nombre entier allant de 0 à 10 ;
      - q représente 0 ou 1 ;
      - e est un nombre entier allant de 1 à 3 ;
   - et un composé de formule (VI) suivante :

      Z'(X'H)ₓ (VI)

      dans laquelle :
      - Z' représente un atome d'hydrogène ou un radical organique monovalent Z'^{m}, divalent Z'^{d} ou trivalent Z'^{t}, ayant masse molaire ou une une masse moléculaire moyenne en nombre (Mn) allant de 1 à 20 000 g/mol ;
      - X' représente O ou NR' avec R' représentant un atome d'hydrogène ou un groupe alkyle linéaire ou ramifié comprenant de 1 à 20 atomes de carbone pouvant éventuellement comprendre un ou plusieurs hétéroatomes, un groupe arylalkyle comprenant de 1 à 20 atomes de carbone, un groupe (hétéro)aryle comprenant de 4 à 12 atomes de carbone, ou un groupe (hétéro)cycloalkyle comprenant de 3 à 20 atomes de carbone ;
      - x représente un nombre entier ou non entier allant de 1 à 3 ;
   - et éventuellement un composé de formule (VII) suivante, lorsque le composé de formule (VI) susmentionnée est tel que x = 2 ou 3 :

      R^{ac}-C(=O)-OH (VII)

      dans laquelle R^{ac} représente un hydrogène ou radical hydrocarboné monovalent comprenant de 1 à 60 atomes de carbone, linéaire ou ramifié, saturé ou instauré, préférentiellement de 8 à 20 atomes de carbones, ledit radical pouvant éventuellement comprendre un ou plusieurs hétéroatomes ;
ii) une éventuelle étape de déprotection lorsque l'étape i) est réalisée en présence d'un dérivé d'un composé de formule (V).

On entend par dérivé d'un composé de formule (V) susmentionnée, un composé de formule (V) dans laquelle le groupe -NH₂ est protégé par un groupement protecteur P formant un groupe -NHP. L'étape i) est notamment réalisée avec un tel dérivé de composé de formule (V) lorsqu'elle a lieu en présence d'un composé de formule (VI) et/ou (VII). Un dérivé d'un composé de formule (V) peut par exemple être un composé de formule (V) dans laquelle le(s) groupe(s) -NH₂ est(sont) protégé(s) sous forme -NH-Boc (Boc étant tertbutyloxycarbonyl), sous forme de -NH-CBz (Cbz étant N-benzyloxycarbonyl) ou sous forme de -NH-Fmoc (Fmoc étant 9-fluorenylmethylenoxy-carbonyl).

Un dérivé d'un composé de formule (V) peut être par exemple préparé par réaction d'un composé de formule (V) avec le dicarbonate de di-tert-butyle pour protéger le(s) groupe(s) -NH₂ sous forme -NHBoc afin d'obtenir les N-Boc aminoacides de formule suivante :

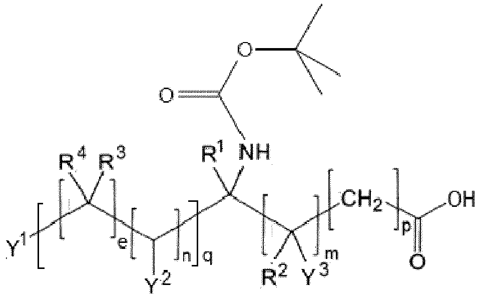

Un dérivé d'un composé de formule (V) peut être par exemple préparé par réaction d'un composé de formule (V) avec le tertbutyldimethylsilyl chloride dans la dimethylformamide en présence d'imidazole pour protéger le(s) groupe(s) -XH et -Ph-XH sous forme -X-TBDMS.

Selon un mode de réalisation, l'étape i) est réalisée en présence d'un composé de formule (VII) lorsque l'on cherche à préparer un composé de formule (III) dans laquelle f' est différent de 0. La réaction peut avoir lieu à basse température, c'est-à-dire à une température inférieure ou égale à 25 °C.

De préférence, l'étape i) est réalisée avec au moins un composé de formule (V), et en l'absence de composé de formule (VII).

L'étape i) peut avoir lieu dans des quantités molaires telles que le ratio molaire -X'H / -C(=O)OH peut aller de 10 : 1 à 1 : 1.

Quand Z'(XH)x représente Z'(OH)x, la synthèse des aminoesters lors de l'étape i) peut être réalisée dans un solvant organique, par exemple choisi parmi les éthers (par exemple l'éther éthylique ou le tétrahydrofurane), les solvants chlorés (par exemple le dichlorométhane) ou directement dans Z'(OH)x en excès selon son point d'ébullition sous pression réduite tel que décrit dans Tetrahedron Letters (1986), 27 (41), pages 4975-4978 et dans Tetrahedron (1988), 44 (17), 5495-506 (1988).

Quand Z'(XH)x représente Z'(NHR')x, la synthèse des aminoamides, lors de l'étape i) peut être réalisée dans un solvant organique, par exemple choisi parmi les éthers (par exemple l'éther éthylique ou le tétrahydrofurane), les solvants chlorés (par exemple le dichlorométhane) tel que décrit dans Journal of Organic Chemistry (2006), 71(4), 1750-1753 à partir des aminoesters correspondants.

L'étape ii) de déprotection des fonctions -NH₂ sous forme -NHBoc peut être réalisée par toute méthode conventionnelle connue de l'homme du métier. Il peut par exemple s'agir d'une étape d'hydrolyse acide. Par exemple, il est possible de réaliser l'étape ii) en présence d'acide trifluoroacétique ou d'acide chlorhydrique.

L'étape ii) de déprotection des fonctions -XH et -Ph-XH sous forme -X-TBDMS peut être réalisée par toute méthode conventionnelle connue de l'homme du métier. Il peut par exemple s'agir d'une étape de coupure. Par exemple, il est possible de réaliser l'étape ii) en présence de fluorure de tetra-n-butylammonium (TBAF).

Les composés de formule (VI) peuvent par exemple être choisis parmi les monols, les diols, les triols, les monoamines, les diamines, les triamines, les aminoalcools.

A titre de composés de formule (VI), on peut par exemple citer :
- les monols linéaires ou ramifiés, cycliques ou acycliques, saturés ou insaturés, tels que le méthanol (CAS : 67-56-1), l'éthanol (CAS : 64-17-5), le propanol (CAS : 71-23-8), l'isopropanol (CAS : 67-63-0), l'alcool allylique (CAS : 107-18-6), le 1-butanol (CAS : 71-36-3), l'isobutanol (CAS : 78-83-1), le 1-pentanol ou alcool amylique (CAS : 71-41-0), le 3-méthyl-1-butanol ou alcool isoamylique (CAS : 123-51-3), le 2-pentanol (CAS : 6032-29-7), le 3-pentanol (CAS: 584-02-1), le 4-methyl-2-pentanol (CAS : 108-11-2), 5-phenyl-1-pentanol (CAS : 10521-91-2), cyclopentanol (CAS : 96-41-3), le 1-hexanol (CAS: 111-27-3),), le cyclohexanol (CAS: 108-93-0), le 2-butoxyethanol (CAS: 111-76-2), le 1-heptanol (CAS: 111-70-6), le 2-heptanol (CAS: 543-49-7), le 3-heptanol (CAS: 589-82-2), le 4-methyl-3-heptanol (CAS : 14979-39-6), le 3-methyl-4-heptanol (CAS : 1838-73-9), le 6-methyl-2-heptanol (CAS : 4730-22-7), le 6-methyl-3-heptanol (CAS : 18720-66-6), le 2-(2-(2-methoxyethoxy)ethoxy)ethanol (CAS: 112-35-6), l'alcool benzylique (CAS: 100-51-6), le 1-octanol-1 (CAS : 111-87-5), le 2-octanol (CAS : 123-96-6), le 3-octanol (CAS : 589-98-0), le 4-octanol (CAS : 589-62-8), le 2-éthyl-1-hexanol (CAS : 104-76-7), le 2-hexyloxyethanol (CAS : 112-25-4), le 2-(2-butoxyethoxy)ethanol (CAS : 112-34-5), le 1-nonanol (CAS : 143-08-8), le 2-nonanol (CAS : 628-99-9), le 3-nonanol (CAS : 624-51-1), le 4-nonanol (CAS : 5932-79-6), le 5-nonanol (CAS : 623-93-8), l'isononanol (CAS : 27458-94-2), le 2,6-dimethyl-2-heptanol (CAS : 13254-34-7)), le 1-octanol (CAS : 111-87-5), le 2-octanol (CAS : 123-96-6), le 3-octanol (CAS : 589-98-0), le 4-octanol (CAS : 589-62-8), le 2-methyl-3-octanol (CAS : 26533-34-6), le 2,2-dimethyl-3-octanol (CAS : 19841-72-6), le 3,5-dimethyl-4-octanol (CAS : 19781-12-5), le 3,6-dimethyl-3-octanol (CAS : 151-19-9), le 3-methyl-4-octanol (CAS : 26533-35-7), le 6-ethyl-3-octanol (CAS : 19781-27-2), le 3,7-dimethyl-1-octanol (CAS : 106-21-8), le 3,7-dimethyl-3-octanol ou Tetrahydrolinalool (CAS : 78-69-3), le 2-butyl-1-octanol (CAS : 3913-02-8), le 8-phenyl-1-octanol (CAS : 10472-97-6), le 3-methyl-3-octanol (CAS: 5340-36-3), le 3,6-dimethyl-2-heptanol (CAS: 1247790-47-1), 3,6,9,12-tetraoxotridecanol (CAS : 23783-42-8), le 1-nonanol (CAS : 143-08-8), le 2-nonanol (CAS : 628-99-9), le 3-nonanol (CAS : 624-51-1), le 4-nonanol (CAS : 5932-79-6), le 5-nonanol (CAS : 623-93-8), le 2-methyl-3-nonanol (CAS : 26533-33-5), le 2-methyl-4-nonanol (CAS : 26533-31-3), 2-pentyl-1-nonanol (CAS : 5333-48-2), le 3-methyl-4-nonanol (CAS : 26533-32-4), le 4-methyl-4-nonanol (CAS : 23418-38-4), le 5-ethyl-2-nonanol (CAS : 103-08-2), le 8-methyl-1-nonanol (CAS : 55505-26-5)), 4,8-dimethyl-1-nonanol (CAS : 33933-80-1), le 1-decanol (CAS : 112-30-1), le 2-decanol (CAS : 1120-06-5), le 3-decanol (CAS : 1565-81-7), le 2-hexyl-1-decanol (CAS: 2425-77-6), le 6-ethyl-3-decanol (CAS: 19780-31-5), l'isodécanol (CAS : 25339-17-7), le 2-propyl-1-heptanol (CAS : 10042-59-8), le cyclodécanol (CAS : 1502-05-2), le 1-undecanol (CAS : 112-42-5), le 2-undecanol (CAS : 1653-30-1), le 7-ethyl-2-methyl-4-undecanol (CAS: 103-20-8), le 10-undecénol (CAS: 112-43-6), le 2-undecénol (CAS : 75039-84-8), l'isoundécanol (CAS : 55505-28-7), le 1-dodécanol (CAS : 112-53-8), le 2-dodecanol (CAS: 10203-28-8), le 2-octyl-1-dodecanol (CAS: 5333-42-6), l'isododecanol (CAS : 25428-98-2), le 2,6,8-trimethyl-4-nonanol (CAS : 123-17-1), le 1-tridecanol (CAS: 112-70-9), le 7-tridecanol (CAS : 927-45-7), le 3,9-diethyl-6-tridecanol (CAS : 123-24-0), l'isotridecanol (CAS : 27458-92-0)), le 1-tétradécanol (CAS : 112-72-1), le 2-tetradecanol (CAS : 4706-81-4), le 2-decyl-1-tetradecanol (CAS : 58670-89-6), le 1-pentadecanol (CAS : 629-76-5), le 2-benzyl-1-heptanol (CAS : 92368-90-6), le 1-hexadécanol (CAS : 36653-82-4), le 2-hexadecanol (CAS : 14852-31-4), le 1-heptadecanol, (CAS : 1454-85-9), le 1-octadecanol (CAS : 112-92-5), le 9-octadecanol (CAS : 1454-85-9), le 1-isooctadecanol (CAS : 27458-93-1), le 1-eicosanol ou alcool arachidylique (CAS : 629-96-9), le 1-docosanol ou alcool béhénylique (CAS : 661-19-8), le 2-dodecyl-1-hexadecanol (C28), le 2-tetradecyl-1-octadecanol (CAS : 32582-32-4), l'abiétinol (CAS : 666-84-2), les alcools oligoméres linéaires allant de C14 à C60 (CAS : 71750-71-5) comme les UNILIN® alcohols commercialisé par la société BAKER HUGHES, les monols alkoxylés, les acides gras alkoxylés, et leurs mélanges ;
- les diols linéaires ou ramifiés, cycliques ou non, saturés ou insaturés, tels que l'éthylène glycol (CAS : 107-21-1), le diéthylène glycol (CAS : 111-46-6), le triéthylène glycol (CAS : 112-27-6), le tétraéthylène glycol (CAS : 112-60-7), le pentaéthylène glycol (CAS : 4792-15-8), l'hexaéthylène glycol (CAS : 2615-15-8), le propylène glycol (CAS : 57-55-6), le dipropylène glycol (CAS : 25265-71-8), le tripropylène glycol (CAS : 24800-44-0), le tétrapropylène glycol (CAS : 24800-25-7), le pentapropylene glycol (CAS : 21482-12-2), l'hexapropylene glycol (CAS: 52794-80-6), le 1,3-propanediol (CAS: 504-63-2), le 2,2-dibenzyl-1,3-propanediol (CAS: 31952-16-6), le 1,2-butanediol (CAS: 584-03-2), le 1,3-butanediol (CAS : 107-88-0), le 1,4-butanediol (CAS : 110-63-4), le 2-butène-1,4-diol (CAS : 6117-80-2), le 1,2-pentanediol (CAS: 5343-92-0), le 1,3-pentanediol (CAS: 3174-67-2), le 1,4-pentanediol (CAS: 626-95-9), le 1,5-pentanediol (CAS: 111-29-5), le 1,2-hexanediol (CAS : 6920-22-5), le 1,3-hexanediol (CAS : 21531-91-9), le 1,4-hexanediol (CAS : 16432-53-4), le 1,5-hexanediol (CAS: 928-40-5), le 1,6-hexanediol (CAS: 629-11-8), le 1,4-cyclohexanedimethanol (CAS: 105-08-8), le 3-éthyl-2-méthyl-1,5-pentanediol, le 2-éthyl-3-propyl-1,5-pentanediol, le 2,4-diméthyl-3-éthyl-1,5-pentanediol, le 2-éthyl-4-méthyl-3-propyl-1,5-pentadiol, le 2,3-diéthyl-4-méthyl-1,5-pentanediol, le 3-éthyl-2,2,4-triméthyl-1,5-pentadiol, le 2,2-diméthyl-4-éthyl-3-propyl-1,5-pentanediol, le 2-méthyl-2-propyl-1,5-pentanediol, le 2,4-diméthyl-3-éthyl-2-propyl-1,5-pentanediol, le 2,3-dipropyl-4-éthyl-2-méthyl-1,5-pentanediol, le 2-butyl-2-éthyl-1,5-pentanediol, le 2-butyl-2,3-diéthyl-4-méthyl-1,5-pentanediol, le 1,4-benzenediméthanol (CAS : 589-29-7), le 2-butyl-2,4-diéthyl-3-propyl-1,5-pentanediol, le 3-butyl-2-propyl-1,5-pentanediol, le 2-méthyl-1,5-pentanediol (CAS :42856-62-2), le 3-méthyl-1,5-pentanediol (CAS : 4457-71-0), le 2,2-diméthyl-1,3-pentanediol (CAS : 2157-31-5), le 2,2-diméthyl-1,5-pentanediol (CAS : 3121-82-2), le 3,3-diméthyl-1,5-pentanediol (CAS : 53120-74-4), le 2,3-diméthyl-1,5-pentanediol (CAS : 81554-20-3), le néopentyl glycol (CAS : 126-30-7), le 2,2-diethyl-1,3-propanediol (CAS 115-76-4), le 2-méthyl-2-propyl-1,3-propanediol (CAS : 78-26-2), le 2-butyl-2-éthyl-1,3-propanediol (CAS : 115-84-4), le 2-méthyl-1,3-propanediol (CAS : 2163-42-0), le 2-benzyloxy-1,3-propanediol (CAS : 14690-00-7), le 2,2-dibenzyl-1,3-propanediol (CAS : 31952-16-6), le 2,2-dibutyl-1,3-propanediol (CAS : 24765-57-9), le 2,2-diisobutyl-1,3-propanediol, le 2,4-diéthyl-1,5-pentanediol, le 2-éthyl-1,6-hexanediol (CAS : 15208-19-2), le 2,5-diméthyl-1,6-hexanediol (CAS : 49623-11-2), le 5-méthyl-2-(1-methylethyl)-1,3-hexanediol (CAS : 80220-07-1), le 1,4-diméthyl-1,4-butanediol (CAS : ), le 1,5-hexanediol (CAS : 928-40-5), le 3-méthyl-1,6-hexanediol (CAS : 4089-71-8), le 3-tert-butyl-1,6-hexanediol (CAS : 82111-97-5), le 1,3-heptanediol (CAS : 23433-04-7), le 1,2-octanediol (CAS : 1117-86-8), le 1,3-octanediol (CAS : 23433-05-8), le 2,2,7,7-tétraméthyl-1,8-octanediol (CAS : 27143-31-3), le 2-méthyl-1,8-octanediol (CAS : 109359-36-6), le 2,6-diméthyl-1,8-octanediol (CAS : 75656-41-6), le 1,7-octanediol (CAS : 3207-95-2), le 4,4,5,5-tétraméthyl-3,6-dioxa-1,8-octanediol (CAS : 76779-60-7), le 1,9-nonanediol (CAS: 3937-56-2), le 3,7-dioxa-1,9-nonanediol (CAS : 67439-82-1), le 2,2,8,8-tétraméthyl-1,9-nonanediol (CAS : 85018-58-2), le 1,2-nonanediol (CAS : 42789-13-9), le 2,8-diméthyl-1,9-nonanediol (CAS : 40326-00-9), le 1,5-nonanediol (CAS : 13686-96-9), le 2,9-diméthyl-2,9-dipropyl-1,10-décanediol (CAS : 85018-64-0), le 2,9-dibutyl-2,9-diméthyl-1,10-decanediol (CAS : 85018-65-1), le 2,9-diméthyl-2,9-dipropyl-1,10-décanediol (CAS : 85018-64-0), le 2,9-diéthyl-2,9-diméthyl-1,10-décanediol (CAS 85018-63-9), le 2,2,9,9-tétraméthyl-1,10-decanediol (CAS : 35449-36-6), le 2-nonyl-1,10-décanediol (CAS : 48074-20-0), le 1,9-décanediol (CAS : 128705-94-2), le 2,2,6,6,10,10-hexaméthyl-4,8-dioxa-1,11-undécanediol (CAS : 112548-49-9), le 1-phényl-1,11-undécanediol (CAS : 109217-58-5), le 2-octyl-1,11-undécanediol (CAS : 48074-21-1), le 2,10-5 diéthyl-2,10-diméthyl-1,11-undécanediol (CAS : 85018-66-2), le 2,2,10,10-tétramethyl-1,11-undécanediol (CAS : 35449-37-7), le 1-phényl-1,11-undécanediol (CAS : 109217-58-5), le 1,2-undécanediol (CAS : 13006-29-6), le 1,2-dodécanediol (CAS : 1119-87-5), le 2,11-dodécanediol (CAS : 33666-71-6), le 2,11-diéthyl-2,11-diméthyl-1,12-dodécanediol (CAS : 85018-68-4), le 2,11-diméthyl-2,11-dipropyl-1,12-dodécanediol (CAS : 85018-69-5), le 2,11-dibutyl-2,11-diméthyl-1,12-dodécanediol (CAS : 85018-70-8), le 2,2,11,11-tétraméthyl-1,12-dodécanediol (CAS : 5658-47-9), le 1,11-dodécanediol (CAS : 80158-99-2), le 11-méthyl-1,7-dodécanediol (CAS : 62870-49-9), le 1,4-dodécanediol (CAS : 38146-95-1), le 1,3-dodécanediol (CAS : 39516-24-0), le 1,10-dodécanediol (CAS : 39516-27-3), le 2,11-diméthyl-2,11-dodécanediol (CAS : 22092-59-7), le 1,5-dodécanediol (CAS : 20999-41-1), le 6,7-dodécanediol (CAS : 91635-53-9), 1,13-tridecandiol (CAS: 13362-52-2), le 1,2-tridecandiol (CAS: 90091-76-2), le 1,14-tétradécanediol (CAS: 19812-64-7), le 1,2-tétradécanediol (CAS: 21129-09-9), le 1,15-pentadecanediol (CAS: 14722-40-8), le 1,2-pentadecanediol (CAS: 33968-47-7), le 1,16-hexanediol (CAS: 7735-42-4), le 1,2-hexadecanediol (CAS: 6920-24-7), le 1,17-heptadecanediol (CAS: 66577-59-1), le 1,2-heptadecanediol (CAS: 34719-63-6), le 1,18-octadecanediol (CAS: 3155-43-9), 1,2-octadecanediol (CAS: 20294-76-2), le 1,12-octadecanediol (CAS: 2726-73-0), 1,20-eicosanediol (CAS: 7735-43-5), le 1,15-eicosanediol (CAS: 20301-18-2), le 1,4-eicosanediol (CAS: 16274-31-0), le 1,2-eicosanediol (CAS : 39825-93-9), les alcools gras dimères (CAS : 61788-89-4) comme les PRIPOL® disponible chez CRODA, les polybutadiène diols (CAS : 69102-90-5) comme les POLY BD™ disponible chez IDEMITSU KOSAN et CRAY VALLEY, les polyisoprène diols (CAS : 308067-82-5) comme les POLY IP™ disponible chez IDEMITSU KOSA, les polyisoprene diols hydrogénés (CAS : 1028331-63-6) comme les EPOL™ disponible chez IDEMITSU KOSAN, les polyéthylènes glycols (CAS : 25322-68-3), les polypropylènes glycols (CAS : 25322-69-4) comme par exemple les ACCLAIMS® polyols disponibles chez COVESTRO, les polybutylène glycols (CAS : 24969-07-1), les polytétrahydrofurane ou PolyTHF (CAS : 25322-69-4), les poly(éthylène-propylène) glycols, les poly(éthylène-butylène) glycols, les poly(éthylène-propylène-butylène) glycols, les acides gras dimères alkoxylés, et leurs mélanges ;
- les diols hétérocycliques tel que le 2,5-furanediméthanol (CAS : 1883-75-6), l'isosorbide (CAS : 652-67-5) ainsi que leurs mélanges ;
- les triols linéaires ou ramifiés, saturés ou insaturés tels que le glycérol (CAS : 56-81-15), le triméthylolméthane (CAS : 4704-94-3), le triméthyloléthane (CAS : 77-85-0), le triméthylolpropane (CAS : 77-99-6), les alcools gras trimères (C54) et les triols alkoxylés tels que les polyéthylène glycols (PEG), les polybutylène glycols (PBG), les polypropylènes glycols (PPG), les poly(éthylène-propylène) glycols, les poly(éthylène-butylène) glycols, les poly(éthylène-propylène-butylène) glycols comme par exemple le VORANOL CP3355 disponible chez DOW CHEMICAL, les polyols synthétiques dérivés d'huile de Colza à haute teneur en chaînes oléique (CAS : 2059146-46-0), les polyols d'origine naturelle comme l'huile de ricin (CAS : 8001-79-4), les polyols naturels ou synthétiques alkoxylés, et leurs mélanges ;
- les triols hétérocycliques, tels que le 1,3,5-cyclohexanetriol (CAS :, le 1,3,5-cyclohexanetrimethanol (CAS : 5962-82-3) ainsi que leurs mélanges ;
- les monoamines linéaires, cycliques ou ramifiées telles que la méthylamine (CAS : 74-89-5), la diméthylamine (CAS : 124-40-3), l'éthylamine (CAS : 75-04-7), la diéthylamine (CAS : 109-89-7), la propylamine (CAS: 107-10-8), la dipropylamine (CAS: 142-84-7), la diisopropylamine (CAS : 108-18-9), la butylamine (CAS : 109-73-9), la dibutylamine (CAS : 111-92-2), Di-*sec*-Butylamine (CAS: 626-23-3), la diisobutylamine (CAS: 110-96-3), la 1-pentylamine (CAS : 110-58-7), la morpholine (CAS : 110-91-8), cyclohexylamine (CAS : 108-91-8), la cyclohexanemethanamine (CAS : 3218-02-8), la 1-amino-3-methylbutane (CAS : 107-85-7), la 1-aminohexane (CAS : 111-26-2), la 2-ethyl-1-hexylamine (CAS : 104-75-6), la bis(2-ethylhexyl)amine (CAS : 106-20-7), la 1-aminoheptane (CAS : 1-Aminoheptane), 2-aminoheptane (CAS : 123-82-0), la 2-amino-6-methylheptane (CAS : 543-82-8), la benzylamine (CAS : 100-46-9), la furfurylamine (CAS : 617-89-0), la dibenzylamine (CAS : 103-49-1), 1-aminooctane (CAS: 111-86-4), la 2-aminooctane (CAS: 693-16-3), la dioctylamine (CAS : 1120-48-5), 1-aminononane (CAS : 112-20-9), la 1-aminodecane (CAS : 2016-57-1), la didecylamine (CAS: 1120-49-6), la 1-aminoundecane (CAS: 7307-55-3), la diundecylamine (CAS : 16165-33-6), la 1-aminododecane (CAS : 124-22-1), la didodecylamine (CAS : 3007-31-6), la 1-aminotridecane (CAS : 2869-34-3), la ditridecylamine (CAS : 5910-75-8), la 1aminotetradecane (CAS : 2016-42-4), la ditetradecylamine (CAS : 17361-44-3), la 1-aminopentadecane (CAS : 2570-26-5), la dipentadecylamine (CAS : 35551-81-6), la 1-aminohexadecane (CAS : 143-27-1), la dihexadecylamine (CAS : 16724-63-3), la 1-aminooctadecane (CAS : 124-30-1), la dioctadecylamine (CAS: 112-99-2), la 1-aminoeicosane (CAS: 10525-37-8), la dieicosylamine (CAS : 3071-00-9), les amines grasses primaires de coprah (CAS : 61788-46-3) comme par exemple les NORAM® C de la société ARKEMA, les amines grasses primaires de suif (CAS : 161544-60-1) comme par exemple les NORAM® S de la société ARKEMA, les amines grasses primaires oléique (CAS : 112-90-3) comme par exemple les NORAM® O de la société ARKEMA, les polyéther amines comme les JEFFAMINES M de la société HUNTSMAN ainsi que leurs mélanges ;
- les monoamines hétérocycliques telles que la furfurylamine (CAS : 617-89-0) ainsi que leurs mélanges ;
- les diamines, primaires ou secondaires, linéaires ou ramifiées, cycliques ou non, telles que l'éthylènediamine (CAS: 107-15-3), la 1,3-diaminopropane (CAS: 109-76-2), la 1,2-diaminopropane (CAS: 78-90-0), la 1,4-diaminobutane (CAS: 110-60-1), la pipérazine (CAS: 110-85-0), la 1,5-diaminopentane (CAS: 462-94-2), la 1,6-diaminohexane (CAS: 124-09-4), la 1,7-diaminoheptane (CAS : 646-19-5), la 1,8-diaminooctane (CAS : 373-44-4), la m-xylylènediamine (CAS: 1477-55-0), la 1,9-diaminononane (CAS: 646-24-2), la 1,10-diaminodecane (CAS: 646-25-3), 1,11-diaminoundecane (CAS: 822-08-2), la 1,12-dodecane (CAS: 2783-17-7), la 1,13-diaminotridécane (CAS: 14465-66-8), la 1,14-diaminotetradecane (CAS : 7735-02-6), la 1,15-diaminopentadecane (CAS : 131459-33-1), la 1,16-diaminohexadecane (CAS: 929-94-2), la 1,17-diaminoheptadecane (CAS: 929-94-2), la 1,18-diaminooctadecane (CAS: 10341-25-0), la 1,19-diaminononanedecane (CAS: 13439-54-8), la 1,20-diaminoeicosane (CAS: 34540-46-0), la *N*-isopropyl-1,3-propanediamine (CAS: 3360-16-5), la *N*-methyl-1,3-diaminopropane (CAS: 6291-84-5), la *N,N*-Dimethyl-1,3-diaminopropane (CAS: 109-55-7), la N-coco-1,3-diaminopropane (CAS :) comme par exemple le DlNORAM® C disponible chez ARKEMA, la N-tallow-1,3-diaminopropane (CAS : 61791-55-7) comme par exemple le DlNORAM® S disponible chez ARKEMA, la N- 9-octadecen-1-yl--1,3-diaminopropane (CAS: 7173-62-8) comme par exemple le DlNORAM® O disponible chez ARKEMA, les amines grasses dimères (CAS : 68955-56-6), comme par exemple les PRIAMINE™ de chez CRODA et les homologues *N,N'*-diméthylées de ces diamines, les polyéther diamines comme les JEFFAMINES D de la société HUNTSMAN ainsi que leurs mélanges ;
- les diamines hétérocycliques telles que 2,5-furandimethanamine (CAS : 2213-51-6), le 2,5-bis(aminomethyl)tetrahydrofurane (CAS : 66918-21-6) ainsi que leurs mélanges ;
- les triamines telles que les amines grasses trimères obtenues par ammoniation réductrice (NH₃/H₂/Ni Raney) des acides gras trimères correspondants, les polyéther triamines comme les JEFFAMINES T de la société HUNSTMAN ainsi que leurs mélanges ;
- les triamines hétérocycliques ;
- les triamines aromatiques telles que 1,3,5-benzenetrimethanamine (CAS : 77372-56-6) ainsi que leurs mélanges ; les aminoalcools linéaires, cycliques ou ramifiés tels que l'éthanolamine (CAS: 141-43-5), la diéthanolamine (CAS: 111-42-2), la triéthanolamine (CAS : 102-71-6), la N-methydiéthanolamine (CAS : 105-59-9), l'isopropanolamine (CAS : 78-96-6), la diisopropanolamine (CAS: 110-97-4), la N-methyl diisopropanolamine (CAS: 4402-30-6), le 3-amino-1-propanol (CAS : 156-87-6), le 2-amino-2-methyl-1-propanol, (CAS : 124-68-5), le 3-dimethylamino-1-propanol (CAS : 3179-63-3), la *N*-butyldiéthanolamine (CAS : 102-79-4), *N-tert*-Butyldiethanolamine (CAS : 2160-93-2), , les amines grasses primaires dialkoxylées comme par exemple les NORAMOX C , O et SH de la société ARKEMA, les amines secondaires alkoxylées, la N-(2-hydroxyéthyl)cyclohexylamine (CAS : 2842-38-8), la *N,N*-bis(2-hydroxyéthyl)cyclohexylamine (CAS : 4500-29-2), N-(2-hydroxypropyl)cyclohexylamine (CAS : 103-00-4) N,N-bis(2-hydroxypropyl)cyclohexylamine (CAS : 14548-72-2) ainsi que leurs mélanges ;
- les aminoalcools hétérocycliques tels que 1-amino-2,5-anhydro-1,3,4-trideoxyhexitol (CAS : 589-14-0), le 5-(aminomethyl)-2-furanmethanol (CAS : 88910-22-9) ainsi que leurs mélanges ; les aminoalcools aromatiques tels que *N*-phényldiéthanolamine (CAS : 120-07-0), la *N*-phényldipropanolamine (CAS : 3077-13-2), la *N*-(p-tolyl)diéthanolamine (CAS : 3077-12-1), *N*-(p-tolyl)dipropanolamine (CAS : 38668-48-3) ainsi que leurs mélanges ;
- les alcanolamides linéaires ou ramifiées, cycliques ou non, saturées ou insaturées, dérivées des acides carboxyliques correspondants précédemment cités telles que la N-(2-hydroxyéthyl)formamide (CAS : 693-06-1), *N,N*-Bis(2-hydroxyethyl)formamide (CAS : 25209-66-9), la *N*-(2-hydroxyéthyl)acétamide (CAS : 142-26-7), la *N,N*-Bis(2-hydroxyéthyl)acétamide (CAS : 7435-67-8), la *N,N*-bis(2-hydroxyéthyl)-2-éthylhexanamide (CAS: 114214-77-6), la *N*-(2-hydroxyéthyl)octanamide (CAS: 7112-02-9), la *N,N*-Bis(2-hydroxyéthyl)octanamide (CAS : 3077-30-3), la *N*-(2-hydroxypropyl)octanamide (CAS : 23054-60-6), la *N,N*-Bis(2-hydroxypropyl)octanamide (CAS : 106476-15-7), la N-(2-Hydroxyethyl)octanamide (CAS: 7112-02-9), la *N,N*-bis(2-hydroxyéthyl)décanamide (CAS: 136-26-5), la N-(2-hydroxypropyl)octanamide (CAS : 23054-60-6), la *N,N*-Bis(2-hydroxypropyl)décanamide (CAS : 16516-34-0), la *N*-(2-hydroxéthyl)undecanamide (CAS : 28245-87-6), la *N,N*-Bis(2-hydroxyethyl)undecanamide (CAS: 45233-61-2), la N-(2-hydroxypropyl)undécanamide (CAS : 45205-25-2), la *N,N*-Bis(2-hydroxypropyl)undécanamide (CAS : 54914-38-4), la N-(2-hydroxyéthyl)-10-undécènamide (CAS : 20545-92-0), la *N,N*-Bis(2-hydroxyéthyl)undécènamide (CAS : 25377-64-4), la N-(2-Hydroxypropyl)-10-undécènamide (CAS : 68052-41-5), la *N*-(2-hydroxyéthyl)dodécanamide (CAS : 142-78-9), la *N,N*-Bis(2-hydroxyéthyl)dodécanamide (CAS : 120-40-1), la N-(2-hydroxypropyl)dodécanamide (CAS : 142-54-1), (CAS : 45233-61-2), *N,N*-Bis(2-hydroxypropyl)dodécanamide (CAS : 54914-38-4), la N-(2-hydroxyéthyl)tétradécanamide (CAS : 142-58-5), la N,N-Bis(2-hydroxyéthyl)tétradécanamide (CAS : 7545-23-5), la N-(2-hydroxypropyl)tétradécanamide (CAS 10525-14-1), *N,N*-Bis(2-hydroxypropyl)tétradécanamide (CAS : 16516-36-2), la N-(2-hydroxyéthyl)hexadécanamide (CAS : 544-31-0), la N,N-Bis(2-hydroxyéthyl)hexadécanamide (CAS : 7545-24-6), la N-(2-hydroxypropyl)hexadécanamide (CAS 18738-25-5), *N,N*-Bis(2-hydroxypropyl)hexadécanamide (CAS : 16516-37-3), la N-(2-hydroxyéthyl)octaécanamide (CAS : 111-57-9), la N,N-Bis(2-hydroxyéthyl)octadécanamide (CAS : 93-82-3), la N-(2-hydroxypropyl)octadécanamide (CAS 35627-96-4), *N,N*-Bis(2-hydroxypropyl)octadécanamide (CAS : 17097-52-8), la N-(2-hydroxyéthyl)eicosanamide (CAS : 94421-69-9), la N,N-Bis(2-hydroxyéthyl)eicosanamide (CAS : 117841-46-0), les alcanolamides dérivés d'acides gras comme les AMINON et AMIDET de la société KAO, les alcanolamides alkoxylées ainsi que leurs mélanges.
- les alcanolamides hétérocycliques telles que la *N*-(2-hydroxyéthyl)-2-furancarboxamide (CAS: 107973-15-9), la N,N-bis(2-hydroxyéthyl)-2-furancarboxamide (CAS: 1226164-87-9, la *N*-(2-hydroxypropyl)-2-furancarboxamide (CAS : 66671-73-6), la N,N-bis(2-hydroxypropyl)-2-furancarboxamide (CAS : 1226036-86-7) ainsi que leur mélanges ;
- les alcanolamides aromatiques telles que la *N*-(2-hydroxyéthyl)benzamide (CAS : 18838-10-3), la *N,N*-Bis(2-hydroxyethyl)benzamide (CAS: 58566-44-2), la N-(2-hydroxypropyl)benzamide (CAS : 23054-66-2), la *N,N*-bis(2-hydroxypropyl)benzamide (CAS : 422278-61-3) ainsi que leurs mélanges.

Les composés de formule (VII) peuvent être ceux choisis parmi :
- les acides carboxyliques linéaires ou ramifiés, cycliques ou non, saturés ou insaturés, tels que l'acide formique (C1), l'acide acétique (C2), l'acide propionique (C3), l'acide acrylique (C3:1), l'acide butyrique (C4), l'acide méthacrylique (C4:1), l'acide valérique (C5), l'acide isovalérique (C5), l'acide caproïque (C6), l'acide heptanoique (C7), l'acide cyclohexanoique (C7), l'acide caprylique (C8), l'acide éthyl-2-hexanoique, l'acide pélargonique (C9), l'acide caprique (C10), l'acide 11-undécylénique (C11), l'acide laurique (C12), l'acide lauroléique (C12:1), l'acide myristique (C14), l'acide myristoléique (C14:1), l'acide palmitique (C16), l'acide isopalmitique (C16), l'acide palmitoléique (C16:1), l'acide isomargarique (C17), l'acide antéisomargarique (C17), l'acide stéarique (C18), l'acide isostéarique (C18), l'acide antéisostéarique (C18), l'acide oléique (C18:1), l'acide élaidique (C18 :1), l'acide vaccénique (C18:1), l'acide linoléique (C18:2), l'acide linolénique (C18:3), l'acide eicosanoique ou arachidique (C20), l'acide gadoléique (C20:1), l'acide gondoique (C20:1), l'acide arachidonique (C20:4), l'acide béhénique (C22), l'acide cétoléique (C22:1), l'acide lignocérique (C24), l'acide cérotique (C24), les acides naphténiques ainsi que leurs mélanges ;
- les acides carboxyliques oligoméres linéaires en C14 à C60 tel que l'UNICID® acid commercialisé par la société BAKER HUGHES ;
- les acides carboxyliques terpéniques ou résiniques tels que l'acide abiétique, l'acide dehydroabiétique, l'acide néoabiétique, l'acide palustrique, l'acide pimarique, l'acide isopimarique, l'acide lévopimarique ainsi que leurs mélanges présents dans les acides gras de tall-oil ;
- les acides carboxyliques aromatiques tels que l'acide benzoique (C7), l'acide naphtoique (C11) ainsi que leurs mélanges ;
- les acides carboxyliques hétérocycliques tel que l'acide furanoique, l'acide tétrahydrofuranoique ainsi que leurs mélanges ;
- les acides carboxyliques (poly)éthers tel que l'acide 2-(2-méthoxyéthoxy)acétique, l'acide (2-butoxyéthoxy)acétique, l'acide méthoxypoly(éthylène glycol) acétique, l'acide méthoxypoly(éthylène glycol) propionique disponibles chez SIGMA-ALDRICH ainsi que leurs mélanges.

Les composés de formule (III-A) sont notamment obtenus à partir d'un composé de formule (V-A) suivante : 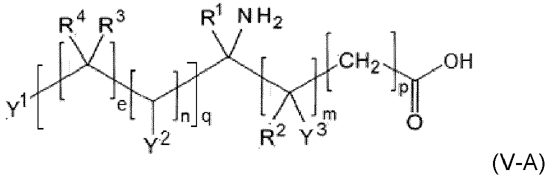
- R¹, R², R³, R⁴, n, m, p, q et e sont tels que définis précédemment dans la formule (V),
- Y¹ représente F¹ tel que défini précédemment dans la formule (III) ou -Ph-XH, ou -XH avec X représentant O ou S ou Se et Ph représentant un groupe phényle;
- Y² représente F² tel que défini précédemment dans la formule (III) ou -Ph-XH, ou -XH avec X représentant O ou S ou Se et Ph représentant un groupe phényle;
- Y³ représente F³ tel que défini précédemment dans la formule (III) ou -Ph-XH, ou -XH avec X représentant O ou S ou Se et Ph représentant un groupe phényle;
   à condition qu'au plus un des radicaux Y¹, Y² ou Y³ représente un radical -Ph-XH ou -XH avec X représentant O ou S ou Se et Ph représentant un radical phényle.

Les composés de formule (V-A) peuvent se présenter sous la forme d'un énantiomère L, d'un énantiomère D ou sous la forme d'un racémique.

Parmi les composés de formule (V-A), on peut par exemple citer l'arginine, l'histidine, l'acide aspartique, l'acide glutamique, l'acide 2-aminoadipique (CAS : 542-32-5), l'acide 2-aminopimélique (CAS : 627-76-9), l'acide 2-aminooctanedioïque (CAS : 3054-07-7), l'aspargine, la glutamine, l'alanine, la thréonine, l'acide α-amino-β-hydroxy-β-methyl-benzenebutanoique (CAS : 93505-16-9), l'acide 2,12-diaminododécanoïque, la norvaline, l'isoleucine, la leucine, la 3-hydroxy isoleucine, la 4-hydroxy isoleucine, la 5-hydroxy isoleucine, la 3-mercapto isoleucine, la méthionine, la phénylalanine, le tryptophane, la glycine, l'acide 11-aminoundécanoique, l'acide 12-aminododécanoique, la sérine, la valine, le 3-hydroxy-4-phényl valine, la norvaline, la 3-hydroxy isoleucine, la 3-mercapto isoleucine, la méthionine, la phénylalanine, la 2-méthyl statine, la cystéine, la sélénocysteine, l'isoleucine, l'acide 2-aminohentriacontanoïque, la tyrosine, l'acide 4-amino-3-hydroxybutyrique, la statine, l'isostatine et la phénylstatine.

Les composés de formule (III-B) sont notamment obtenus à partir d'un composé de formule (V-B) suivante : 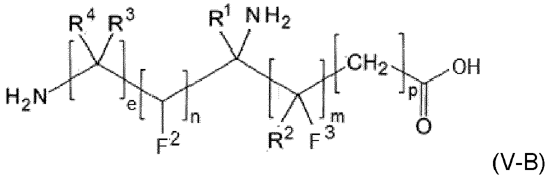 dans laquelle R¹, R², R³, R⁴, F², F³, n, m, p et e sont tels que définis précédemment dans la formule (V).

Les composés de formule (V-B) peuvent se présenter sous la forme d'un énantiomère L, d'un énantiomère D ou sous la forme d'un racémique.

Parmi les composés de formule (V-B), on peut par exemple citer la lysine et l'homolysine.

Les composés de formule (III-C) sont notamment obtenus à partir d'un composé de formule (V-C) suivante : 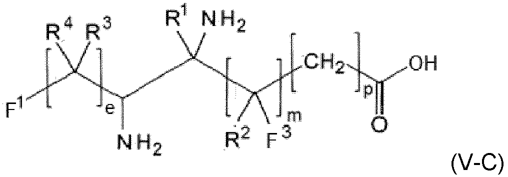 dans laquelle R¹, R², R³, R⁴, F¹, F³, m, p et e, tels que définis précédemment dans la formule (V).

Les composés de formule (V-C) peuvent se présenter sous la forme d'un énantiomère L, d'un énantiomère D ou sous la forme d'un racémique.

Parmi les composés de formule (V-C), on peut par exemple citer la 3-aminonorvaline (CAS : 80573-35-9), l'acide α,β-diaminobenzenebutanoique (CAS : 70984-76-8) et l'acide 2,3-diaminobutanoique (CAS : 2643-66-5).

Les composés de formule (III-D) sont notamment obtenus à partir d'un composé de formule (V-D) suivante : 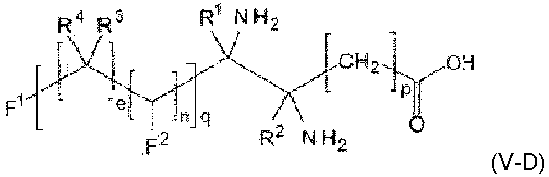 dans laquelle R¹, R², R³, R⁴, F¹, F², n, p, q et e, sont tels que définis précédemment dans la formule (V).

Les composés de formule (V-D) peuvent se présenter sous la forme d'un énantiomère L, d'un énantiomère D ou sous la forme d'un racémique.

Parmi les composés de formule (V-D), on peut par exemple citer l'acide 2,3-diaminopropionique (CAS : 515-94-6), l'acide 3,4-diaminobutanoique (CAS : 131530-16-0) et la β-aminophenylalanine (CAS : 64765-83-9).

### B.2. Précurseurs aldimines de formule (III')

Les composés de formule (II) susmentionnée peuvent être obtenus par réaction entre :
- un composé de formule (III') suivante : 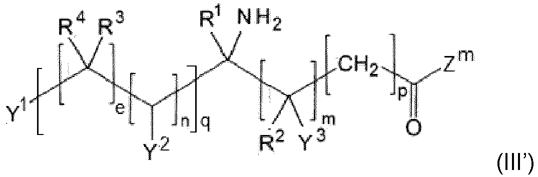 dans laquelle :
   - Y¹ représente F¹ ou -Ph-XH ou -XH avec X représentant O ou S ou Se et Ph représentant un groupe phényle;
   - Y² représente F² ou -Ph-XH ou -XH avec X représentant O ou S ou Se et Ph représentant un groupe phényle;
   - Y³ représente F³ ou -Ph-XH ou -XH avec X représentant O ou S ou Se et Ph représentant un groupe phényle;
      à condition qu'au maximum un seul radical parmi les radicaux Y¹, Y² et Y³ représente -Ph-XH ou -XH ;
      à condition que quand Y¹ = -XH ou -Ph-XH, alors Y² = F² et Y³ = F³ ;
      à condition que quand Y² = -XH ou -Ph-XH, alors Y¹ = F¹ et Y³ = F³ ;
      à condition que quand Y³ = -XH ou -Ph-XH, alors Y¹ = F¹ et Y² = F² ;
   - F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe -COOZ^{m}, un groupe -C(O)NH₂, un groupe -SMe, ou un groupe guanidyl de formule -NH-C(=NH)-NH₂,
   - F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe -COOZ^{m}, un groupe -C(O)NH₂, un groupe -SMe, ou un groupe guanidyl de formule -NH-C(=NH)-NH₂,
   - F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe -COOZ^{m}, un groupe -C(O)NH₂, un groupe -SMe, ou un groupe guanidyl de formule -NH-C(=NH)-NH₂,
   - R¹, R², R³ et R⁴ représente chacun, indépendamment les uns des autres, un radical choisi parmi un atome d'hydrogène, un groupe alkyle linéaire ou ramifié, comprenant de 1 à 20 atomes de carbone, ledit groupe alkyle comprenant éventuellement un ou plusieurs hétéroatomes, un groupe (hétéro)cycloalkyle comprenant de 3 à 20 atomes de carbone, un groupe arylalkyle comprenant de 7 à 20 atomes de carbone ou un groupe (hétéro)aryle comprenant de 5 à 12 atomes de carbone ;
   - n est un nombre entier allant de 0 à 28 ;
   - p est un nombre entier allant de 0 à 10 ;
   - m représente 0 ou 1 ;
   - q représente 0 ou 1 ;
   - e est un nombre entier allant de 1 à 3 ;
   - Z^{m} représente un radical organique monovalent ayant une masse moléculaire moyenne en nombre (Mn) allant de 16 à 22 000 g/mol;
- et un composé de formule (VIII) suivante : 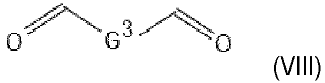 dans laquelle G³ est tel que défini précédemment dans la formule (II) ci-dessus.

La réaction entre un composé de formule (III') susmentionnée et un composé de formule (VIII) susmentionnée peut être réalisée selon un mode opératoire similaire à celui utilisé (exemple 15) dans US 5,087,661, soit par entrainement azéotropique de l'eau formée à environ 90°C en présence de toluène comme solvant de réaction.

Le composé de formule (III') peut être utilisé en excès par rapport au composé de formule (VIII).

De préférence, les composés de formule (III') sont ceux dans laquelle :
- m représente 0 ou 1 ;
- p représente 0 ou 1 ;
- n représente 0, 1, 2, 3 ou 4 ;
- q représente 0 ou 1 ;
- F¹ représente un radical choisi parmi un atome d'hydrogène, un groupe (hétéro)aryle comprenant de 4 à 12 atomes de carbone, un groupe -COOZ^{m}, un groupe -C(O)NH₂, un groupe -SMe, ou un groupe guanidyl de formule -NH-C(=NH)-NH₂,
- F² représente un radical choisi parmi un atome d'hydrogène ou un groupe alkyle linéaire ou ramifié comprenant de 1 à 10 atomes de carbone,
- F³ représente un atome d'hydrogène;
- R¹, R², R³ et R⁴ représente chacun, indépendamment les uns des autres, un atome d'hydrogène ou un groupe alkyle linéaire ou ramifié comprenant de 1 à 10 atomes de carbone, encore plus préférentiellement de 1 à 5 atomes de carbone ou un groupe benzyle, ou un groupe phényle.

Les composés de formule (III') peuvent avoir l'une des formules (III'-1), (III'-2) ou (III'-3) suivantes : 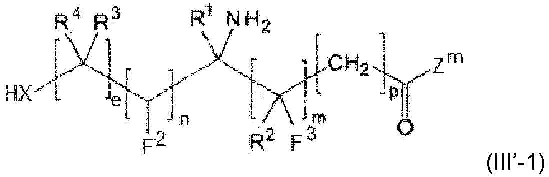 dans laquelle X, R¹, R², R³, R⁴, X, F², F³, n, m, p, e et Z^{m} sont tels que définis précédemment dans la formule (III') ; 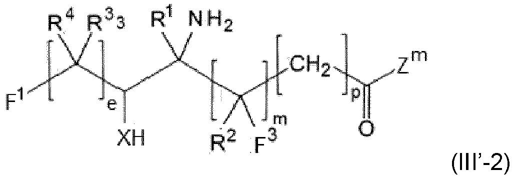 dans laquelle X, R¹, R², R³, R⁴, X, F¹, F³, m, p, e et Z^{m} sont tels que définis précédemment dans la formule (III'), 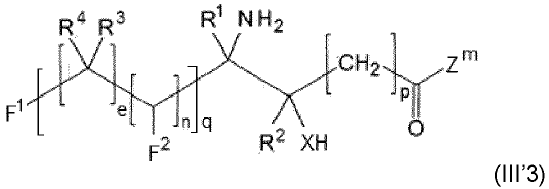 dans laquelle X, R¹, R², R³, R⁴, X, F¹, F², n, p, q, e et Z^{m} sont tels que définis précédemment dans la formule (III').

### Composés de formule (VIII)

Les composés de formule (VIII) peuvent avoir l'une des formules (VIII-A) ou (VIII-B) suivantes : 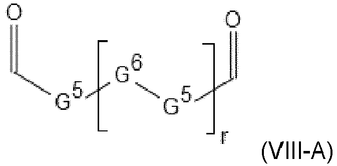 dans laquelle :
- G⁵ représente un radical (hétéro)arylène éventuellement substitué ;
- r représente 0 ou 1 ;
- à condition que quand r = 0, alors G⁵ représente un radical hétéroarylène éventuellement substitué ;
- G⁶ représente un atome d'oxygène, un atome de soufre ou un radical choisi parmi l'un des radicaux suivants : -O-R²⁷-O-, -CH₂-O-R²⁸-O-CH₂-, -CH₂-O-C(=O)-R²⁹-C(=O)-O-CH₂-, -CH₂-O-C(=O)-NH-R³⁰-NH-C(=O)-O-CH2-, -O-C(=O)-NH-R³¹-NH-C(=O)-O-, -O-C(=O)-R¹²-C(=O)-O-, -C(=O)-O-G⁹-O-C(=O)-, et -C(=O)-NR¹⁶-G⁷-NR¹⁶-C(=O)-, avec R²⁷, R²⁸, R²⁹, R³⁰, R³¹ et R³² représentent indépendamment les uns des autres un radical hydrocarboné comprenant éventuellement au moins un hétéroatome ; et G⁹ représentant un radical hydrocarboné divalent comprenant de 1 à 15 atomes de carbone, comprenant éventuellement au moins un hétéroatome ;
le radical G⁵-[G⁶-G⁵]r ayant une masse molaire ou de masse moléculaire moyenne en nombre (Mn) allant de 14 à 4 000 g/mol, de préférence allant de 60 à 2 000 g/mol, préférentiellement allant de 60 à 1 000 g/mol et encore plus préférentiellement allant de 60 à 500 g/mol, 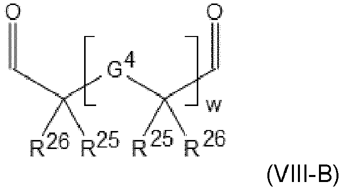 dans laquelle :
- R²⁵ et R²⁶ représente chacun, indépendamment l'un de l'autre, un radical hydrocarboné monovalent comprenant de 1 à 12 atomes de carbone, ledit radical étant éventuellement substitué par un groupe -OH ;
   ou R²⁵ et R²⁶ forment ensemble un cycle aliphatique comprenant de 4 à 12 atomes de carbone, ledit cycle étant éventuellement substitué ;
- G⁴ représente une liaison carbone-carbone ou un radical hydrocarboné divalent, ledit radical comprenant éventuellement au moins un hétéroatome ; et
- w représente un nombre entier égal à 0 ou 1.

Les composés de formule (VIII-A) peuvent avoir l'une des formules (VIII-A-1), (VIII-A-2), (VIII-A-3), (VIII-A-4), (VIII-A-5),(VIII-A-6), (VIII-A-7) et (VIII-A-8) suivantes : 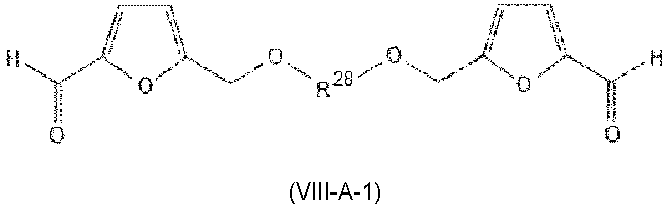 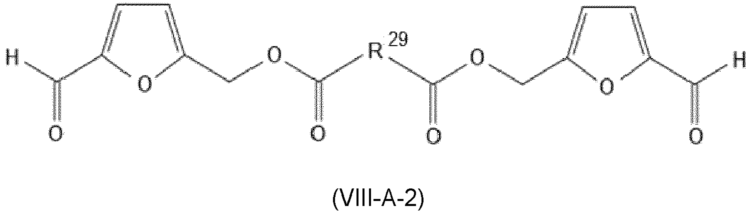 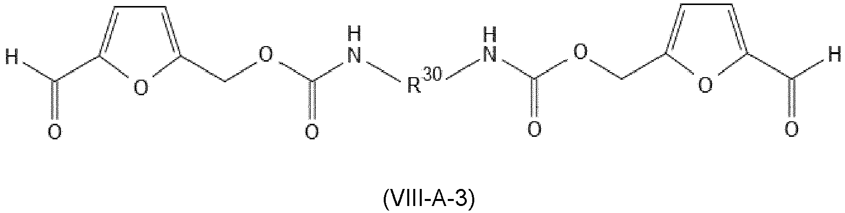 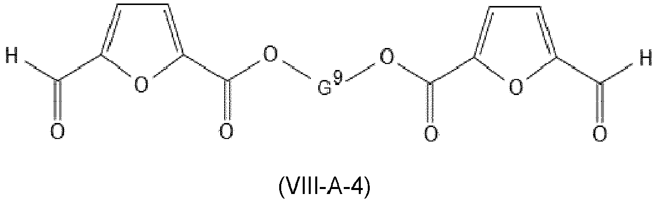 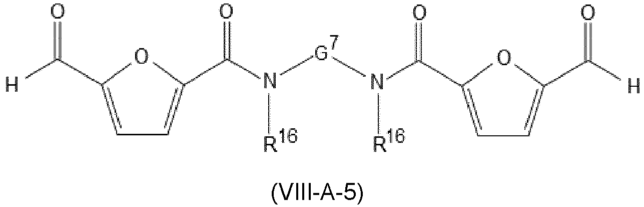 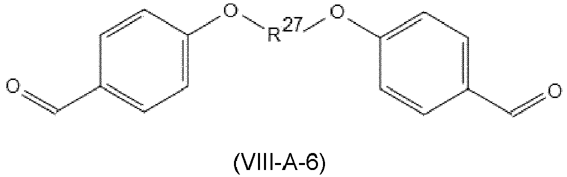 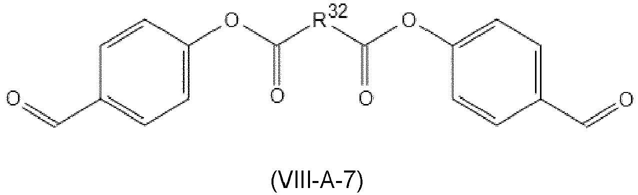 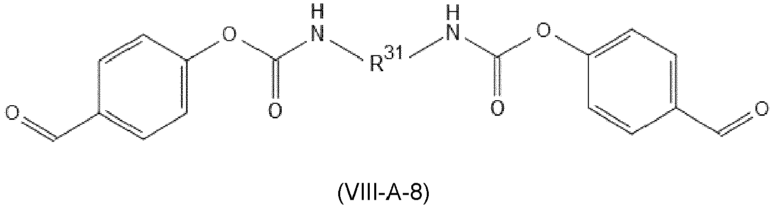 dans lesquelles R²⁷, R²⁸, R²⁹, R³⁰, R³¹ et R³² sont tels que définis précédemment.

Parmi les composés de formule (VIII-A), on peut par exemple citer le phtalaldéhyde, l'isophtalaldéhyde, le téréphtalaldéhyde, le 9,10-anthracènedicarbaldéhyde, le 2,3-naphtalènedicarboxaldéhyde, le 1,4 bis(4-formylphényl ester) butanedioique acide (CAS : 163463-91-0), le 4,4'-[1,2-ethaneduylbis(oxy)bis[benzaldéhyde] (CAS: 34074-28-7), le carbamic acid 1,6-hexanediylbis-, bis(4-formylphényl)ester (CAS: 99562-92-2) et leurs isomères.

Les composés de formule (VIII-B) ont de préférence l'une des formules (VIII-B-1), (VIII-B-2), (VIII-B-3), (VIII-B-4), (VIII-B-5), (VIII-B-6), (VIII-B-7) suivantes : 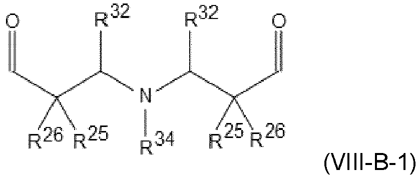 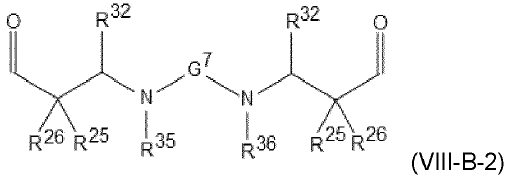 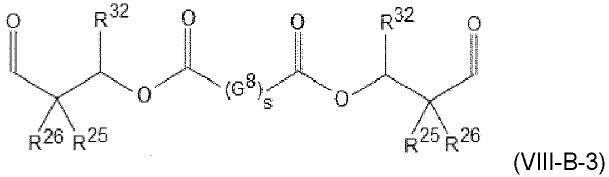 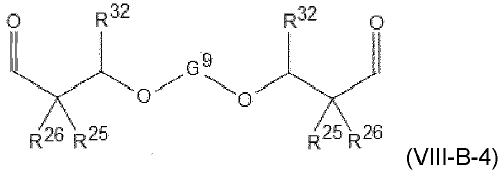 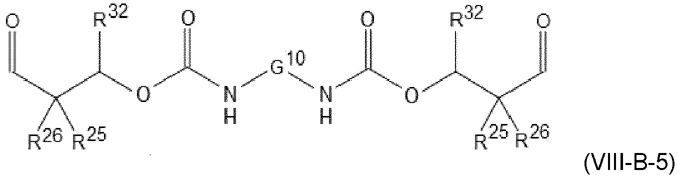 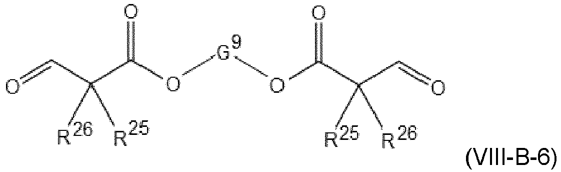 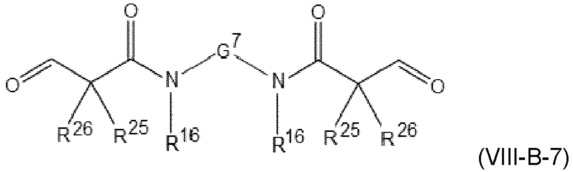 dans lesquelles:
- R²⁶ et R²⁵ représente chacun, indépendamment l'un de l'autre, un radical hydrocarboné monovalent comprenant de 1 à 12 atomes de carbone, ledit radical étant éventuellement substitué par un groupe -OH ;
   ou R²⁶ et R²⁵ forment ensemble un cycle aliphatique comprenant de 4 à 12 atomes de carbone, ledit cycle étant éventuellement substitué ;
- R³² représente un hydrogène, un radical alkyle linéaire ou ramifié, un radical cycloalkyle, un radical arylalkyle, ou un radical alcoxycarbonyle comprenant de 1 à 12 atomes de carbone ;
- R³⁴ représente un atome d'hydrogène, un radical alkyle linéaire ou ramifié, un radical cycloalkyle ou un radical arylalkyle ;
- R³⁵ représente un atome d'hydrogène, un radical alkyle linéaire ou ramifié, un radical cycloalkyle ou un radical arylalkyle ;
- R³⁶ représente un atome d'hydrogène, un radical alkyle linéaire ou ramifié, un radical cycloalkyle ou un radical arylalkyle ;
- R³⁷ représente un atome d'hydrogène, un radical alkyle linéaire ou ramifié, un radical cycloalkyle ou un radical arylalkyle ;
- G⁷ représente un groupe carbonyle ou un groupe alkylène linéaire ou ramifié comprenant de 2 à 15 atomes de carbone comprenant éventuellement un atome d'oxygène ;
- G⁸ représente un radical hydrocarboné divalent comprenant de 1 à 15 atomes de carbone, comprenant éventuellement au moins un hétéroatome ;
- G⁹ représente un radical hydrocarboné divalent comprenant de 1 à 15 atomes de carbone, comprenant éventuellement au moins un hétéroatome ;
- G¹⁰ représente un radical hydrocarboné divalent comprenant de 1 à 15 atomes de carbone, comprenant éventuellement au moins un hétéroatome ;
- s représente 0 ou 1.

Les composés de formule (VIII-B-1), (VIII-B-2), (VIII-B-3), (VIII-B-4), (VIII-B-5), (VIII-B-6), (VIII-7) peuvent être préparés selon les procédés décrits dans US 2011/0214810.

Parmi les composés de formule (VIII-B-1), on peut par exemple citer le 3,3'-(méthylimino)bis[2,2-diméthyl]propanal (CAS : 41348-50-9).

Parmi les composés de formule (VIII-B-2), on peut par exemple citer le 4,4'-[1,2-ethanediylbis(methylimino)bis[3,3-dimethyl]-2butanone (CAS : 89685-94-9), ou le α,α,α,α-tétraméthyl-1,4-pipérazine dipropanal (CAS : 92370-25-7).

### Composés de formule (III')

Les composés de formule (III') peuvent être obtenus par un procédé comprenant les étapes suivantes :
i) étape de réaction entre :
   - un composé de formule (V') suivante ou l'un de ses dérivés: 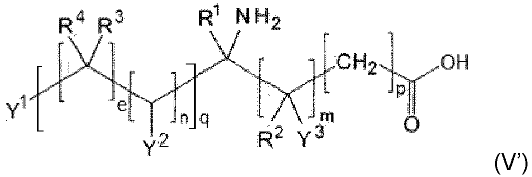 dans laquelle :
      - Y¹ représente F¹ ou -Ph-XH ou -XH avec X représentant O ou S ou Se et Ph représentant un groupe phényle;
      - Y² représente F² ou -Ph-XH ou -XH avec X représentant O ou S ou Se et Ph représentant un groupe phényle;
      - Y³ représente F³ ou -Ph-XH ou -XH avec X représentant O ou S ou Se et Ph représentant un groupe phényle ;
         à condition qu'au maximum un seul radical parmi les radicaux Y¹, Y² et Y³ représente -Ph-XH ou -XH ;
         à condition que quand Y¹ = -Ph-XH ou -XH, alors Y² = F² et Y³ = F³ ;
         à condition que quand Y² = -Ph-XH ou -XH, alors Y¹ = F¹ et Y³ = F³ ;
         à condition que quand Y³ = -Ph-XH ou -XH, alors Y² = F² et Y¹ = F¹ ;
      - F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOH, un groupe -C(O)NH₂, un groupe -SMe, ou un groupe guanidyl de formule -NH-C(=NH)-NH₂,
      - F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOH, un groupe -C(O)NH₂, un groupe -SMe, ou un groupe guanidyl de formule -NH-C(=NH)-NH₂,
      - F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOH, un groupe -C(O)NH₂, un groupe -SMe, ou un groupe guanidyl de formule -NH-C(=NH)-NH₂,
      - R¹, R², R³ et R⁴ représente chacun, indépendamment les uns des autres, un radical choisi parmi un atome d'hydrogène, un groupe alkyle linéaire ou ramifié, comprenant de 1 à 20 atomes de carbone, ledit groupe alkyle comprenant éventuellement un ou plusieurs hétéroatomes, un groupe (hétéro)cycloalkyle comprenant de 3 à 20 atomes de carbone, un groupe arylalkyle comprenant de 7 à 20 atomes de carbone ou un groupe (hétéro)aryle comprenant de 5 à 12 atomes de carbone ;
      - n est un nombre entier allant de 0 à 28 ;
      - m représente 0 ou 1 ;
      - p est un nombre entier allant de 0 à 10 ;
      - q représente 0 ou 1 ;
      - e est un nombre entier allant de 1 à 3 .
   - et un composé de formule (VI') suivante :

      Z'-(X'H)ₓ (VI')

      dans laquelle :
      - Z' représente un atome d'hydrogène ou un radical organique monovalent Z'^{m} ayant une masse molaire ou une une masse moléculaire moyenne en nombre (Mn) allant de 1 à 20 000 g/mol ;
      - X' représente O ou NR' avec R' représentant un atome d'hydrogène ou un groupe alkyle linéaire ou ramifié comprenant de 1 à 20 atomes de carbone pouvant éventuellement comprendre un ou plusieurs hétéroatomes, un groupe arylalkyle comprenant de 1 à 20 atomes de carbone, un groupe (hétéro)aryle comprenant de 4 à 12 atomes de carbone, ou un groupe (hétéro)cycloalkyle comprenant de 3 à 20 atomes de carbone ;
      - x représente 1 ;
ii) une éventuelle étape de déprotection lorsque l'étape i) est réalisée en présence d'un dérivé d'un composé de formule (V').

On entend par dérivé d'un composé de formule (V') susmentionnée, un composé de formule (V') dans laquelle le groupe -NH₂ est protégé par un groupement protecteur formant un groupe -NHP. Un dérivé d'un composé de formule (V') peut par exemple être un composé de formule (V') dans laquelle le(s) groupe(s) -NH₂ est(sont) protégé(s) sous forme -NH-Boc (Boc étant tertbutyloxycarbonyl).

Un dérivé d'un composé de formule (V') peut être par exemple préparé par réaction d'un composé de formule (V) avec le dicarbonate de di-tert-butyle pour protéger le(s) groupe(s) -NH₂ sous forme -NHBoc.

Un dérivé d'un composé de formule (V') peut être par exemple préparé par réaction d'un composé de formule (V') avec le tertbutyldimethylsilyl chloride dans la dimethylformamide en présence d'imidazole pour protéger le(s) groupe(s) -XH et -Ph-XH sous forme -X-TBDMS.

L'étape i) peut avoir lieu dans des quantités molaires telles que le ratio molaire -X'H / -C(=O)OH peut aller de 10 : 1 à 1 : 1.

Quand Z'(XH)x représente Z'(OH)x, la synthèse des aminoesters lors de l'étape i) peut être réalisée dans un solvant organique, par exemple choisi parmi les éthers (par exemple l'éther éthylique ou le tétrahydrofurane), les solvants chlorés (par exemple le dichlorométhane) ou directement dans Z'(OH)x en excès selon son point d'ébullition sous pression réduite tel que décrit dans Tetrahedron Letters (1986), 27 (41), pages 4975-4978 et dans Tetrahedron (1988), 44 (17), 5495-506 (1988).

Quand Z'(XH)x représente Z'(NHR')x, la synthèse des aminoamides, lors de l'étape i) peut être réalisée dans un solvant organique, par exemple choisi parmi les éthers (par exemple l'éther éthylique ou le tétrahydrofurane), les solvants chlorés (par exemple le dichlorométhane) tel que décrit dans Journal of Organic Chemistry (2006), 71(4), 1750-1753 à partir des aminoesters correspondants.

L'étape ii) de déprotection des fonctions -NH₂ sous forme -NHBoc peut être réalisée par toute méthode conventionnelle connue de l'homme du métier. Il peut par exemple s'agir d'une étape d'hydrolyse acide. Par exemple, il est possible de réaliser l'étape ii) en présence d'acide trifluoroacétique ou d'acide chlorhydrique.

L'étape ii) de déprotection des fonctions -XH et -Ph-XH sous forme -X-TBDMS peut être réalisée par toute méthode conventionnelle connue de l'homme du métier. Il peut par exemple s'agir d'une étape de coupure. Par exemple, il est possible de réaliser l'étape ii) en présence de fluorure de tetra-n-butylammonium (TBAF).

A titre de composés de formule (VI'), on peut par exemple citer :
- les monols linéairesou ramifiés, cycliques ou non, saturés ou insaturés, tels que le méthanol (CAS : 67-56-1), l'éthanol (CAS : 64-17-5), le propanol (CAS : 71-23-8), l'isopropanol (CAS : 67-63-0), l'alcool allylique (CAS : 107-18-6), le 1-butanol (CAS : 71-36-3), l'isobutanol (CAS : 78-83-1), le 1-pentanol ou alcool amylique (CAS : 71-41-0), le 3-méthyl-1-butanol ou alcool isoamylique (CAS : 123-51-3), le 2-pentanol (CAS : 6032-29-7), le 3-pentanol (CAS: 584-02-1), le 4-methyl-2-pentanol (CAS : 108-11-2), 5-phenyl-1-pentanol (CAS : 10521-91-2), le cyclopentanol (CAS : 96-41-3), le 1-hexanol (CAS :), le cyclohexanol (CAS : 108-93-0), le 2-butoxyethanol (CAS : 111-76-2), le 1-heptanol (CAS : 111-70-6), le 2-heptanol (CAS : 543-49-7), le 3-heptanol (CAS : 589-82-2), le 4-methyl-3-heptanol (CAS : 14979-39-6), le 3-methyl-4-heptanol (CAS : 1838-73-9), le 6-methyl-2-heptanol (CAS : 4730-22-7), le 6-methyl-3-heptanol (CAS : 18720-66-6), le 2-(2-(2-methoxyethoxy)ethoxy)ethanol (CAS : 112-35-6), l'alcool benzylique (CAS : 100-51-6), le 1-octanol-1 (CAS : 111-87-5), le 2-octanol (CAS : 123-96-6), le 3-octanol (CAS : 589-98-0), le 4-octanol (CAS : 589-62-8), le 2-éthyl-1-hexanol (CAS: 104-76-7), le 2-hexyloxyethanol (CAS: 112-25-4), le 2-(2-butoxyethoxy)ethanol (CAS : 112-34-5), le 1-nonanol (CAS : 143-08-8), le 2-nonanol (CAS : 628-99-9), le 3-nonanol (CAS : 624-51-1), le 4-nonanol (CAS : 5932-79-6), le 5-nonanol (CAS : 623-93-8), l'isononanol (CAS : 27458-94-2), le 2,6-dimethyl-2-heptanol (CAS : 13254-34-7)), le 1-octanol (CAS : 111-87-5), le 2-octanol (CAS : 123-96-6), le 3-octanol (CAS : 589-98-0), le 4-octanol (CAS : 589-62-8), le 2-methyl-3-octanol (CAS : 26533-34-6), le 2,2-dimethyl-3-octanol (CAS : 19841-72-6), le 3,5-dimethyl-4-octanol (CAS : 19781-12-5), le 3,6-dimethyl-3-octanol (CAS : 151-19-9), le 3-methyl-4-octanol (CAS : 26533-35-7), le 6-ethyl-3-octanol (CAS : 19781-27-2), le 3,7-dimethyl-1-octanol (CAS : 106-21-8), le 3,7-dimethyl-3-octanol ou Tetrahydrolinalool (CAS : 78-69-3), le 2-butyl-1-octanol (CAS : 3913-02-8), le 8-phenyl-1-octanol (CAS : 10472-97-6), le 3-methyl-3-octanol (CAS : 5340-36-3), le 3,6-dimethyl-2-heptanol (CAS : 1247790-47-1), 3,6,9,12-tetraoxotridecanol (CAS : 23783-42-8), le 1-nonanol (CAS : 143-08-8), le 2-nonanol (CAS : 628-99-9), le 3-nonanol (CAS : 624-51-1), le 4-nonanol (CAS : 5932-79-6), le 5-nonanol (CAS : 623-93-8), le 2-methyl-3-nonanol (CAS : 26533-33-5), le 2-methyl-4-nonanol (CAS : 26533-31-3), 2-pentyl-1-nonanol (CAS : 5333-48-2), le 3-methyl-4-nonanol (CAS : 26533-32-4), le 4-methyl-4-nonanol (CAS : 23418-38-4), le 5-ethyl-2-nonanol (CAS : 103-08-2), le 8-methyl-1-nonanol (CAS : 55505-26-5)), 4,8-dimethyl-1-nonanol (CAS: 33933-80-1), le 1-decanol (CAS: 112-30-1), le 2-decanol (CAS : 1120-06-5), le 3-decanol (CAS : 1565-81-7), le 2-hexyl-1-decanol (CAS : 2425-77-6), le 6-ethyl-3-decanol (CAS : 19780-31-5), l'isodécanol (CAS : 25339-17-7), le 2-propyl-1-heptanol (CAS : 10042-59-8), le cyclodécanol (CAS : 1502-05-2), le 1-undecanol (CAS : 112-42-5), le 2-undecanol (CAS : 1653-30-1), le 7-ethyl-2-methyl-4-undecanol (CAS : 103-20-8), le 10-undecénol (CAS : 112-43-6), le 2-undecénol (CAS : 75039-84-8), l'isoundécanol (CAS : 55505-28-7), le 1-dodécanol (CAS: 112-53-8), le 2-dodecanol (CAS: 10203-28-8), le 2-octyl-1-dodecanol (CAS : 5333-42-6), l'isododecanol (CAS : 25428-98-2), le 2,6,8-trimethyl-4-nonanol (CAS : 123-17-1), le 1-tridecanol (CAS : 112-70-9), le 7-tridecanol (CAS : 927-45-7), le 3,9-diethyl-6-tridecanol (CAS : 123-24-0), l'isotridecanol (CAS : 27458-92-0)), le 1-tétradécanol (CAS: 112-72-1), le 2-tetradecanol (CAS: 4706-81-4), le 2-decyl-1-tetradecanol (CAS : 58670-89-6), le 1-pentadecanol (CAS : 629-76-5), le 2-benzyl-1-heptanol (CAS : 92368-90-6), le 1-hexadécanol (CAS : 36653-82-4), le 2-hexadecanol (CAS : 14852-31-4), le 1-heptadecanol, (CAS : 1454-85-9), le 1-octadecanol (CAS : 112-92-5), le 9-octadecanol (CAS : 1454-85-9), le 1-isooctadecanol (CAS : 27458-93-1), le 1-eicosanol ou alcool arachidylique (CAS : 629-96-9), le 1-docosanol ou alcool béhénylique (CAS : 661-19-8), le 2-dodecyl-1-hexadecanol (C28), le 2-tetradecyl-1-octadecanol (CAS : 32582-32-4), l'abiétinol (CAS : 666-84-2), les alcools oligoméres linéaires allant de C14 à C60 (CAS : 71750-71-5) comme les UNILIN® alcohols commercialisé par la société BAKER HUGHES, les monols alkoxylés, les acides gras alkoxylés, et leurs mélanges ;
- les monoamines linéaires ou ramifiées, cycliques ou non, telles que la méthylamine (CAS : 74-89-5), la diméthylamine (CAS : 124-40-3), l'éthylamine (CAS : 75-04-7), la diéthylamine (CAS: 109-89-7), la propylamine (CAS: 107-10-8), la dipropylamine (CAS: 142-84-7), la diisopropylamine (CAS : 108-18-9), la butylamine (CAS : 109-73-9), la dibutylamine (CAS : 111-92-2), Di-sec-Butylamine (CAS: 626-23-3), la diisobutylamine (CAS: 110-96-3), la 1-pentylamine (CAS: 110-58-7), la morpholine (CAS: 110-91-8), la 1-amino-3-methylbutane (CAS : 107-85-7), la 1-aminohexane (CAS : 111-26-2), la cyclohexylamine (CAS : 108-91-8), la cyclohexaneméthanamine (CAS : 3218-02-8), la 2-ethyl-1-hexylamine (CAS : 104-75-6), la bis(2-ethylhexyl)amine (CAS : 106-20-7), la 1-aminoheptane (CAS : 1-Aminoheptane), 2-aminoheptane (CAS : 123-82-0), la 2-amino-6-methylheptane (CAS : 543-82-8), la benzylamine (CAS : 100-46-9), la furfurylamine (CAS : 617-89-0), la dibenzylamine (CAS : 103-49-1), 1-aminooctane (CAS: 111-86-4), la 2-aminooctane (CAS: 693-16-3), la dioctylamine (CAS : 1120-48-5), 1-aminononane (CAS : 112-20-9), la 1-aminodecane (CAS : 2016-57-1), la didecylamine (CAS: 1120-49-6), la 1-aminoundecane (CAS: 7307-55-3), la diundecylamine (CAS : 16165-33-6), la 1-aminododecane (CAS : 124-22-1), la didodecylamine (CAS : 3007-31-6), la 1-aminotridecane (CAS : 2869-34-3), la ditridecylamine (CAS : 5910-75-8), la 1aminotetradecane (CAS : 2016-42-4), la ditetradecylamine (CAS : 17361-44-3), la 1-aminopentadecane (CAS : 2570-26-5), la dipentadecylamine (CAS : 35551-81-6), la 1-aminohexadecane (CAS : 143-27-1), la dihexadecylamine (CAS : 16724-63-3), la 1-aminooctadecane (CAS : 124-30-1), la dioctadecylamine (CAS: 112-99-2), la 1-aminoeicosane (CAS: 10525-37-8), la dieicosylamine (CAS : 3071-00-9), les amines grasses primaires de coprah (CAS : 61788-46-3) comme par exemple les NORAM® C de la société ARKEMA, les amines grasses primaires de suif (CAS : 161544-60-1) comme par exemple les NORAM® S de la société ARKEMA, les amines grasses primaires oléique (CAS : 112-90-3) comme par exemple les NORAM® O de la société ARKEMA, les polyéther amines comme les JEFFAMINES M de la société HUNTSMAN ainsi que leurs mélanges ;
- les monoamines hétérocycliques telles que la furfurylamine ainsi que leurs mélanges.

Les composés de formule (V') peuvent se présenter sous la forme d'un énantiomère L, d'un énantiomère D ou sous la forme d'un racémique.

Parmi les composés de formule (V'), on peut par exemple citer l'arginine, l'histidine, l'acide aspartique, l'acide glutamique, l'acide 2-aminoadipique (CAS : 542-32-5), l'acide 2-aminopimélique (CAS : 627-76-9), l'acide 2-aminooctanedioïque (CAS : 3054-07-7), l'aspargine, la glutamine, l'alanine, la thréonine, l'acide α-amino-β-hydroxy-β-methyl-benzenebutanoique (CAS : 93505-16-9), l'acide 2,12-diaminododécanoïque, la norvaline, l'isoleucine, la leucine, la 3-hydroxy isoleucine, la 4-hydroxy isoleucine, la 5-hydroxy isoleucine, la 3-mercapto isoleucine, la méthionine, la phénylalanine, le tryptophane, la glycine, l'acide 11-aminoundécanoique, l'acide 12-aminododécanoique, la sérine, la valine, le 3-hydroxy-4-phényl valine, la norvaline, la 3-hydroxy isoleucine, la 3-mercapto isoleucine, la méthionine, la phénylalanine, la 2-méthyl statine, la cystéine, la sélénocysteine, l'isoleucine, l'acide 2-aminohentriacontanoïque, la tyrosine, l'acide 4-amino-3-hydroxybutyrique, la statine, l'isostatine et la phénylstatine.

### C. Utilisations des aldimines de formule (I) ou (II)

La présente invention concerne également l'utilisation des composés de formule (X) ou (XI) suivantescomme absorbeur d'humidité (« water-scavenger ») notamment dans une composition à base de polyuréthane, ou comme agent durcisseur dans une composition à base de résine époxy : 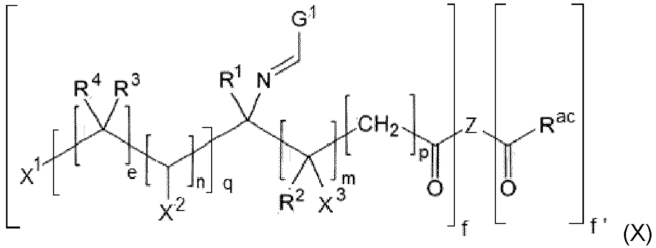 dans laquelle:
- X¹ représente F¹ ou -N=C(H)-G¹ ou -Ph-XH, ou -XH, avec X représentant O ou S ou Se et Ph représentant un groupe phényle;
- X² représente F² ou -N=C(H)-G¹ ou -Ph-XH, ou -XH, avec X représentant O ou S ou Se et Ph représentant un groupe phényle;
- X³ représente F³ ou -N=C(H)-G¹ ou -Ph-XH, ou -XH, avec X représentant O ou S ou Se et Ph représentant un groupe phényle;
   à condition qu'au maximum un seul radical parmi les radicaux X¹, X² et X³ représente -N=C(H)-G¹ ;
   à condition qu'au maximum un seul radical parmi les radicaux X¹, X² et X³ représente -XH ou -Ph-XH;
   à condition que quand X¹ = -N=C(H)-G¹ alors q = 1 ;
   à condition que quand X¹ = -N=C(H)-G¹ ou -Ph-XH ou -XH, alors X² = F² et X³ = F³ ;
   à condition que quand X² = -N=C(H)-G¹ ou -Ph-XH ou -XH, alors X¹ = F¹ et X³ = F³ ;
   à condition que quand X³ = -N=C(H)-G¹ ou -Ph-XH ou -XH, alors X² = F² et X¹ = F¹ ;
- F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- R¹, R², R³ et R⁴ représente chacun, indépendamment les uns des autres, un radical choisi parmi un atome d'hydrogène, un groupe alkyle linéaire ou ramifié, comprenant de 1 à 20 atomes de carbone, ledit groupe alkyle comprenant éventuellement un ou plusieurs hétéroatomes, un groupe (hétéro)cycloalkyle comprenant de 3 à 20 atomes de carbone, un groupe arylalkyle comprenant de 7 à 20 atomes de carbone ou un groupe (hétéro)aryle comprenant de 4 à 12 atomes de carbone ;
- n est un nombre entier allant de 0 à 28 ;
- p est un nombre entier allant de 0 à 10 ;
- m représente 0 ou 1 ;
- q représente 0 ou 1 ;
- e est un nombre entier allant de 1 à 3 ;
- f est un nombre entier ou non entier allant de 1 à 3, f étant de préférence un nombre entier égal à 1, 2 ou 3 ;
- f' est un nombre entier ou non entier allant de 0 à 2, f' étant de préférence un nombre entier égal à 0, 1 ou 2 ;
- la somme f + f' représente un entier variant de 1 à 3 ;
- Z représente un radical -organique monovalent Z^{m}, divalent Z^{d} ou trivalent Z^{t}, ayant une masse molaire ou une masse moléculaire moyenne en nombre (Mn) allant de 16 à 22 000 g/mol, de préférence de 16 à 12 000 g/mol, de préférence encore de 16 à 8 000 g/mol, encore plus préférentiellement de 16 à 4 000 g/mol ;
- R^{ac} représente un atome d'hydrogène ou un radical hydrocarboné monovalent comprenant de 1 à 60 atomes de carbone, ledit radical pouvant éventuellement comprendre un ou plusieurs hétéroatomes ;
- G¹ représente un radical hydrocarboné monovalent de masse molaire ou de masse moléculaire moyenne en nombre (Mn) allant de 15 à 4000 g/mol, de préférence de 60 à 2 000 g/mol, préférentiellement de 60 à 1 000 g/mol et encore plus préférentiellement de 60 à 500 g/mol, ledit radical pouvant éventuellement comprendre un ou plusieurs hétéroatomes; 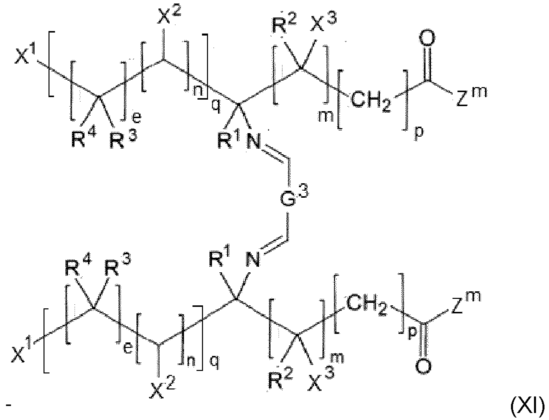
dans laquelle :
- X¹ représente F¹ ou -Ph-XH, ou -XH;
- X² représente F² ou -Ph-XH, ou -XH;
- X³ représente F³ ou -Ph-XH, ou -XH;
- F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- X, R¹, R², R³, R⁴, n, q, m, p, e et Z^{m} étant tels que définis pour la formule (I) ci-dessus ;
- G³ représente un radical hydrocarboné monovalent de masse molaire ou de masse moléculaire moyenne en nombre (Mn) allant de 14 à 4 000 g/mol, de préférence de 42 à 2 000 g/mol, préférentiellement de 42 à 1 000 g/mol et encore plus préférentiellement de 42 à 500 g/mol, ledit radical pouvant éventuellement comprendre un ou plusieurs hétéroatomes; à condition qu'au maximum un seul radical parmi les radicaux X¹, X² et X³ représente -XH ou -Ph-XH;
   à condition que quand X¹ = -Ph-XH ou -XH, alors X² = F² et X³ = F³ ;
   à condition que quand X² = -Ph-XH ou -XH, alors X¹ = F¹ et X³ = F³ ;
   à condition que quand X³ = -Ph-XH ou -XH, alors X² = F² et X¹ = F¹.

Les modes de réalisation, et modes préférés pour les différents radicaux des formules (I) et (II) s'appliquent pour les formules (X) et (XI) respectivement.

Selon un mode de réalisation, les composés de formule (X) sont les composés de formule (I) telle que décrite ci-dessus.

Selon un mode de réalisation, les composés de formule (XI) sont les composés de formule (II) telle que décrite précédemment.

La présente invention concerne une composition réticulable à l'humidité comprenant au moins un composé de formule (X) (de préférence de formule (I)) ou de formule (XI) (de préférence de formule (II)) telles que décrites ci-dessus.

La présente invention concerne notamment une composition C1 réticulable à l'humidité comprenant :
- au moins un composé de formule (X) (de préférence de formule (I)) ou (XI) (de préférence de formule (II)) telles que décrites ci-dessus ; et
- au moins un polymère P comprenant au moins deux fonctions terminales NCO ou au moins une résine époxy.

### Polymère P

Le polymère P peut être obtenu par toute méthode conventionnelle connue de l'homme du métier. Le polymère P est notamment obtenu par réaction de polyaddition entre une composition de polyol(s) et une composition de polyisocyanate(s), éventuellement en présence d'un catalyseur.

### Polyol(s)

La composition de polyol(s) susmentionnée peut être constituée d'un polyol ou d'un mélange de polyols.

Le(s) polyol(s) utilisable(s) peu(ven)t être choisi(s) parmi celui(ceux) possédant une masse molaire ou une masse moléculaire moyenne en nombre (Mn) allant de 60 g/mol à 22 000 g/mol, de préférence de 600 g/mol à 18 000 g/mol, de préférence 1 000 g/mol à 12 000 g/mol, de préférence de 1 000 à 8 000 g/mol et encore plus préférentiellement de 1 000 g/mol à 4 000 g/mol.

La masse moléculaire moyenne en nombre des polyols peut être calculée à partir de l'indice d'hydroxyle (IOH) exprimé en mg KOH/g et de la fonctionnalité du polyol ou déterminée par des méthodes bien connues de l'homme du métier, par exemple par chromatographie d'exclusion stérique (ou SEC en anglais) avec étalon PEG (polyéthylène glycol).

Les polyols peuvent avoir une fonctionnalité hydroxyle allant de 2 à 6, de préférence 2 à 3. Dans le cadre de l'invention, et sauf mention contraire, la fonctionnalité hydroxyle d'un polyol est le nombre moyen de fonction hydroxyle par mole de polyol.

Le(s) polyol(s) utilisable(s) peu(ven)t être choisi(s) parmi les polyester polyols, les polyéther polyols, les polydiène polyols, les polycarbonate polyols, les poly(éther-carbonate) polyols, les pré-polymères à terminaisons -OH, et leurs mélanges.

Le(s) polyol(s) utilisable(s) peu(ven)t être choisi(s) parmi les polyols aliphatiques, les polyols arylaliphatiques, les polyols aromatiques, les polyols carbonates et les mélanges de ces composés.

Selon l'invention, le(s) polyester polyol(s) peuvent avoir une masse moléculaire moyenne en nombre allant de 500 g/mol à 22 000 g/mol, de préférence de 700 g/mol à 10 000 g/mol et encore plus préférentiellement de 900 à 6 000 g/mol.

Parmi les polyester polyols, on peut par exemple citer :
- les polyesters polyols d'origine naturelle telle que l'huile de ricin ;
- les polyesters polyols résultant de la condensation :
   - d'un ou plusieurs polyols aliphatiques (linéaires, ramifiés ou cycliques) ou aromatiques tels que par exemple l'éthanediol, le 1,2-propanediol, le 1,3-propanediol, le glycérol, le triméthylolpropane, le 1,6-hexanediol, le 1,2,6-hexanetriol, le butènediol, le cyclohexanediméthanol, le sucrose, le glucose, le sorbitol, le glycérol, le triméthylolpropane, le pentaérythritol, le mannitol, la triéthanolamine, la N-méthyldiéthanolamine, et leurs mélanges, avec
   - un ou plusieurs acide polycarboxylique ou son dérivé ester ou anhydride tel que l'acide 1,6-hexanedioïque, l'acide dodécanedioïque, l'acide azélaïque, l'acide sébacique, l'acide adipique, l'acide 1,18-octadécanedioïque, l'acide phtalique, l'acide succinique et les mélanges de ces acides, un anhydride insaturé tel que par exemple l'anhydride maléique ou phtalique, ou une lactone telle que par exemple la caprolactone.

Les polyester polyols suscités peuvent être préparés de manière conventionnelle, et sont pour la plupart disponibles commercialement.

Parmi les polyesters polyols, on peut par exemple citer les produits suivants de fonctionnalité hydroxyle égale à 2 :
- le TONE® 0240 (commercialisé par UNION CARBIDE) qui est une polycaprolactone de masse moléculaire moyenne en nombre d'environ 2000 g/mol, et un point de fusion de 50°C environ,
- le DYNACOLL® 7381 (comercialisé par EVONIK) de masse moléculaire moyenne en nombre d'environ 3500 g/mol, et ayant un point de fusion de 65°C environ,
- le DYNACOLL® 7360 (commercialisé par EVONIK) qui résulte de la condensation de l'acide adipique avec l'hexane diol, et a une masse moléculaire moyenne en nombre d'environ 3500 g/mol, et un point de fusion de 55°C environ,
- le DYNACOLL® 7330 (commercialisé par EVONIK) de masse moléculaire moyenne en nombre d'environ 3500 g/mol, et ayant un point de fusion de 85°C environ,
- le DYNACOLL® 7363 (commercialisé par EVONIK) qui résulte également de la condensation de l'acide adipique avec l'hexane diol, et a une masse moléculaire moyenne en nombre d'environ 5500 g/mol, et un point de fusion de 57°C environ,
- le DYNACOLL® 7250 (commercialisé par EVONIK) : polyester polyol ayant une viscosité de 180 Pa.s à 23°C, une masse moléculaire moyenne en nombre Mn égale à 5 500 g/mol, et une T_{g} égale à -50°C,
- le KURARAY® P-6010 (commercialisé par KURARAY) : polyester polyol ayant une viscosité de 68 Pa.s à 23°C, une masse moléculaire moyenne en nombre égale à 6 000 g/mol, et une T_{g} égale à -64°C,
- le KURARAY® P-10010 (commercialisé par KURARAY) : polyester polyol ayant une viscosité de 687 Pa.s à 23°C, et une masse moléculaire moyenne en nombre égale à 10 000 g/mol.

Selon un mode de réalisation préféré, le polyester polyol est choisie parmi : une polycaprolactone ; l'huile de ricin ; un polyester polyol résultant de la condensation de l'éthylène glycol, du propylène glycol, du 1,3-propanediol et/ou du 1,6-hexanediol avec l'acide adipique et/ou les différents isomères de l'acide phtalique ; et leurs mélanges.

Selon l'invention, le(s) polyéther polyol(s) peuvent avoir une masse moléculaire moyenne en nombre allant de 200 g/mol à 22 000 g/mol, de préférence de 600 g/mol à 18 000 g/mol, de préférence 1 000 g/mol à 12 000 g/mol, de préférence 1 000 à 4 000 g/mol et encore plus préférentiellement de 1 000 g/mol à 8 000 g/mol.

De préférence, le(s) polyéther polyol(s) a(ont) une fonctionnalité hydroxyle allant de 2 à 3.

Le(s) polyéther polyol(s) utilisable(s) selon l'invention est (sont) de préférence choisi(s) parmi les polyoxyalkylène-polyol, dont la partie alkylène, linéaire ou ramifiée, comprend de 1 à 4 atomes de carbone, de préférence de 2 à 3 atomes de carbone.

Plus préférentiellement, le(s) polyéther polyol(s) utilisable(s) selon l'invention est (sont) de préférence choisi(s) parmi les polyoxyalkylène diols ou polyoxyalkylène triols, et mieux encore des polyoxyalkylène diols, dont la partie alkylène, linéaire ou ramifiée, comprend de 1 à 4 atomes de carbone, de préférence de 2 à 3 atomes de carbone.

A titre d'exemple de polyoxyalkylène diols ou triols utilisables selon l'invention, on peut par exemple citer :
- les polyoxypropylène diol ou triol (aussi désignés par polypropylène glycols (PPG) diol ou triol) ayant une masse moléculaire moyenne en nombre allant de 400 g/mol à 22 000 g/mol et de préférence allant de 400 g/mol à 12 000 g/mol,
- les polyoxyéthylène diol ou triol (aussi désignés par polyéthylène glycols (PEG) diol ou triol) ayant une masse moléculaire moyenne en nombre allant de 400 g/mol à 22 000 g/mol et de préférence allant de 400 g/mol à 12 000 g/mol,
- les polyoxybutylène glycols (aussi désignés par polybutylène glycols (PBG) diol ou triol) ayant une masse moléculaire moyenne en nombre allant de 200 g/mol à 12 000 g/mol,
- les copolymères ou terpolymères de PPG/PEG/PBG diol ou triol ayant une masse moléculaire moyenne en nombre allant de 400 g/mol à 22 000 g/mol et de préférence allant de 400 g/mol à 12 000 g/mol,
- les polytétrahydrofurane (PolyTHF) diol ou triol ayant une masse moléculaire moyenne en nombre allant de 250 g/mol à 12 000 g/mol,
- les polytétraméthylène glycols (PTMG) ayant une masse moléculaire moyenne en nombre allant de 200 g/mol à 12 000 g/mol,
- et leurs mélanges.

De préférence, le(s) polyéther polyol(s) utilisable(s) est (sont) choisi(s) parmi les polyoxypropylène diols ou triols. Les polyéther polyols mentionnés ci-dessus peuvent être préparés de manière conventionnelle, et sont largement disponibles dans le commerce. Ils peuvent par exemple être obtenus par polymérisation de l'oxyde d'alkylène correspondant en présence d'un catalyseur à base d'un double complexe métal-cyanure.

A titre d'exemples de polyéther diols, on peut citer les polyoxypropylène diols commercialisés sous la dénomination « ACCLAlM® » par la société COVESTRO, tels que l'« ACCLAIM® 18200 » de masse moléculaire moyenne en nombre voisine de 18 700 g/mol, l'« ACCLAIM® 12200 » de masse moléculaire moyenne en nombre voisine de 11 335 g/mol, l'« ACCLAlM® 8200 » de masse moléculaire moyenne en nombre voisine de 8 057 g/mol, et l'« ACCLAlM® 4200 » de masse moléculaire moyenne en nombre voisine de 4 020 g/mol, ou encore le polyoxypropylène diol commercialisé sous la dénomination « VORANOL P2000 » par la société DOW de masse moléculaire moyenne en nombre voisine de 2 004 g/mol.

A titre d'exemples de polyéther triols, on peut citer le polyoxypropylène triol commercialisés sous la dénomination « VORANOL CP3355 » par la société DOW, de masse moléculaire moyenne en nombre voisine de 3 554 g/mol.

Le(s) polydiène polyol(s) utilisable(s) selon l'invention peu(ven)t être choisi(s) de préférence parmi les polydiènes comportant des groupes hydroxyles terminaux, et leurs dérivés correspondants hydrogénés ou époxydés.

De préférence, le(s) polydiène polyol(s) utilisable(s) selon l'invention est (sont) choisi(s) parmi les polybutadiènes comportant des groupes hydroxyles terminaux, éventuellement hydrogénés ou époxydés. Préférentiellement, le(s) polydiène polyol(s) utilisable(s) selon l'invention est (sont) choisi(s) parmi les homopolymères et copolymères de butadiène comportant des groupes hydroxyles terminaux, éventuellement hydrogénés ou époxydés.

Dans le cadre de l'invention, et sauf mention contraire, on entend par « groupes hydroxyles terminaux » d'un polydiène polyol, les groupes hydroxyles situés aux extrémités de la chaîne principale du polydiène polyol.

Les dérivés hydrogénés mentionnés ci-dessus peuvent être obtenus par hydrogénation totale ou partielle des doubles liaisons d'un polydiène comportant des groupes hydroxyles terminaux, et sont donc saturé(s) ou insaturé(s).

Les dérivés époxydés mentionnés ci-dessus peuvent être obtenus par époxydation chémio sélective des doubles liaisons de la chaîne principale d'un polydiène comportant des groupes hydroxyles terminaux, et comportent donc au moins un groupe époxy dans sa chaîne principale.

A titre d'exemples de polybutadiène polyols, on peut citer les homopolymères de butadiène, saturé ou insaturé, comprenant des groupes hydroxyles terminaux, éventuellement époxydés, tels que par exemple ceux commercialisés sous la dénomination POLYBD® ou KRASOL® par la société CRAY VALLEY.

A titre d'exemple de polycarbonate diol, on peut citer le CONVERGE POLYOL 212-10 et CONVERGE POLYOL 212-20 commercialisés par la société NOVOMER respectivement de masse moléculaire en nombre (Mn) égales à 1000 et 2000 g/mol dont les indices hydroxyles sont respectivement de 112 et 56 mg KOH/g, le DESMOPHEN® C XP 2716 commercialisé par COVESTRO de masse moléculaire en nombre (Mn) égale à 326 g/mol dont l'indice hydroxyle est de 344 mg KOH/g, les POLYOL C-590, C1090, C-2090 et C-3090 commercialisés par KURARAY ayant une masse moléculaire en nombre (Mn) allant de 500 à 3000 g/mol et un indice hydroxyle allant de 224 à 37 mg KOH/g.

### Polyisocyanatel(s)

Dans le cadre de l'invention, on entend par « polyisocyanate », un composé comprenant au moins deux groupes isocyanates (NCO).

La composition de polyisocyanate(s) susmentionnée peut être constituée d'un polyisocyanate ou d'un mélange de polyisocyanates.

Le(s) polyisocyanate(s) utilisable(s) peu(ven)t être choisi(s) parmi ceux utilisés typiquement dans la synthèse d'un polyuréthane à terminaisons NCO.

Le(s) polyisocyanate(s) utilisable(s) peu(ven)t être aliphatique(s) (linéaire(s) ou ramifié(s)) ou aromatique(s), et éventuellement substitué(s).

De préférence, le(s) polyisocyanate(s) est(sont) choisi(s) parmi les diisocyanates, les triisocyanates, et leurs mélanges.

Selon un mode de réalisation préféré, le(s) polyisocyanate(s) est(sont) choisi(s) dans le groupe constitué du pentaméthylène diisocyanate (PDI), de l'hexaméthylène diisocyanate (HDI), de l'heptane diisocyanate, de l'octane diisocyanate, du nonane diisocyanate, du décane diisocyanate, de l'undécane diisocyanate, du dodécane diisocyanate, de l'isophorone diisocyanate (IPDI), du norbornane diisocyanate, du norbornène diisocyanate, du 1,4-cyclohexane diisocyanate (CHDI), du méthylcyclohexane diisocyanate, de l'éthylcyclohexane diisocyanate, du propylcyclohexane diisocyanate, du méthyldiéthylcyclohexane diisocyanate, du cyclohexane diméthylène diisocyanate, du 1,5-diisocyanato-2-méthylpentane (MPDI), du 1,6-diisocyanato-2,4,4-triméthylhexane, du 1,6-diisocyanato-2,2,4-triméthylhexane (TMDI), du 4-isocyanatomethyl-1,8-octane diisocyanate (TIN), du (2,5)-bis(isocyanatométhyl)bicyclo[2.2.1]heptane (2,5-NBDI), du (2,6)-bis(isocyanatométhyl)bicyclo[2.2.1]heptane (2,6-NBDI), du 1,3-bis(isocyanatométhyl)cyclohexane (1,3-H6-XDI), du 1,4-bis(isocyanatométhyl)-cyclohexane (1,4-H6-XDI), du toluène diisocyanate (TDI), du diphényleméthylène diisocyanate (MDI), du dicyclohexyleméthylène diisocyanate (H12-MDI), du xylylène-diisocyanate (XDI) (en particulier le méta-xylylène diisocyanate (m-XDI)), et de leurs mélanges.

Le MDI peut se présenter sous la forme d'un isomère ou d'un mélange d'isomères, tel que le 4,4'-MDI et/ou le 2,4'-MDI.

Le TDI peut se présenter sous la forme d'un isomère ou d'un mélange d'isomères, tel que le 2,4-TDI et/ou le 2,6-TDI.

Le(s) polyisocyanate(s) utilisable(s) pour préparer le polyuréthane utilisé selon l'invention sont typiquement largement disponibles dans le commerce. A titre d'exemple, on peut citer le SCURANATE® TX commercialisé par la société VENCOREX, correspondant à un 2,4-TDI de pureté de l'ordre de 95%, le SCURANATE® T100 commercialisé par la société VENCOREX, correspondant à un 2,4-TDI de pureté supérieure à 99% en poids, le DESMODUR® I » commercialisé par la société COVESTRO, correspondant à un IPDI, ou encore l'ISONATE® M125 commercialisé par DOW, correspondant à un MDI contenant au moins 97% en poids de l'isomère 4,4'-MDI.

Dans le cadre de l'invention, on entend de manière équivalente les expressions « réaction de polyaddition » et « polyaddition ».

L'étape de polyaddition peut être réalisée à une température inférieure à 95°C, de préférence allant de 60°C à 90°C, plus préférentiellement allant de 65°C à 80°C.

L'étape de polyaddition peut être réalisée dans des conditions anhydres, par exemple sous atmosphère d'azote.

L'étape de polyaddition peut être réalisée dans des quantités de polyisocyanate(s) et de polyol(s) tels que le rapport molaire NCO/OH est strictement supérieur à 1, par exemple compris entre 1,1 et 2,5, de préférence entre 1,1 et 2,2, préférentiellement entre 1,2 et 2,0, par exemple entre 1,20 et 1,80, avantageusement entre 1,20 et 1,50, en particulier entre 1,30 et 1,40, de façon à obtenir avantageusement un polyuréthane à terminaisons NCO.

Dans le cadre de l'invention, et sauf mention contraire, le rapport molaire NCO/OH correspond au rapport molaire du nombre de groupes isocyanates (NCO) sur le nombre de groupes hydroxyles (OH) portés respectivement par les polyisocyanates et les polyols utilisés.

Le polymère à terminaisons NCO peut avoir une masse moléculaire moyenne en nombre allant de 1 000 g/mol à 50 000 g/mol, de préférence de 1 000 g/mol à 30 000 g/mol, préférentiellement de 5 000 g/mol à 22 000 g/mol.

La masse moléculaire moyenne en nombre des polyuréthanes à terminaisons NCO peut être mesurée par des méthodes bien connues de l'homme du métier, par exemple par chromatographie d'exclusion stérique (ou SEC en anglais) en utilisant des étalons de type polyéthylène glycol.

Le polymère à terminaisons NCO peut avoir un indice de polymolécularité allant de 1,5 à 3,5, de préférence de 2,0 à 3,3.

Dans le cadre de l'invention, l'indice de polymolécularité est défini comme le rapport M_{w} (masse moléculaire moyenne en poids) / Mₙ (masse moléculaire moyenne en nombre) du polyuréthane.

Le polymère à terminaisons NCO peut avoir une teneur massique en groupes NCO allant de 0,1% à 5% en poids par rapport au poids total du polyuréthane.

Le polyuréthane comprenant au moins deux fonctions terminales NCO peut avoir une teneur massique en groupes NCO allant de 0,1 % à 6,0 %, de préférence de 0,5 % à 5,0 %, de préférence encore de 1,0 % à 4,0 %, encore plus préférentiellement de 1,5 % à 3,0 % et en particulier de 1,8 % à 2,5 %.

### Résine époxy

La résine époxy peut être aliphatique, cycloaliphatique, hétérocyclique ou aromatique et peut comprendre au moins un hétéroatome.

La résine époxy peut être monomérique ou polymérique.

De préférence, la résine époxy a une viscosité mesurée à 25°C allant de 7 à 13 000 mPa.s, préférentiellement de 400 à 5 000 mPa.s.

Selon un mode de réalisation, les résines époxy sont choisies parmi les polyglycidyl éthers de composés polyphénoliques, de préférence comprenant de 2 à 6 fonctions glycidyl éther par mole de résine.

Un composé phénolique est un composé ayant au moins deux groupes hydroxyles aromatiques.

Les composés phénoliques peuvent être choisi dans le groupe constitué du résorcinol, du catéchol, de l'hydroquinone, du bisphénol A (2,2-bis-(4-hydroxyphényl)propane), bisphénol AP (1,1-bis-(4-hydroxyphényl)-1-phényléthane), bisphénol AF (2,2-bis-(4-hydroxyphényl)-hexafluoropropane), bisphénol B ((2,2-bis-(4-hydroxyphényl)butane), bisphénol BP (bis-(4-hydroxyphényl)-diphénylméthane), bisphénol C (2,2-bis-(3-méthyl-4-hydroxyphényl)propane), bisphénol CII (bis(4-hydroxyphényl)-2,2-dichloroéthylène), bisphénol E (1,1-bis-(4-hydroxyphényl)éthane), bisphénol F (bis(4-hydroxyphényl)-2,2-dichloroéthylène), bisphénol FL (4,4'-(9H-fluorén-9-ylidène)bisphénol, bisphénol G (2,2-bis-(4-hydroxy-3-isopropylphényl)propane), bisphénol M (1,3-bis-(2-(4-hydroxyphényl)-2-propyl)benzène), bisphénol P (1,4-bis(2-4-hydroxyphényl)-2-propyl)benzène), bisphénol PH (5,5'-(1-méthyléthylidène)-bis[1,1'-(bisphényl)-2ol]propane), bisphénol S (bis(4-hydroxyphényl)sulfone), bisphénol TMC (1,1-bis(4-hydroxyphényl)-3,3,5-triméthylcyclohexane) ; bisphénol Z (1,1-bis(4-hydroxyphényl)cyclohexane), bisphénol K, tétraéthylbiphénol, et leurs mélanges.

La résine époxy peut avoir une teneur en fonction époxy allant de 0,2 à 10,8 éq pour 100 g de résine.

La fonctionnalité époxy de la résine époxy peut aller de 1 à 6.

La fonctionnalité époxy de la résine époxy est le nombre moyen de fonction époxy par mole de résine époxy.

Les résines peuvent être choisies parmi les résines suivantes :
- les résines ayant la formule (A) suivante : 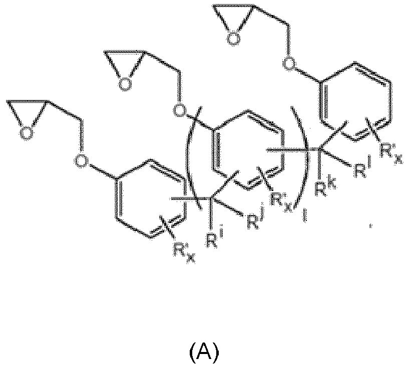 dans laquelle :
   - I représente un nombre allant de 0 à 8, de préférence de 0 à 4,
   - chaque R' représente indépendamment les uns des autres un radical alkyle comprenant de 1 à 20 atomes de carbone, de préférence un méthyle ;
   - chacun de Rⁱ, R^{j}, R^{k}, et R^{l}, indépendamment les uns des autres, représente l'un des radicaux suivants : H ; un radical alkyle linéaire ou ramifié, cyclique ou aliphatique, comprenant de 1 à 10 atomes de carbone ; un radical aryle comprenant de 6 à 12 atomes de carbone ; ou un radical -CF₃ ;
   - chaque x représente un nombre entier allant de 0 à 4, de préférence x étant 0 ou 1 ;
- le N,N-diglycidyl-4-glycidyloxyaniline (TGAP) : 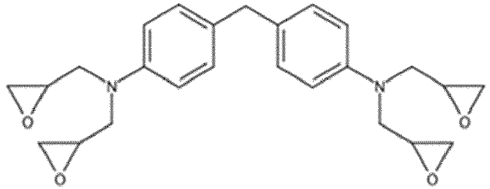
- les résines de formule (B) suivante : 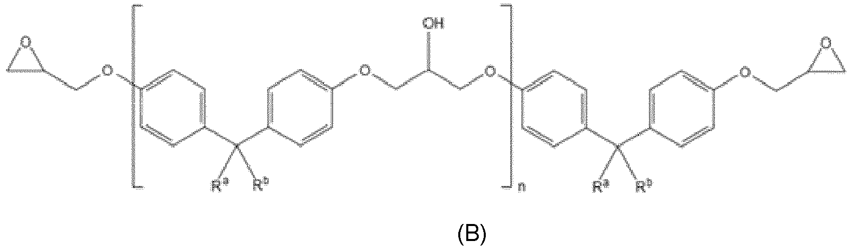 dans laquelle :
   - n est un nombre entier allant de 1 à 25, de préférence de 1 à 5 ;
   - chacun de R^{a} et R^{b} est, indépendamment l'un de l'autre, l'un des radicaux suivants : H ; un radical alkyle linéaire ou ramifié, cyclique ou aliphatique, comprenant de 1 à 10 atomes de carbone ; un radical aryle comprenant de 6 à 12 atomes de carbone ; ou un radical -CF₃.
- et leurs mélanges.

Par « mélange », on entend un mélange de plusieurs résines mentionnées ci-dessus. Il peut par exemple s'agir d'un mélange de résines de formule (A) différentes, ou alors un mélange d'une résine de formule (A) avec une résine de formule (B), ou alors tout autre mélange possible.

De nombreuses résines époxy sont typiquement disponibles commercialement. On peut par exemple citer les résines D.E.R.™ 331, D.E.R.™ 383 commercialisées par la société DOW CHEMICALS, la résine EPON 862 commercialisée par HEXION SPECIALITY CHEMICALS, les résines EPOSIR® sur base bisphénol A commercialisées par SIR INDUSTRIAL (par exemple EPOSIR® 7120, les résines EPOSIR® sur base bisphénol A/bisphénol F (par exemple EPOSIR® F556), les résines GRILONIT® de chez EMS-GRILETCH tel que par exemple GRILONIT® EV1802, GRILONIT® RV1806, GRILONIT® EPOXIDE 8, GRILONIT® V 51-31

### Composition C1

La composition C1 peut comprendre une aldimine de formule (I) ou (II) susmentionnées ou un mélange d'aldimines de formule (I) (ou (II) respectivement).

Dans la composition C1, le ratio molaire fonction aldimine N=C-G¹ / fonction NCO peut aller de 0,50 à 2,0, de préférence 0,50 à 1,5 et préférentiellement de 0,7 à 1,2.

Dans la composition C1, le ratio molaire fonction aldimine N=C-G³-C=N / fonction NCO peut aller de 0,25 à 1,0, de préférence 0,25 à 0,75 et préferentiellement de 0,35 à 0,60.

Selon un mode de réalisation, la composition C1 comprend en outre au moins un additif choisi parmi les plastifiants, les solvants, les pigments, les promoteurs d'adhérence, les absorbeurs d'humidité, les stabilisants UV (ou antioxydants), les tamis moléculaires, les paillettes, les matériaux fluorescents, les additifs rhéologiques, les charges, et leurs mélanges.

La charge peut être choisie parmi les charges organiques, les charges inorganiques et leurs mélanges.

A titre de charge(s) organique(s), on peut utiliser n'importe quelle(s) charge(s) organique(s) et notamment polymérique(s) typiquement utilisée(s) dans le domaine des compositions de mastic.

On peut utiliser par exemple du polychlorure de vinyle (PVC), des polyoléfines, du caoutchouc, de l'éthylène vinyl acétate (EVA), des fibres aramides telles que le KEVLAR®.

On peut utiliser également des microsphères creuses en polymère thermoplastique expansibles ou non expansibles. On peut notamment citer des microsphères creuses en chlorure de vinylidène/acrylonitrile.

La taille moyenne de particule de la (des) charge(s) utilisable(s) est de préférence inférieure ou égale à 10 microns, plus préférentiellement inférieure ou égale à 3 microns, afin d'éviter leur sédimentation dans la composition selon l'invention au cours de son stockage.

La taille moyenne de particule est mesurée pour une distribution granulométrique en volume et correspondant à 50% en volume de l'échantillon de particules analysé. Lorsque les particules sont sphériques, la taille moyenne de particule correspond au diamètre médian (D50 ou Dv50) qui correspond au diamètre tel que 50% des particules en volume ont une taille inférieure audit diamètre. Dans la présente demande, cette valeur est exprimée en micromètres et déterminée selon la Norme NF ISO 13320-1 (1999) par diffraction laser sur un appareil de type MALVERN.

A titre d'exemples de charge(s) minérale(s), on peut utiliser n'importe quelle(s) charge(s) minérale(s) typiquement utilisée(s) dans le domaine des compositions de revêtement de surface, de colle ou de mastic. Les charges inorganiques peuvent se présenter sous la forme de particules de géométrie diverse. Elles peuvent être par exemple sphériques, fibreuses, ou présenter une forme irrégulière.

Selon un mode de réalisation, la charge est choisie parmi le sable, les billes de verre, le verre, le quartz, la barite, l'alumine, le mica, le talc, les charges carbonatées, et leurs mélanges.

Le sable qui peut être utilisé dans la présente invention a de préférence une granulométrie allant de 0,1 à 400 µm, préférentiellement de 1 à 400 µm, de préférence encore de 10 à 350 µm, de préférence encore de 50 à 300 µm.

Les billes de verre qui peuvent être utilisées dans la présente invention ont de préférence une granulométrie allant de 0,1 à 400 µm, préférentiellement de 1 à 400 µm, de préférence encore de 10 à 350 µm, de préférence encore de 50 à 300 µm.

De préférence, la charge est une charge carbonatée choisie parmi les carbonates de métaux alcalins ou alcalino-terreux, tels que par exemple le carbonate de calcium.

Les charges peuvent être naturelles ou traitées, par exemple à l'aide d'un acide organique tel que l'acide stéarique, ou d'un mélange d'acides organiques constituées majoritairement d'acide stéarique.

La quantité totale de charge peut varier de 0,01% à 70% en poids, de préférence de 20% à 65%, préférentiellement de 20% à 50%, avantageusement de 25% à 40% en poids par rapport au poids total de la composition.

La composition C1 peut comprendre au moins un agent plastifiant à raison de 5% à 30% en poids, de préférence de 10% à 30% en poids, préférentiellement de 15% à 25% en poids par rapport au poids total de ladite composition.

A titre d'exemple d'agent plastifiant utilisable, on peut citer n'importe quel agent plastifiant habituellement utilisé dans le domaine des adhésifs, des mastics et/ou des revêtements de surface, tel que par exemples les phtalates, les benzoates, les esters de trimethylolpropane, les esters de triméthyloléthane, les esters de triméthylolméthane, les esters de glycérol, les esters de pentaerythritol, les huiles minérales napthéniques, les adipates, les cyclohexyldicarboxylates, les huiles paraffiniques, les huiles naturelles (éventuellement époxydées), les polypropylènes, les polybutylènes, les polyisoprènes hydrogénés, et leurs mélanges.

Parmi les phtalates, on peut par exemple citer le diisononyl phtalate, le di-isobutyl phtalate, le dioctyle phtalate, le dicyclohexyl phtalate, le diisooctyle phtalate, le diisododécyle phtalate, le dibenzyle phtalate, le diisodécy phtalate (par exemple commercialisé par BASF sous la dénomination PALATINOL™ DIDP), ou le butylbenzyle phtalate.

Parmi les benzoates, on peut par exemple citer : le néopentylglycol dibenzoate (par exemple disponible sous la dénomination UNIPLEX® 512 auprès de LANXESS), le dipropylèneglycol dibenzoate (par exemple disponible sous la dénomination BENZOFLEX® 9-88SG auprès de EASTMAN), un mélange de diéthylène glycol dibenzoate et de dipropylène glycol dibenzoate (par exemple disponible sous la dénomination K-FLEX® 850 S auprès de KALAMA CHEMICAL), ou encore un mélange de diéthylène glycol dibenzoate, de dipropylène glycol dibenzoate et de triéthylène glycol dibenzoate (par exemple disponible sous la dénomination BENZOFLEX® 2088 auprès de EASTMAN).

Parmi les esters de pentaérythritol, on peut par exemple citer le tétravalérate de pentaérythritol (par exemple disponible sous la dénomination PEVALEN™ auprès de la société PERSTORP).

Parmi les cyclohexanedicarboxylates, on peut par exemple citer le diisononyl 1,2-cyclohexanedicarboxylate (par exemple disponible sous la dénomination HEXAMOLL DINCH® auprès de BASF).

A titre d'exemple d'agent(s) de rhéologie utilisable(s), on peut citer n'importe quel agent de rhéologie habituellement utilisé dans le domaine des compositions adhésives, de mastic et/ou de revêtement de surface.

De préférence, on utilise un ou plusieurs agents de rhéologie choisis parmi les agents thixotropiques, et plus préférentiellement parmi:
- les plastisols de PVC, correspondant à une suspension de PVC dans un agent plastifiant miscible avec le PVC, obtenue in situ par chauffage à des températures allant de 60°C à 80°C. Ces plastisols peuvent être ceux décrits notamment dans l'ouvrage « Polyurethane Sealants », Robert M. Evans, ISBN 087762-998-6,
- la silice pyrogénée,
- des dérivés d'urée issus de la réaction d'un monomère diisocyanate aromatique tel que le 4,4'-MDI avec une amine aliphatique telle que la butylamine comme par exemple le « Gelpaste Urea » (EC : 416-600-4). La préparation de tels dérivés d'urée est décrite notamment dans la demande FR 1 591 172.

La teneur totale en agent(s) de rhéologie(s) pouvant être utilisée peut varier de 1% à 40% en poids, de préférence de 5% à 30% en poids, plus préférentiellement de 10% à 25% en poids par rapport au poids total de la composition C1.

Le solvant est de préférence un solvant volatil à température à 23°C. Le solvant volatil peut par exemple être choisi parmi les alcools volatils à 23°C, tel que l'éthanol ou l'isopropanol. Le solvant volatil permet par exemple de diminuer la viscosité de la composition et de rendre la composition plus facile à appliquer. Le caractère volatil du solvant permet notamment au joint, obtenu après durcissement de la composition, de ne plus contenir de solvant. Ainsi, le solvant n'a par exemple pas d'influence négative sur la dureté du joint.

Lorsqu'un solvant, en particulier un solvant volatil, est présent dans la composition C1, sa teneur est de préférence inférieure ou égale à 5% en poids, de préférence encore inférieure ou égale à 3% en poids, par rapport au poids total de la composition.

De préférence, la teneur en solvant(s) dans la composition C1 est comprise entre 0% et 5% en poids.

Les pigments peuvent être des pigments organiques ou inorganiques.

Par exemple, le pigment est TiO₂, en particulier le KRONOS® 2059 commercialisé par la société KRONOS.

La composition C1 peut comprendre une quantité de 0,1% à 3%, de préférence de 0,1% à 3%, encore plus préférentiellement de 0,1% à 1% en poids, d'au moins un stabilisant UV ou antioxydant. Ces composés sont typiquement introduits pour protéger la composition d'une dégradation résultant d'une réaction avec de l'oxygène qui est susceptible de se former par action de la chaleur ou de la lumière. Ces composés peuvent inclure des antioxydants primaires qui piègent les radicaux libres. Les antioxydants primaires peuvent être utilisés seuls ou en combinaison avec d'autres antioxydants secondaires ou des stabilisants UV.

On peut par exemple citer l'IRGANOX® 1010, l'IRGANOX® B561, l'IRGANOX® 245, l'IRGAFOS® 168, TINUVIN® 328 ou TINUVIN™ 770 commercialisés par BASF.

De préférence, la composition C1 est une composition d'adhésif, de mastic ou de revêtement.

Dans le cadre de l'invention, par « comprise entre x et y », ou « allant de x à y », on entend un intervalle dans lequel les bornes x et y sont incluses. Par exemple, la gamme «comprise entre 0% et 25% » inclus notamment les valeurs 0% et 25%.

L'invention est à présent décrite dans les exemples de réalisation suivants qui sont donnés à titre purement illustratif, et ne sauraient être interprétés pour en limiter la portée.

### EXEMPLES

Les ingrédients suivants ont été utilisés :
- Méthanol disponible chez SIGMA-ALDRICH
- 2-éthyl-1-hexanol disponible chez SIGMA-ALDRICH
- Dipropylène glycol n-butyl éther disponible chez DOW
- Polypropylène glycol (PPG) difonctionnel de Mn = 400 g/mol disponible chez SIGMA ALDRICH
- Polypropylène glycol (PPG) trifonctionnel (TMP propoxylé) de Mn = 308 g/mol disponible chez SIGMA ALDRICH
- Acide laurique disponible chez SIGMA-ALDRICH
- (±)-Alanine (CAS : 302-72-7) disponible chez ACROTEIN ChemBio 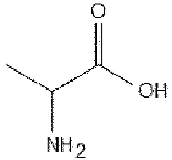
- (±)-2-Aminoisobutyrique acide (CAS : 62-57-7) disponible chez ACROTEIN ChemBio 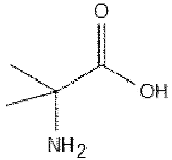
- (±)-Sérine (CAS : 302-84-1) disponible chez ACROTEIN ChemBio 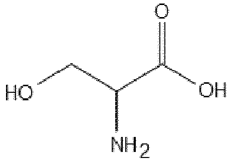
- (±)-Statine (CAS : 27512-69-2) disponible chez AURORA Building Blocks 5 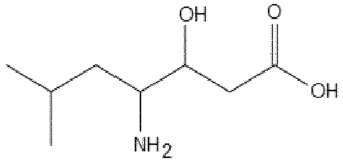
- (±)-Acide 4-amino-3-hydroxy-3-méthyl butanoique (CAS : 63278-07-9) disponible chez ENAMINE Building Blocks 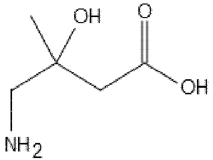
- (±)-Acide Glutamique (CAS : 617-65-2) disponible chez ACROTEIN ChemBio 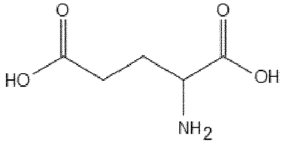
- (±)-Lysine (CAS : 70-54-2) disponible chez ACROTEIN ChemBio 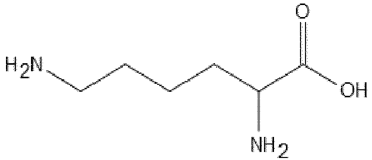
- N-Boc-(±)-Alaninate de méthyle disponible chez ACROTEIN ChemBio

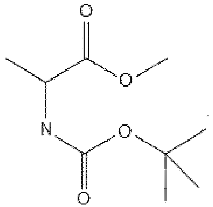

### Exemple 1 - Synthèse des N-Boc-Aminoacides

On dissout 1,00 mole d'aminoacide dans un minimum de 800 ml d'eau maintenue à 0°C. On ajoute lentement à la solution aqueuse précédemment préparée et sous agitation, une solution de ditertiobutylbutyl dicarbonate (BOC) dans 400 ml de THF dans une quantité telle que le rapport molaire BOC / -NH2 est de 1,05. On ajuste le pH de la solution entre 10-12 avec successifs des ajouts de carbonate de potassium (K2CO3). Après 30 minutes sous agitation à 0°C, on laisse remonter le mélange réactionnel à température ambiante (env. 23°C). Après 8 heures à température ambiante et sous agitation, on élimine le THF sous pression réduite à l'évaporateur rotatif, on lave la phase aqueuse avec de l'éther diéthylique, on l'acidifie avec de l'acide citrique puis on extrait le produit de réaction 3 fois 100 ml d'acétate d'éthyle. La phase organique est séchée sur sulfate de sodium anhydre (Na₂SO₄) et concentrée sous pression réduite à l'évaporateur rotatif. Le N-Boc Aminoacide est isolé avec un rendement supérieur à 98 %.

### Exemple 2 - Synthèse des X-TBDMS Aminoacides

On dissout 1,00 mole 285,5 mmol d'aminoacide dans 1000 ml d'acétonitrile, on ajoute à température ambiante (env. 23°C) le dimethyl-tert-butylchlorosilane (TBDMSCI) en présence de DBU dans des quantités telles que le rapport molaire TBSCI / OH est de 1,05 et un rapport molaire DBU / TBDMSCI stœchiométrique, puis on maintient sous agitation pendant 12 heures à température ambiante. Le produit insoluble est filtré, lavé à l'acétonitrile et séché sous pression réduite. Le X-TBDMS Aminoacide est isolé avec un rendement supérieur à 92 %.

### Exemple 3 - Synthèse des N-Boc / X-TBDMS Aminoacides

On applique successivement le mode opératoire 1 puis le mode opératoire 2. Le N-Boc / X-TBDMS Aminoacide est isolé avec des rendements allant de 92 à plus de 98 %.

### Exemple 4 - Synthèse des Aminoesters (A à L)

En fonction de la nature de l'alcool (monol, diol ou triol) utilisé pour l'estérification de l'aminoacide et selon qu'il est plus ou moins facile de les éliminer sous pression réduite, on utilise préférentiellement le mode opératoire 4a pour les alcools de point d'ébullition ≤ 160°C à Patm ou le mode opératoire 4b pour les alcools de point d'ébullition > 160°C à Patm.

### Mode opératoire 4a

On disperse 2 moles d'aminoacide dans 500 ml d'alcool anhydre (monol) puis on additionne sous agitation, à -15°C, une quantité de chlorure de thionyle (SOCl₂) telle que le rapport molaire -COOH / SOCI2 est compris entre 0,98 et 1,00. Selon l'aminoacide, le mélange réactionnel est ensuite porté à 40°C ou laissé à température ambiante (env. 23°C) pendant environ 12 heures. Après avoir éliminé l'alcool en excès sous pression réduite à l'évaporateur rotatif, on additionne 2000 ml d'éther éthylique anhydre. On agite 1 heure entre 0 et 5°C. Le solide est isolé par filtration et lavé à l'éther anhydre refroidit à 0°C puis séché sur P₂O₅. Le chlorhydrate d'aminoester est isolé avec un rendement supérieur à 98%.

Les 2 moles de chlorhydrate d'aminoester précédemment obtenues sont mises en suspension dans 2000 ml d'éther éthylique anhydride en présence de 2,2 moles de triéthylamine (Et₃N). On agite 1 heure à température ambiante (env. 23°C) puis la solution est filtrée et la phase organique est concentrée sous pression réduite à l'évaporateur rotatif. L'aminoester est isolé avec un rendement supérieur à 98%.

### Mode opératoire 4b

On dissout dans 500 ml de dichlorométhane (CH2Cl2), 52 mmoles de N-Boc aminoacide ou de N-Boc / X-TBDMS Aminoacide, l'alcool anhydre (monol, diol ou triol) et la 4-diméthylaminopyridine (DMAP) dans des quantités telles que les rapports molaires -COOH / -OH et -COOH / DMAP soient respectivement 1,02 et de 1,7. On ajoute le chlorure de 1-éthyl-3-[3-(diméthylaminopropyl]carbodiimide (EDCI) dans une quantité telle que le rapport molaire -COOH / EDCI soit de 0,92, le mélange est maintenu sous agitation pendant 1 heure puis à température ambiante (23°C) pendant environ 12 heures. Après avoir éliminé le dichlorométhane (CH2Cl2) sous pression réduite à l'évaporateur rotatif, le résidu est dilué dans 1500 ml d'acétate d'éthyle, on lave avec 750 ml d'eau et on isole la phase organique. La phase organique est ensuite extraite avec 3 fois 500 ml d'une solution saturée de bicarbonate de sodium (NaHCO3) et lavée avec 3 fois 500 ml d'eau. Après séchage de la phase organique sur sulfate de sodium anhydre (Na2SO4), on élimine l'acétate d'éthyle sous pression réduite à l'évaporateur rotatif et le résidu est mis à sécher sous pression réduite. Le N-Boc Aminoester ou le N-Boc / X-TBDMS Aminoester est isolé avec un rendement de 97 %.

Les 50 mmoles de N-Boc Aminoester ou de N-Boc / X-TBDMS Aminoester précédemment obtenu sont dissout dans 750 ml de CH2Cl2. On ajoute 250 ml de TFA puis on maintient sous agitation à température ambiante pendant environ 12 heures et on neutralise le trifluoroacétate d'aminoester avec un ajout de bicarbonate de sodium. On élimine par filtration le trifluoroacétate de sodium, on récupère la phase organique et le dichlorométhane est éliminé sous pression réduite à l'évaporateur rotatif. L'aminoester est isolé avec un rendement de 96 %.

Les aminoesters A à L suivants ont été synthétisés :

| **Aminoester** | | **Mode opératoire** | **réactifs** |
|---|---|---|---|
| (±)-Alaninate de méthyle | A | 4a | - (±)-Alanine |
| 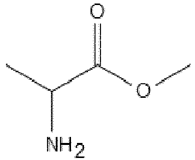 | | | - méthanol |
| (±)-2-Aminoisobutyrate de méthyle | B | 4a | - (±)-Acide 2-Aminoisobutyrique |
| 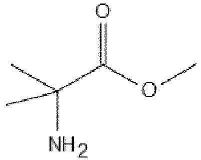 | | | |
| | | | - méthanol |
| (±)-Serinate de méthyle | C | 4a | - (±)-Serine |
| 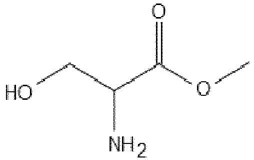 | | | - méthanol |
| Statinate de méthyle | D | 4a | - statine |
| | | | - méthanol |
| 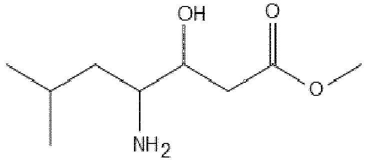 | | | |
| (±)-Acide 4-amino-3-hydroxy-3-méthyl butanoate de méthyle | E | 4a | - (±)-Acide 4-amino-3-hydroxy-3-méthyl butanoique |
| 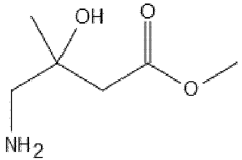 | | | - méthanol |
| (±)-Glutamate de méthyle | F | 4a | - (±)-Acide Glutamique |
| 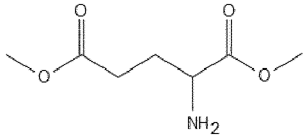 | | | - méthanol |
| (±)-Lysinate de méthyle | G | 4a | - (±)-Lysine |
| 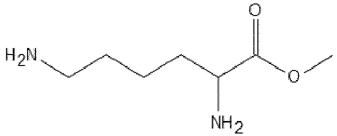 | | | - méthanol |
| (±)-Alaninate de 2-éthylhexyle | H | 4a | - (±)-Alanine |
| 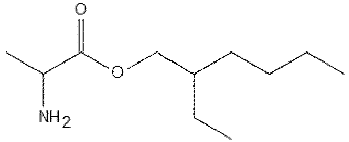 | | | - 2-éthyl-1-hexanol |
| (±)-Alaninate 1-méthyl-2-[1-méthyl-2-(butyloxy)éthoxy]éthyle | I | 4b | - N-Boc (±)-Alanine - dipropylène glycol n-butyl éther |
| 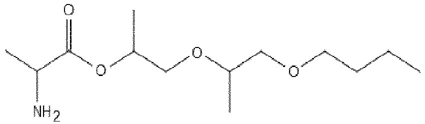 | | | |
| Di-(±)-Alaninate de PPG difonctionnel | J | 4b | - N-Boc (±)-Alanine |
| | | | - polypropylène glycol de Mn = 400 g/mol |
| 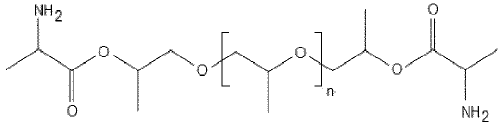 | | | |
| Tri-(±)-Alaninate de PPG trifonctionnel | K | 4b | - N-Boc (±)-Alanine |
| 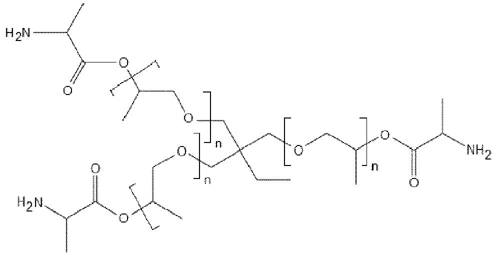 | | | - triméthylolpropane propoxylé de Mn = 308 g/mol |
| Di-(±)-Alaninate / Mono laurate de PPG trifonctionnel | L | 4b | - N-Boc (±)-Alanine |
| 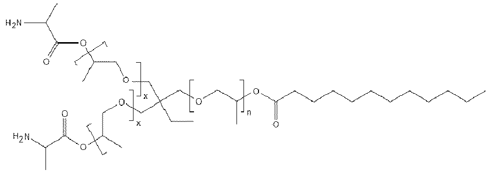 | | | - triméthylolpropane propoxylé de Mn = 308 g/mol |

### Exemple 5 - Synthèse des aminoamides (M, N et O)

On dissout dans 500 ml de chlorure de méthyle sous atmosphère inerte, 60 mmoles de N-Boc Aminoacide ou de N-Boc / X-TBDMS Aminoacide, l'amine primaire ou secondaire et le chlorure de 1-éthyl-3-(3-diméthylaminopropyl)-carbodiimide (EDCI) dans des quantités telles que les rapports molaires COOH / NH et COOH / EDCI sont respectivement de 1,2 et de 0,80. On maintient sous agitation à température ambiante pendant au moins 5 heures. Après avoir éliminé le dichlorométhane (CH2Cl2) sous pression réduite à l'évaporateur rotatif, le résidu est dilué dans 500 ml d'acétate d'éthyle, on lave avec 250 ml d'eau et on isole la phase organique. La phase organique est ensuite extraite avec 3 fois 150 ml d'une solution saturée de bicarbonate de sodium (NaHCO3) et lavée avec 3 fois 150 ml d'eau. Après séchage de la phase organique sur sulfate de magnésium anhydre (Mg2SO4), on élimine l'acétate d'éthyle sous pression réduite à l'évaporateur rotatif et le résidu est mis à sécher sous pression réduite. Le N-Boc Aminoester ou le N-Boc / X-TBDMS Aminoester est isolé avec un rendement de 98 %.

Les 50 mmoles de N-Boc Aminoamide ou de N-Boc / X-TBDMS Aminoamides précédemment obtenu sont dissout dans 500 ml de CH₂Cl₂. On ajoute 250 ml de TFA puis on maintient sous agitation à température ambiante pendant environ 12 heures et on neutralise le trifluoroacétate d'aminoester avec un ajout de bicarbonate de sodium. On élimine par filtration le trifluoroacétate de sodium, on récupère la phase organique et le dichlorométhane est éliminé sous pression réduite à l'évaporateur rotatif. L'aminoamide est isolé avec un rendement de 96 % minimum.

Les aminoamides suivantes ont été synthétisées :

| **Aminoamide** | **N°** | **Voie** | **Réactifs** |
|---|---|---|---|
| (±)-N-(2-éthylhexyl)alaninamide | M | 5a | - N-Boc(±)-Alanine |
| 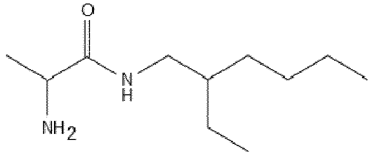 | | | - 2-éthyl-1-hexylamine |
| (±)-N-(2-éthylhexyl)alaninamide | N | 5a | - N-Boc(±)-Alanine |
| 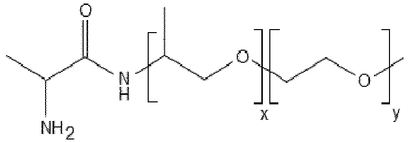 | | | - JEFFAMINE M 600 ayant une masse moléculaire moyenne en nombre (Mn) de 600 g/mol (x/y = 9/1) |
| | O | 5a | - N-Boc(±)-Alanine |
| (±)-N-(2-éthylhexyl)alaninamide | | | - JEFFAMINE D 400 ayant une masse moléculaire moyenne en nombre (Mn) de 430 g/mol (x = 6,1) |
| 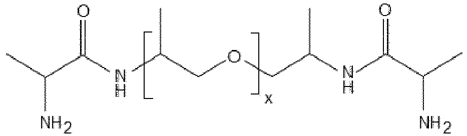 | | | |

### Exemple 6 - Synthèse des aldéhydes P à Z5

Les aldehydes P à Z suivants ont été synthétisés selon les modes opératoires mentionnés ou achetés :

| Aldéhyde | N° | synthèse |
|---|---|---|
| 2,2-diméthyl-3-(morpholin-4-yl)propanal (CAS Number : 23588-51-4, Téb : 199°C à Patm) | P | Disponible chez BOC SCIENCES |
| 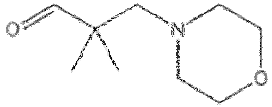 | | |
| 2,2-diméthyl-3-oxopropyl acetate (CAS Number : 16184-79-5, Téb : 124 °C à Patm) | Q | Disponible chez CHEMIELIVA PHARMACEUTICA L |
| 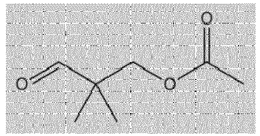 | | |
| 2,2-dimethyl-3-oxopropyl laurate (CAS Number : 102985-93-3, Téb > 160°C à Patm) | R | Synthétisé d'après les modes opératoires décrits dans Macromolecular Chemistry and Physics (2004), 205(7), 973-978 |
| 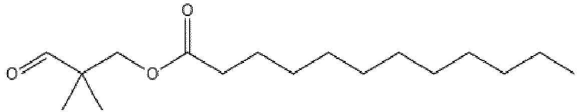 | | |
| (5-formyl-2-furanyl)méthyl acétate (CAS Number : 10551-58-3, Téb > 160°C à Patm) | S | Synthétisé, à partir du 5-(Hydroxymethyl)furf ural (CAS Number : 67-47-0) disponible chez SIGMA-ALDRICH, d'après le mode opératoire décrit dans WO 2013/141523 |
| 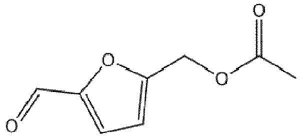 | | |
| (5-formyl-2-furanyl)méthyl laurate (CAS Number : 1428770-85-7, Téb > 160°C à Patm) | T | Synthétisé, à partir du 5-(Hydroxymethyl)furf ural (CAS Number : 67-47-0) disponible chez PURAC BIOQUIMICA, d'après le mode opératoire décrit dans US 10,259,797 |
| 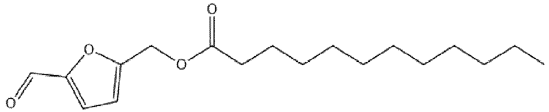 | | |
| 2,2-diméthyl-3-phenylpropanal ou Normajantal (CAS Number : 1009-62-7, Téb : 228°C à Patm) | U | Disponible chez SYMRISE |
| 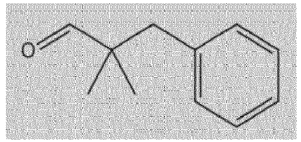 | | |
| dimethylcyclohex-3-ene-1-carbaldehyde (CAS Number : 27939-60-2, Téb : 178°C à Patm) | V | Disponible chez SIGMA-ALDRICH |
| 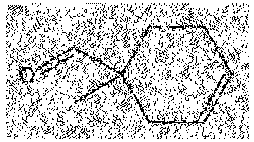 | | |
| Propanoic acid, 2,2-dimethyl-3-oxo-propanoate de dodecyle (CAS Number : 14002-70-1, Téb : 140°C à Patm) | W | Synthétisé d'après le mode opératoire décrit dans la demande de brevet WO 00/02890 de NESTE CHEMICALS OY |
| 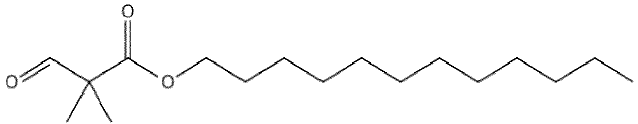 | | |
| 5-formyl-2-furancarboxylate de dodecyle (Téb > 160°C à Patm) | X | Synthétisé d'après le mode opératoire décrit dans Synlett 1993(2), 117-118 |
| 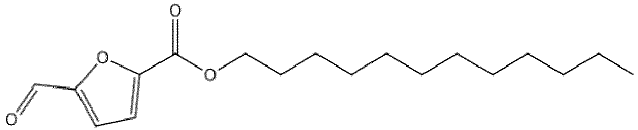 | | |
| (5-Formylfuran-2-yl)methylN-dodecylcarbamate | Y | Synthétisé, à partir du 5-(Hydroxymethyl)furf ural disponible chez PURAC BIOQUIMICA et de dodecyl isocyanate disponible chez CREACHEM BVBA, d'après le mode opératoire décrit dans WO 2004/037804 |
| 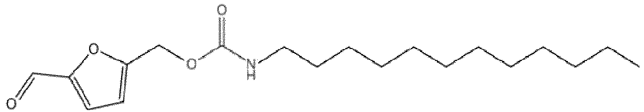 | | |
| 2,2-dimethyl-3-[[(dodecylamino)carbonyl]oxy] propanal | Z1 | Synthétisé à partir de 3-hydroxy-2,2-diméthyl-propanal disponible chez ALFA CHEMISTRY et de dodecyl isocyanate (disponible chez CREACHEM BVBA, d'après le mode opératoire décrit EP 0,375,318 |
| 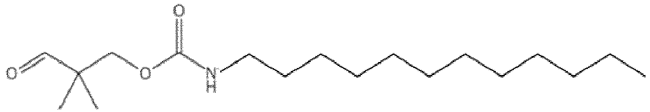 | | |
| 3-[2,2-dimethyl-3-[[(dodecylamino)carbonyl]oxy]]-2-[[2,2-dimethyl-3-[[(dodecylamino)carbonyl]oxy]]-methyl]-2-methylpropionaldehyde | Z2 | Synthétisé, à partir de dimethylol propanal (CAS Number : 18516-18-2) disponible chez CROMOGENIA et de dodecyl isocyanate (CAS Number : 4202-38-4) disponible chez CREACHEM BVBA, d'après le mode opératoire décrit dans EP0,375,318 |
| 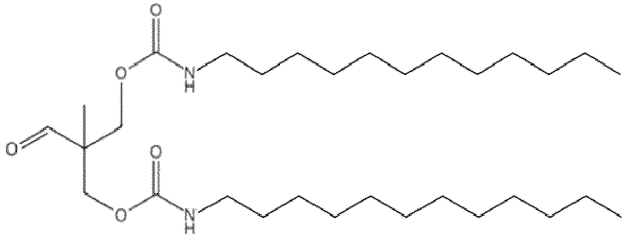 | | |
| 1,12-bis[2,2-dimethyl-3-oxopropyl] dodecanedioate | Z3 | Modes opératoires décrits dans WO 2004/013088 |
| 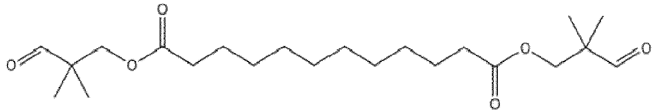 | | |
| N-[2,2-dimethyl-3-[[(6-aminohexyl)carbonyl]oxy] propanal]-N-[[[2,2-dimethyl-3-[[(6-aminohexyl)carbonyl]oxy] propanal]-amino]carbonyl] dodecyl carbamate | Z4 | Mode opératoire décrit dans EP 3,015,486 |
| 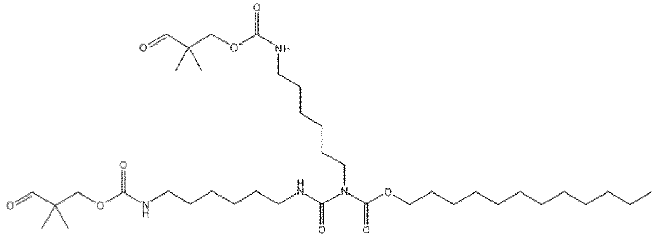 | | |
| N-[[[(5-formylfuran-2-yl)methyl]-1-[[(6-aminohexyl)carbonyl]oxy]]-N-[[(5-formylfuran-2-yl)methyl]-1-[[[[(6-aminohexyl)carbonyl]oxy]-amino]carbonyl] dodecyl carbamate | Z5 | Synthétisé à partir du 5-(Hydroxymethyl)furf ural disponible chez PURAC BIOQUIMICA et de N-(6-isocyanatohexyl)-N-[[(6-isocyanatohexyl)amino]carbonyl] dodecyl carbamate (CAS Number : 1027000-30-1) selon le mode opératoire décrit dans EP 3,015,486 |
| 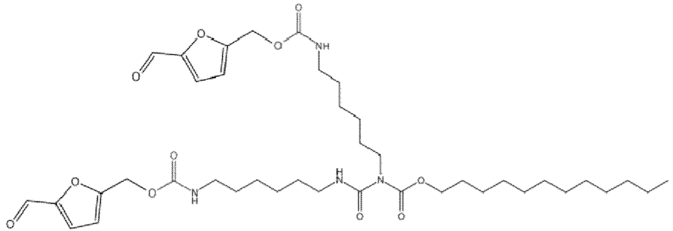 | | |

### Exemple 7 : synthèse des aldmines de formules (I) ou (II)

### Aldimines de formule (I)

En fonction de la nature des aminoesters ou des aminoamides de formule (III) ou (III'), des monoaldéhydes de formule (IV) utilisés pour la synthèse des aldimines de formule (I) et de leurs solubilités respectives dans le toluène, dans le méthanol absolu ou dans l'éther éthylique anhydride, on utilise préférentiellement le mode opératoire 6a pour les aldéhydes de point d'ébullition ≤ 160°C à Patm ou les modes opératoires 6b ou 6c pour les aldéhydes de point d'ébullition > 160°C à Patm.

### Mode opératoire 6a

Dans un réacteur équipé d'une agitation et d'un appareil de Dean-Stark, on dissout dans 500 ml de toluène sous atmosphère inerte (azote), 1 mole de l'un des (di)aminoesters (A à L) ou l'une des (di)aminoamides (M et N) de formule (III) ou (III'), 1 mmol d'acide formique puis on ajoute un monoaldéhyde ayant un point d'ébullition ≤ 160°C à Patm dans une quantité telle que le rapport molaire -CHO / -NH₂ est égal 1,25. On chauffe et on maintient au reflux pendant environ 6 heures jusqu'à ce qu'il n'y ait plus élimination d'eau par distillation azéotropique. On élimine ensuite le toluène et l'aldéhyde en excès par distillation sous pression réduite (1 mm Hg). L'aldimine de formule (I) est obtenue avec un rendement quantitatif.

### Mode opératoire 6b

Dans un réacteur équipé d'une agitation, on dissout dans 500 ml de méthanol absolu sous atmosphère inerte (azote), 1 mole de l'un des (di)aminoesters (A à L) ou l'une des (di)aminoamides (M et N) de formule (III) ou (III') puis on ajoute un monoaldéhyde ayant un point d'ébullition > 160°C à Patm dans une quantité telle que le rapport molaire -CHO / -NH₂ est voisin de 1 et 150 g de tamis 3 A. Le milieu est abandonné à température ambiante jusqu'à disparition du signal aldéhydique en RMN ¹H/¹³C (15 minutes à 3 heures selon la qualité du tamis moléculaire). On filtre la solution et on élimine le méthanol par distillation sous pression réduite (1 mm Hg). L'aldimine de formule (I) est obtenue avec un rendement quantitatif.

### Mode opératoire 6c

Dans un réacteur équipé d'une agitation, on dissout dans 500 ml d'éther éthylique anhydre sous atmosphère inerte (azote), 1 mole de l'un des (di)aminoesters (A à L) ou l'une des (di)aminoamides (M et N) de formule (III) ou (III') puis on ajoute un monoaldéhyde ayant un point d'ébullition > 160°C à Patm dans une quantité telle que le rapport molaire -CHO / -NH₂ est voisin de 1 et 150 g de tamis 3 A. Le milieu est abandonné à température ambiante jusqu'à disparition du signal aldéhydique en RMN ¹H/¹³C (12 à 24 heures selon la qualité du tamis moléculaire). On filtre la solution et on élimine l'éther éthylique par distillation sous pression réduite (10 mm Hg). L'aldimine de formule (I) est obtenue avec un rendement quantitatif.

### Aldimines de formule (II)

En fonction de la nature des aminoesters ou des aminoamides de formule (III) ou (III'), des dialdéhydes de formule (VIII) utilisés pour la synthèse des aldimines de formule (II) et de leurs solubilités respectives dans le toluène, dans le méthanol absolu ou dans l'éther éthylique anhydride et selon qu'il est plus ou moins facile d'éliminer les aldéhydes sous pression réduite, on utilise préférentiellement le mode opératoire 7a pour les aldéhydes de point d'ébullition ≤ 160°C à Patm ou les modes opératoires 7b ou 7c pour les aldéhydes de point d'ébullition > 160°C à Patm.

### Mode opératoire 7a

Dans un réacteur équipé d'une agitation et d'un appareil de Dean-Stark, on dissout dans 500 ml de toluène sous atmophère inerte (azote), 1 mole de l'un des aminoesters (A à F, H et I) ou l'aminoamide (O) de formule (III) ou (III'), 1 mmol d'acide formique puis un dialdéhyde de formule (VIII) ayant un point d'ébullition ≤ 160°C à Patm dans une quantité telle que le rapport molaire -CHO / -NH est égal à 1,25. On chauffe et on maintient au reflux pendant environ 6 heures jusqu'à ce qu'il n'y ait plus élimination d'eau par distillation azéotropique. On élimine ensuite le toluène et le dialdéhyde en excès par distillation sous pression réduite (1 mm Hg). L'aldimine de formule (II) est obtenue avec un rendement quantitatif.

### Mode opératoire 7b

Dans un réacteur équipé d'une agitation, on dissout dans 500 ml de méthanol absolu sous atmosphère inerte (azote), 1 mole de l'un des aminoesters (A à F, H et I) ou l'aminoamide (O) de formule (III) ou (III') puis on ajoute un dialdéhyde de formule (VIII) ayant un point d'ébullition > 160°C à Patm dans une quantité telle que le rapport molaire -CHO / -NH₂ est voisin de 1 et 150 g de tamis 3 A. Le milieu est abandonné à température ambiante jusqu'à disparition du signal aldéhydique en RMN ¹H/¹³C (15 minutes à 3 heures selon la qualité du tamis moléculaire). On filtre la solution et on élimine le méthanol par distillation sous pression réduite (1 mm Hg). L'aldimine de formule (II) est obtenue avec un rendement quantitatif.

### Mode opératoire 7c

Dans un réacteur équipé d'une agitation, on dissout dans 500 ml d'éther éthylique anhydre sous atmosphère inerte (azote), 1 mole de l'un des aminoesters (A à F, H et I) ou l'aminoamide (O) de formule (III) ou (III') puis on ajoute un dialdéhyde de formule (VIII) ayant un point d'ébullition > 160°C à Patm dans une quantité telle que le rapport molaire -CHO / - NH2 est voisin de 1 et 150 g de tamis 3 A. Le milieu est abandonné à température ambiante jusqu'à disparition du signal aldéhydique en RMN ¹H/¹³C (12 à 48 heures selon la qualité du tamis moléculaire). On filtre la solution et on élimine l'éther éthylique par distillation sous pression réduite (10 mm Hg). L'aldimine de formule (II) est obtenue avec un rendement quantitatif.

**Tableau de molécules**

| Les aldimines suivantes ont ainsi été synthétisées : | | | |
|---|---|---|---|
| Aminoester/Aminoamide | Aldéhyde | Aldmines de formule (I) ou (II) | N° |
| A | P | 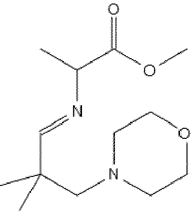 | Ald1 |
| A | Q | 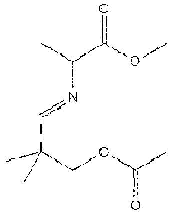 | Ald2 |
| A | R | 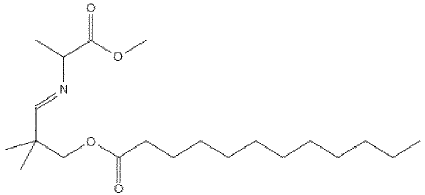 | Ald3 |
| A | T | 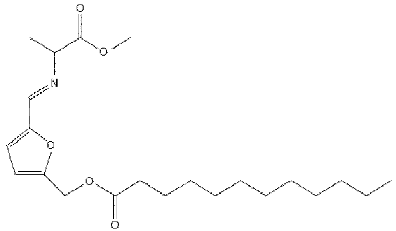 | Ald4 |
| A | U | 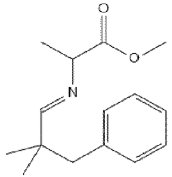 | Ald5 |
| A | V | 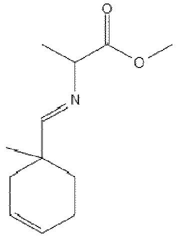 | Ald6 |
| A | W | 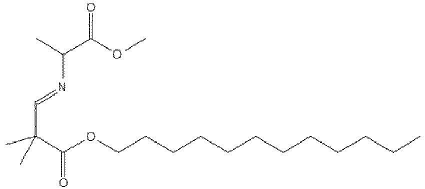 | Ald7 |
| A | X | 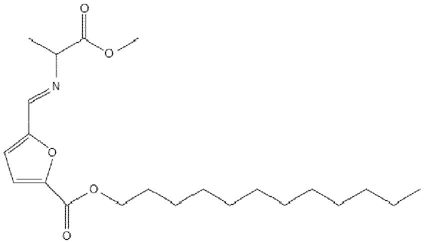 | Ald8 |
| A | Y | 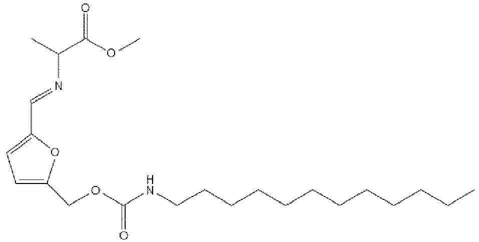 | Ald9 |
| A | Z1 | 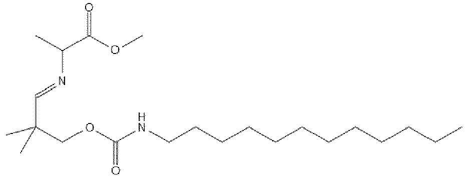 | Ald10 |
| A | Z3 | 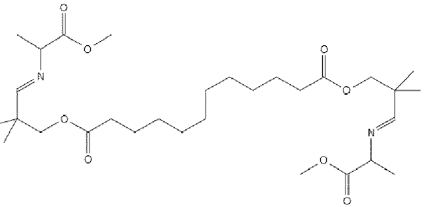 | Ald11 |
| A | Z4 | 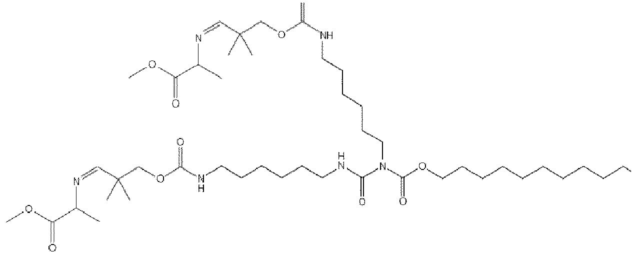 | Ald12 |
| A | Z5 | 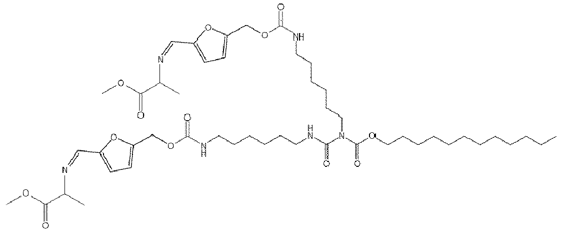 | Ald13 |
| B | R | 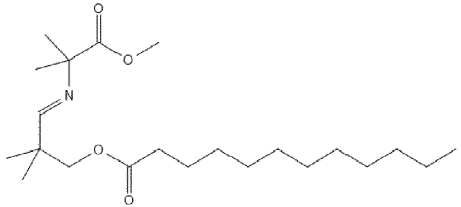 | Ald14 |
| C | R | 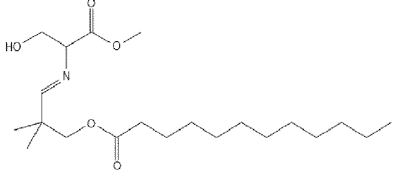 | Ald15 |
| D | R | 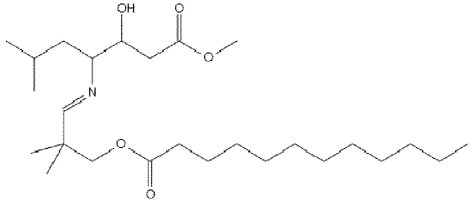 | Ald16 |
| E | R | 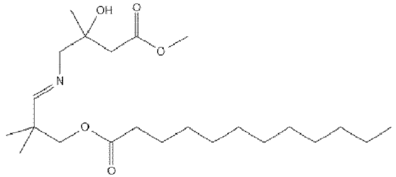 | Ald17 |
| F | R | 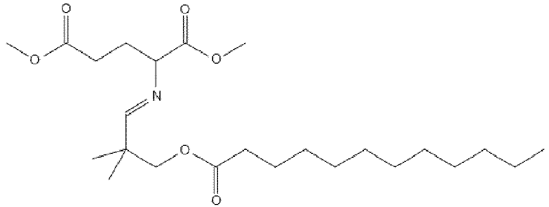 | Ald18 |
| H | R | 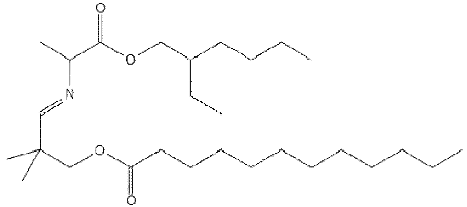 | Ald19 |
| I | R | 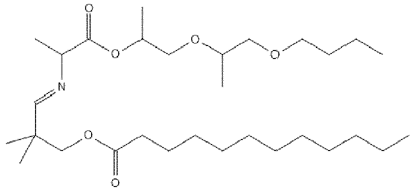 | Ald20 |
| K | S | 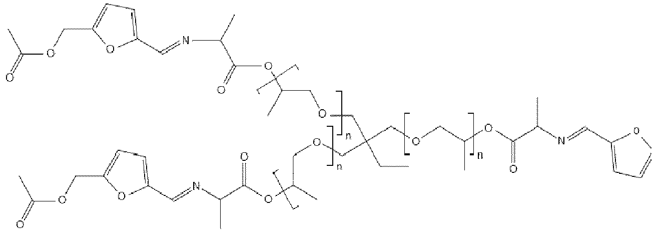 | Ald21 |
| L | P | 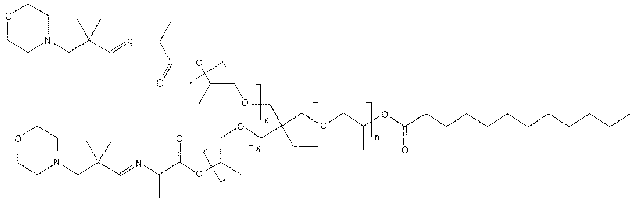 | Ald22 |
| J | U | 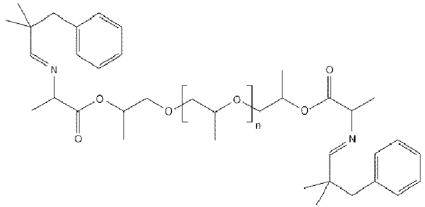 | Ald23 |
| J | Y | 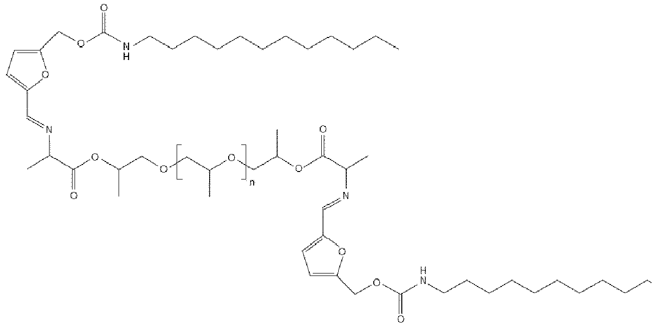 | Ald24 |

### Exemple 9 : test applicatif

### Préparation du polyuréthane terminé NCO (P1)

L'exemple non limitatif de polyuréthane terminé NCO (P1) utilisé dans l'exemple de référence hors invention (sans aldimine) et dans les exemples **C1** à **C2** selon l'invention (avec des aldimines selon l'invention) a été préparé par mélange des ingrédients indiqués dans le tableau suivant à une température inférieure ou égale à 95°C dans des conditions anhydres. Les quantités indiquées dans le tableau ci-dessous sont exprimées en pourcentage en poids par rapport au poids total de la composition de polyuréthane de chacun des exemples.

| **Ingédients** | **P1** |
|---|---|
| PPG triol ayant une masse molaire moyenne en nombre de 3700 g/mol (IOH = 44,5 mg KOH/g) : Desmophen 4042 BT | 38,3 |
| PPG diol ayant une masse molaire moyenne en nombre de 2000 g/mol : VORANOL 2000 L | 36,9 |
| 2,4'-TDI (%NCO ≥ 48,0% en poids) : Desmodur T 100 | 9,6 |
| Catalyseur (DOTL) | 0,012 |
| Xylène | 15,2 |
| Rapport molaire NCO/OH | 1,6 |
| % en poids de NCO final | 1,8 |

Dans le tableau ci-dessus, le % en poids de NCO final correspond à la quantité de fonctions NCO dans la solution de polyuréthane **(P1)** à la fin de la réaction de préparation du polyuréthane, exprimé par rapport au poids total de la solution de polyuréthane.

### Préparation des compositions de mastic

Des compositions de mastic ont ensuite été formulées à partir du polyuréthane terminé NCO préparé conformément au procédé ci-dessus.

La composition de mastic de référence hors invention (sans aldimine) et les compositions de mastics **C1** à **C2** selon l'invention (avec des aldimines selon l'invention) ont été reportées dans le tableau suivant :

| | **Référence** | **C1** | **C2** |
|---|---|---|---|
| Prépolymère (P1) | 19,40 | 17,31 | 17,11 |
| Mesamoll : CAS 91082-17-6 | 16,90 | 16,91 | 16,91 |
| Gel Paste CAS 77703-56-1 disponible chez SIKA (dibutyl-4-4'-methylenedi(phenyl)urée) | 12,33 | 12,34 | 12,34 |
| DIDP : diisodécyl phtalate | 2,10 | 2,10 | 2,10 |
| PTSI p-toluenesulfonyl isocyanate | 0,40 | 0,40 | 0,40 |
| IPDI | 0,40 | 0,40 | 0,40 |
| XDI | 0,20 | 0,20 | 0,20 |
| Silquest A-187 (MOMENTIVE) | 0,17 | 0,17 | 0,17 |
| Tinuvin B 75 (BASF) | 0,14 | 0,14 | 0,14 |
| Xylène | 1,90 | 1,90 | 1,90 |
| | | | |
| PVC (solvin 373 MC) | 15,60 | 15,61 | 15,61 |
| OMYA BSH | 25,00 | 25,01 | 25,01 |
| TiO₂ | 4,90 | 4,90 | 4,90 |
| Aerosil R 202 | 0,51 | 0,51 | 0,51 |
| | | | |
| Ald 23 avec Mn = 590 g/mol | | 2,00 | |
| Ald 21 avec Mn = 890 g/mol | | | 2,10 |
| Acide Salicylique | | 0,10 | 0,20 |
| | | | |
| Catalyseur d'étain | 0,05 | - | - |

Les compositions de mastic ci-dessus formulées à partir du polyuréthane terminé NCO **(P1)** et des aldimines selon l'invention comparativement à la composition de mastic de référence ont été caractérisées et les résultats reportés dans le tableau ci-dessous :

| | **Référence** | **C1** | **C2** |
|---|---|---|---|
| Temps de formation de peau (min) | 7 h | 64 min | 25 min |
| Extrusion (g/min) à t⁰ | 250 | 350 | 200 |
| Boeing test | 0 | 0 | 0 |
| Elongation à la rupture Haltère (%) | 500 | 500 | 400 |
| Module 100% Haltère (MPa) | 0,55 | 0,58 | 0,54 |
| Module max. (MPa) | 0,80 | 1,30 | 1,00 |
| Stabilité à 7 jours | OK | OK | OK |
| Extrusion à t⁰ + 3 semaines à 40°C (g/min) | 210 | 300 | 180 |
| Bullage | Oui | Non | Non |

### Caractérisation :

- Le temps de formation de peau (« skinning time » en anglais) a été mesuré selon la norme ISO 291 à 23°C et 50% d'humidité relative,
- L'extrusion correspond à la quantité en gramme de mastic qui a pu être extrudée par minute, sous une pression du piston de 3 bars, à 23°C,
- Le module 100% Haltère a été mesuré selon la norme NF ISO 37 (mars 2012) avec des éprouvettes haltères,
- Le module max. a été mesuré selon la norme ISO 8339,
- L'élongation à la rupture Haltère a été mesurée selon la norme NF ISO 37 (mars 2012) avec des éprouvettes haltères,
- La résistance au fluage a été vérifiée selon norme ASTM D2202 dite du « Boeing Test ».
- L'extrusion des compositions de mastic est réalisée au travers d'une buse d'extrusion de 4 mm de diamètre sous une pression de 3 bar à 23°C et hygrométrie constante à la fin du mélange (t°) et 3 semaines à 40°C après la fin du mélange (t° + 3 semaines) afin d'évaluer leur viscosité.

## Revendications

1. Composé ayant l'une des formules (I) ou (II) suivantes : 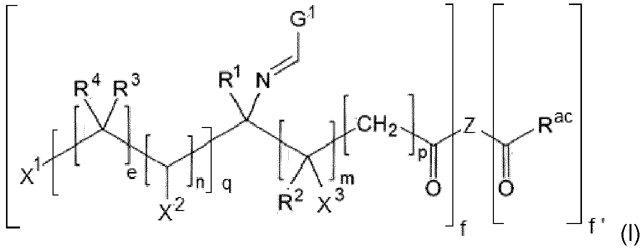 dans laquelle:
- X¹ représente F¹ ou -N=C(H)-G¹ ou -Ph-XH, ou -XH, avec X représentant O ou S ou Se et Ph représentant un groupe phényle;
- X² représente F² ou -N=C(H)-G¹ ou -Ph-XH, ou -XH, avec X représentant O ou S ou Se et Ph représentant un groupe phényle;
- X³ représente F³ ou -N=C(H)-G¹ ou -Ph-XH, ou -XH, avec X représentant O ou S ou Se et Ph représentant un groupe phényle;
à condition qu'au maximum un seul radical parmi les radicaux X¹, X² et X³ représente -N=C(H)-G¹ ;
à condition qu'au maximum un seul radical parmi les radicaux X¹, X² et X³ représente -XH ou -Ph-XH;
à condition que quand X¹ = -N=C(H)-G¹ alors q = 1 ;
à condition que quand X¹ = -N=C(H)-G¹ ou -Ph-XH ou -XH, alors X² = F² et X³ = F³ ;
à condition que quand X² = -N=C(H)-G¹ ou -Ph-XH ou -XH, alors X¹ = F¹ et X³ = F³ ;
à condition que quand X³ = -N=C(H)-G¹ ou -Ph-XH ou -XH, alors X² = F² et X¹ = F¹ ;
- F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- R¹, R², R³ et R⁴ représente chacun, indépendamment les uns des autres, un radical choisi parmi un atome d'hydrogène, un groupe alkyle linéaire ou ramifié, comprenant de 1 à 20 atomes de carbone, ledit groupe alkyle comprenant éventuellement un ou plusieurs hétéroatomes, un groupe (hétéro)cycloalkyle comprenant de 3 à 20 atomes de carbone, un groupe arylalkyle comprenant de 7 à 20 atomes de carbone ou un groupe (hétéro)aryle comprenant de 4 à 12 atomes de carbone ;
- n est un nombre entier allant de 0 à 28 ;
- p est un nombre entier allant de 0 à 10 ;
- m représente 0 ou 1 ;
- q représente 0 ou 1 ;
- e est un nombre entier allant de 1 à 3 ;
- f est un nombre entier ou non entier allant de 1 à 3, f étant de préférence un nombre entier égal à 1, 2 ou 3 ;
- f' est un nombre entier ou non entier allant de 0 à 2, f' étant de préférence un nombre entier égal à 0, 1 ou 2 ;
- la somme f + f' représente un entier variant de 1 à 3 ;
- Z représente un radical organique monovalent Z^{m}, divalent Z^{d} ou trivalent Z^{t}, ayant une masse molaire ou une masse moléculaire moyenne en nombre (Mn) allant de 16 à 22 000 g/mol, de préférence de 16 à 12 000 g/mol, de préférence encore de 16 à 8 000 g/mol, encore plus préférentiellement de 16 à 4 000 g/mol,
- R^{ac} représente un atome d'hydrogène ou un radical hydrocarboné monovalent comprenant de 1 à 60 atomes de carbone, ledit radical pouvant éventuellement comprendre un ou plusieurs hétéroatomes ;
- G¹ représente un radical hydrocarboné monovalent de masse molaire ou de masse moléculaire moyenne en nombre (Mn) allant de 15 à 4 000 g/mol, de préférence de 60 à 2 000 g/mol, préférentiellement de 60 à 1 000 g/mol et encore plus préférentiellement de 60 à 500 g/mol, ledit radical pouvant éventuellement comprendre un ou plusieurs hétéroatomes, à condition que G¹ ne représente pas un radical aryle substitué ou non ; 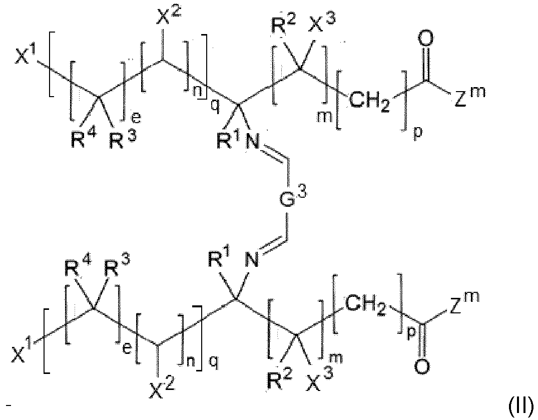
dans laquelle :
- X¹ représente F¹ ou -Ph-XH, ou -XH;
- X² représente F² ou -Ph-XH, ou -XH;
- X³ représente F³ ou -Ph-XH, ou -XH;
- F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- X, R¹, R², R³, R⁴, n, q, m, p, e et Z^{m} étant tels que définis pour la formule (I) ci-dessus ;
- G³ représente un radical hydrocarboné monovalent de masse molaire ou de masse moléculaire moyenne en nombre (Mn) allant de 14 à 4 000 g/mol, de préférence de 42 à 2 000 g/mol, préférentiellement de 42 à 1 000 g/mol et encore plus préférentiellement de 42 à 500 g/mol, ledit radical pouvant éventuellement comprendre un ou plusieurs hétéroatomes, à condition que G³ ne représente pas un radical arylène substitué ou non ;
à condition qu'au maximum un seul radical parmi les radicaux X¹, X² et X³ représente -XH ou -Ph-XH;
à condition que quand X¹ = -Ph-XH ou -XH, alors X² = F² et X³ = F³ ;
à condition que quand X² = -Ph-XH ou -XH, alors X¹ = F¹ et X³ = F³ ;
à condition que quand X³ = -Ph-XH ou -XH, alors X² = F² et X¹ = F¹.

2. Composé selon la revendication 1, **caractérisé en ce que** Z^{m} représente un radical choisi parmi -OR⁵, -NH₂, -NH-R' ou -N(R')(R") dans lesquels :
- R⁵ représente un groupe alkyle linéaire ou ramifié comprenant de 1 à 60 atomes de carbone pouvant éventuellement comprendre un ou plusieurs hétéroatomes, un groupe (hétéro)cycloalkyle comprenant de 3 à 20 atomes de carbone, un groupe arylalkyle comprenant de 7 à 20 atomes de carbone, ou un groupe (hétéro)aryle comprenant de 4 à 12 atomes de carbone;
- R' représente un groupe alkyle linéaire ou ramifié, comprenant de 1 à 60 atomes de carbone pouvant éventuellement comprendre un ou plusieurs hétéroatomes, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone ;
- R" représente un groupe alkyle linéaire ou ramifié, comprenant de 1 à 60 atomes de carbone pouvant éventuellement comprendre un ou plusieurs hétéroatomes, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone.

3. Composé selon l'une quelconque des revendications 1 ou 2, **caractérisé en ce que** radical -G¹ représente un radical -C(R⁶)(R⁷)(R⁸) ou un radical -G² avec:
- R⁶ et R⁷ représentant chacun, indépendamment l'un de l'autre, un radical hydrocarboné monovalent comprenant de 1 à 12 atomes de carbone, ledit radical étant éventuellement substitué par un groupe -OH ;
ou R⁶ et R⁷ forment ensemble un cycle aliphatique comprenant de 4 à 12 atomes de carbone, ledit cycle étant éventuellement substitué ;
- R⁸ représente un radical hydrocarboné monovalent comprenant de 1 à 60 atomes de carbone, ledit radical comprenant éventuellement un hétéroatome ;
- G² représente un radical hétéroaryle éventuellement substitué, ou un radical -C(O)-R¹² avec R¹² représentant un radical alcoxy, un radical alkényle, ou un radical arylalkényle comprenant au moins 6 atomes de carbone,
ledit radical G² ayant une masse molaire ou une masse moléculaire moyenne en nombre (Mn) allant de 15 à 4 000 g/mol, de préférence allant de 60 à 2 000 g/mol, préférentiellement allant de 60 à 1 000 g/mol et encore plus préférentiellement allant de 60 à 500 g/mol.

4. Composé selon l'une quelconque des revendications 1 ou 2, **caractérisé en ce que** dans la formule (II), le radical -G³ représente :
- un radical alkylène linéaire ou ramifié, cyclique ou non, saturé ou insaturé, ou
- un radical -G⁵-(G⁶-G⁵)ᵣ-,
de masse molaire ou de masse moléculaire moyenne en nombre (Mn) allant de 14 à 4 000 g/mol, de préférence allant de 60 à 2 000 g/mol, préférentiellement allant de 60 à 1 000 g/mol et encore plus préférentiellement allant de 60 à 500 g/mol, dans lequel :
- G⁵ représente un radical (hétéro)arylène éventuellement substitué ;
- G⁶ représente un atome d'oxygène, un atome de soufre ou un radical choisi parmi l'un des radicaux suivants : -O-R²⁷-O-, -CH₂-O-R²⁸-O-CH₂-, -CH₂-O-C(=O)-R²⁹-C(=O)-O-CH₂-, -CH₂-O-C(=O)-NH-R³⁰-NH-C(=O)-O-CH₂-, -O-C(=O)-NH-R³¹-NH-C(=O)-O-, -O-C(=O)-R³²-C(=O)-O-, et avec R²⁷, R²⁸, R²⁹, R³⁰, R³¹ et R³² représentant indépendamment les uns des autres un radical hydrocarboné comprenant éventuellement au moins un hétéroatome ;
- r représente 0 ou 1 ;
- à condition que quand r = 0, alors G⁵ représente un hétéroarylène éventuellement substitué ;
- un radical -CH(R²⁵)(R²⁶)-[G⁴-CH(R²⁵)(R²⁶)]_{w}-, de masse molaire ou de masse moléculaire moyenne en nombre (Mn) allant de 42 à 4 000 g/mol, de préférence allant de 42 à 2 000 g/mol, préférentiellement allant de 42 à 1 000 g/mol et encore plus préférentiellement allant de 42 à 500 g/mol, dans lequel:
- R²⁵ et R²⁶ représente chacun, indépendamment l'un de l'autre, un radical hydrocarboné monovalent comprenant de 1 à 12 atomes de carbone, ledit radical étant éventuellement substitué par un groupe -OH ;
ou R²⁵ et R²⁶ forment ensemble un cycle aliphatique comprenant de 4 à 12 atomes de carbone, ledit cycle étant éventuellement substitué ;
- G⁴ représente une liaison carbone-carbone ou un radical hydrocarboné divalent, ledit radical comprenant éventuellement au moins un hétéroatome ;
- w représente un nombre entier égal à 0 ou 1.

5. Composé selon l'une quelconque des revendications 1 à 4, **caractérisé en ce que** dans les formules (I) et (II) :
- n représente 0, 1, 2, 3, 4, 9 ou 28 ;
- m représente 0 ou 1 ;
- p représente 0 ou 1 ou 9 ou 10;
- q représente 0 ou 1 ;
- F¹ représente un radical choisi parmi un atome d'hydrogène, un groupe (hétéro)aryle comprenant de 4 à 12 atomes de carbone, un groupe -COOZ (formule (I) avec Z étant tel que défini précédemment dans la formules (I), ou respectivement -COOZ^{m} avec Z^{m} étant tel que défini dans la formule (II)), un groupe -C(O)NH₂, un groupe -SMe, ou un groupe guanidyl de formule -NH-C(=NH)-NH₂,
- F² représente un radical choisi parmi un atome d'hydrogène ou un groupe alkyle linéaire ou ramifié comprenant de 1 à 10 atomes de carbone,
- F³ représente un radical choisi parmi un atome d'hydrogène ou un groupe alkyle linéaire ou ramifié comprenant de 1 à 10 atomes de carbone ;
- R¹, R², R³ et R⁴ représente chacun, indépendamment les uns des autres, un atome d'hydrogène ou un groupe alkyle linéaire ou ramifié comprenant de 1 à 10 atomes de carbone, encore plus préférentiellement de 1 à 5 atomes de carbone, ou un groupe arylalkyle comprenant de 7 à 20 atomes de carbone, ou un groupe aryle comprenant de 6 à 12 atomes de carbone.

6. Composé selon l'une quelconque des revendications 1 à 5, **caractérisé en ce que** dans les formules (I) et (II) :
- n représente 0, 1, 2, 3 ou 4 ;
- m représente 0 ou 1 ;
- p représente 0 ou 1;
- q représente 0 ou 1 ;
- F¹ représente un radical choisi parmi un atome d'hydrogène, un groupe hétéroaryle comprenant de 4 à 12 atomes de carbone, un groupe -COOZ (Z étant tel que défini précédemment dans la formule (I)), un groupe -C(O)NH₂, un groupe -SMe, ou un groupe guanidyl de formule -NH-C(=NH)-NH₂,
- F² représente un radical choisi parmi un atome d'hydrogène ou un groupe alkyle linéaire ou ramifié comprenant de 1 à 5 atomes de carbone,
- F³ représente un radical choisi parmi un atome d'hydrogène;
- R¹, R², R³ et R⁴ représente chacun, indépendamment les uns des autres, un atome d'hydrogène ou un groupe alkyle linéaire ou ramifié comprenant de 1 à 5 atomes de carbone, ou un groupe benzyle, ou un groupe phényle.

7. Composé selon l'une quelconque des revendications 1 à 6, **caractérisé en ce qu'**il est choisi parmi les composés de formules (I-A), (I-B), (I-C) ou (I-D) suivantes : 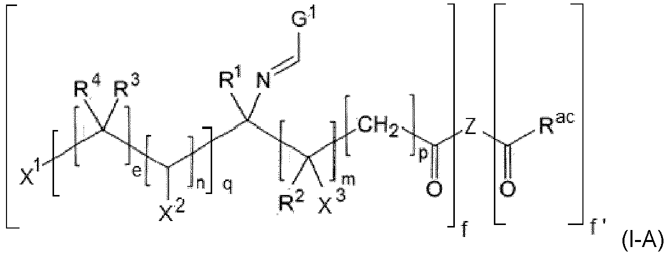 dans laquelle :
- R¹, R², R³, R⁴, n, m, p, q, e, f, f', Z, G¹ et R^{ac} sont tels que définis dans la formule (I), dans l'une quelconque des revendications 1 à 6,
- X¹ représente F¹ tel que défini dans la formule (I) dans l'une quelconque des revendications 1 à 6, ou -Ph-XH, ou -XH avec X représentant O ou S ou Se et Ph représentant un groupe phényle;
- X² représente F² tel que défini dans la formule (I) dans l'une quelconque des revendications 1 à 6, ou -Ph-XH, ou -XH avec X représentant O ou S ou Se et Ph représentant un groupe phényle;
- X³ représente F³ tel que défini dans la formule (I) dans l'une quelconque des revendications 1 à 6, ou -Ph-XH, ou -XH avec X représentant O ou S ou Se et Ph représentant un groupe phényle;
à condition qu'au plus un des radicaux X¹, X² ou X³ représente un radical -Ph-XH ou -XH avec X représentant O ou S ou Se et Ph représentant un radical phényle ; 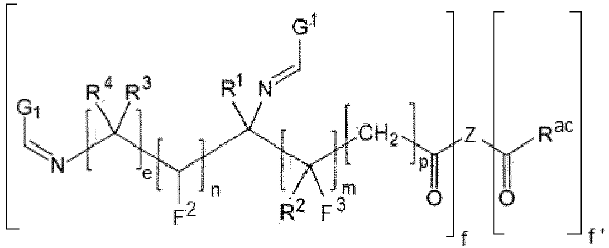 (I-B) dans laquelle F², F³, R¹, R², R³, R⁴, n, m, p, e, f, f', Z, G¹ et R^{ac} sont tels que définis dans la formule (I) dans l'une quelconque des revendications 1 à 6 ; 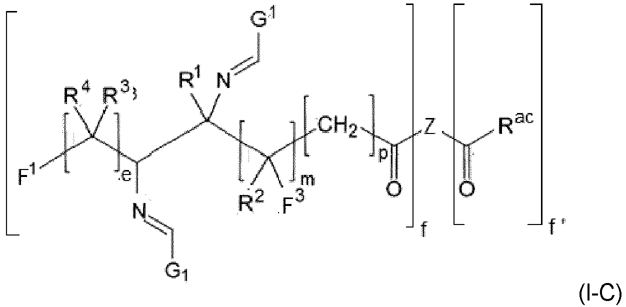 dans laquelle F¹, F³, R¹, R², R³, R⁴, F², m, p, q, e, f, f', G¹, Z et R^{ac} sont tels que définis dans la formule (I) dans l'une quelconque des revendications 1 à 6 ; 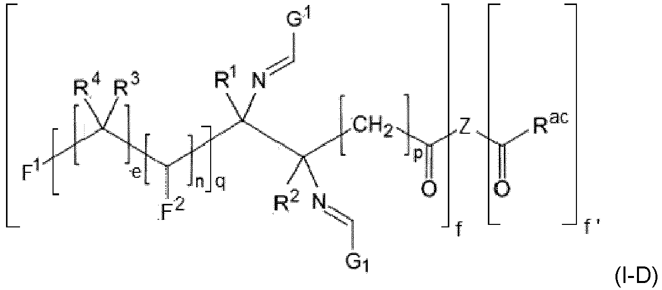 dans laquelle F¹, F², R¹, R², R³, R⁴, F², n, m, p, q, e, f, f', G¹, Z et R^{ac} sont tels que définis dans la formule (I) dans l'une quelconque des revendications 1 à 6.

8. Composé selon la revendication 7, **caractérisé en ce que** les composés de formule (I-A) ont l'une des formules (I-A-1), (I-A-2) ou (I-A-3) suivantes : 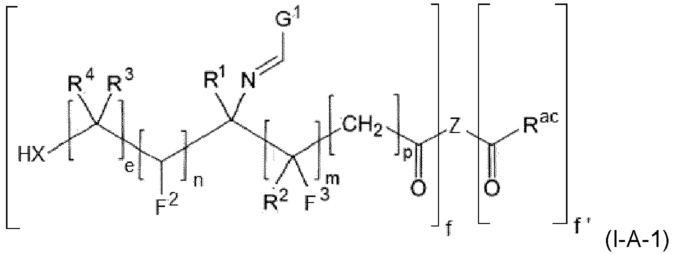 dans laquelle X, R¹, R², R³, R⁴, n, m, p, e, f, f', Z, F², F³, G¹ et R^{ac} sont tels que définis dans la formule (I-A) dans la revendication 7, 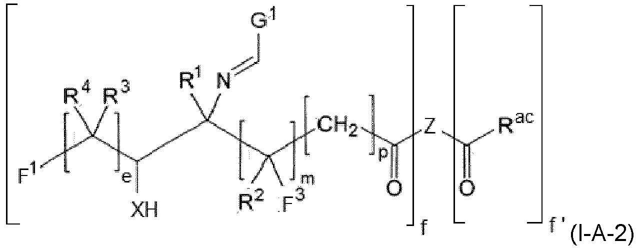 dans laquelle R¹, R², R³, R⁴, m, p, e, f, f', Z, X, G¹, F¹, F³, G¹ et R^{ac} sont tels que définis dans la formule (I-A) dans la revendication 7, 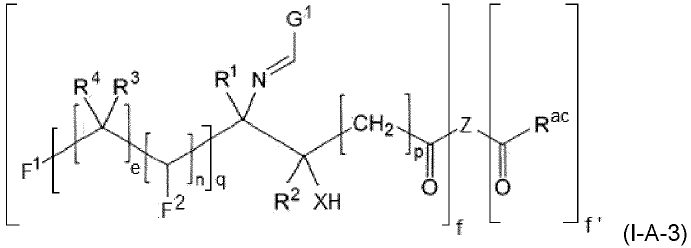 dans laquelle R¹, R², R³, R⁴, n, p, q, e, f, f', Z, X, G¹, F¹, F², R^{ac} sont tels que définis dans la formule (I-A) dans la revendication 7.

9. Composés selon la revendication 7, **caractérisé en ce que** les composés de formule (I-B) ont l'une des formules (I-B-1), (I-B-2) ou (I-B-3) suivantes : 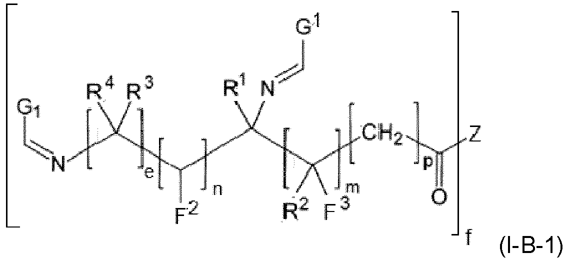 dans laquelle F², F³, R¹, R², R³, R⁴, n, m, p, e, f, G¹ et Z sont tels que définis dans la formule (I-B) dans la revendication 7, 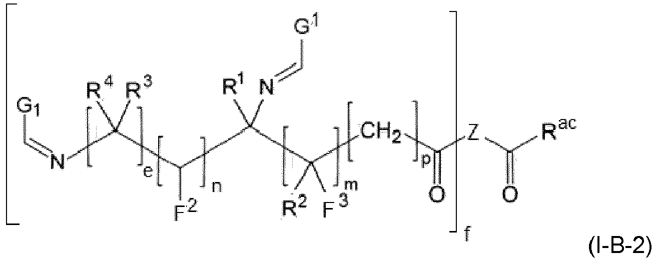 dans laquelle F², F³, R¹, R², R³, R⁴, n, m, p, e, f, G¹, Z et R^{ac} sont tels que définis dans la formule (I-B) dans la revendication 7, 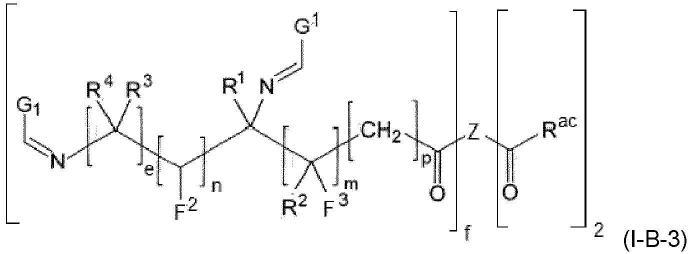 dans laquelle F², F³, R¹, R², R³, R⁴, n, m, p, e, f, G¹, Z et R^{ac} sont tels que définis dans la formule (I-B) dans la revendication 7.

10. Composé selon la revendication 7, **caractérisé en ce que** les composés de formule (I-C) ont l'une des formules (I-C-1), (I-C-2) ou (I-C-3) suivantes : 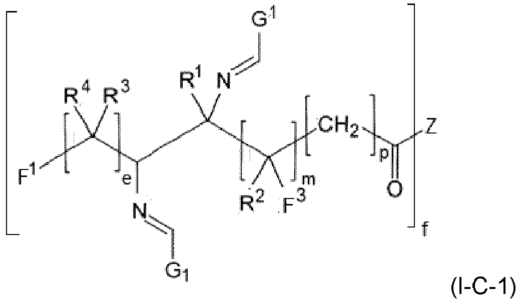 dans laquelle F¹, F³, R¹, R², R³, R⁴, F², m, p, q, e, f, G¹ et Z sont tels que définis dans la formule (I-C) dans la revendication 7, 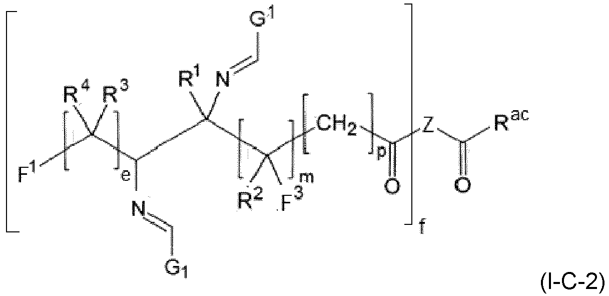 dans laquelle F¹, F³, R¹, R², R³, R⁴, F², m, p, q, e, f, G¹, Z et R^{ac} sont tels que définis dans la formule (I-C) dans la revendication 7, 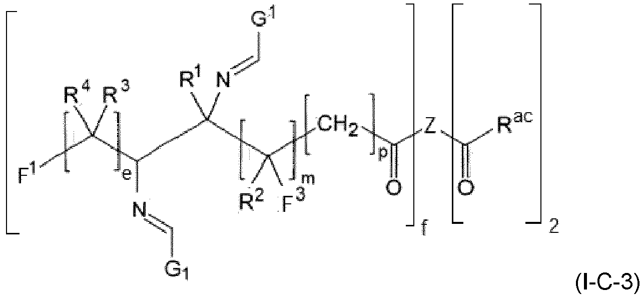 dans laquelle F¹, F³, R¹, R², R³, R⁴, F², m, p, q, e, f, G¹, Z et R^{ac} sont tels que définis dans la formule (I-C) dans la revendication 7.

11. Composé selon la revendication 7, **caractérisé en ce que** les composés de formule (I-D) ont l'une des formules (I-D-1), (I-D-2) ou (I-D-3) suivantes : 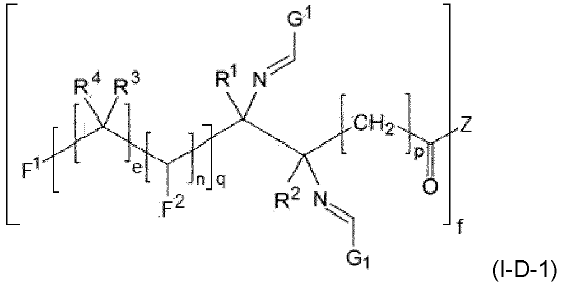 dans laquelle F¹, F², R¹, R², R³, R⁴, F², n, m, p, q, e, f, G¹ et Z sont tels que définis dans la formule (I-D) dans la revendication 7, 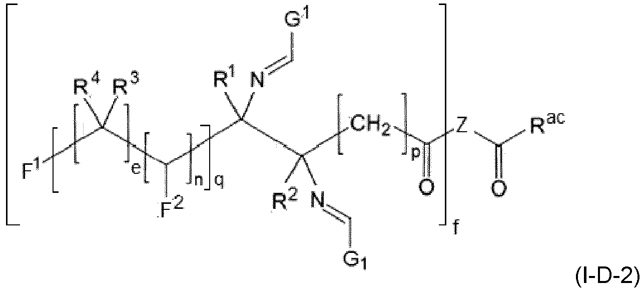 dans laquelle F¹, F², R¹, R², R³, R⁴, F², n, m, p, q, e, f, G¹, Z et R^{ac} sont tels que définis dans la formule (I-D) dans la revendication 7, 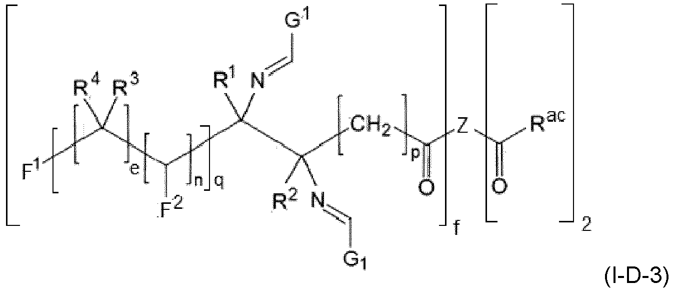 dans laquelle F¹, F², R¹, R², R³, R⁴, F², n, m, p, q, e, f, G¹, Z et R^{ac} sont tels que définis dans la formule (I-D) dans la revendication 7.

12. Composé de formule (I) selon l'une quelconque des revendications 1 à 11, **caractérisé en ce qu'**il est obtenu par réaction entre :
- un composé de formule (III) suivante : 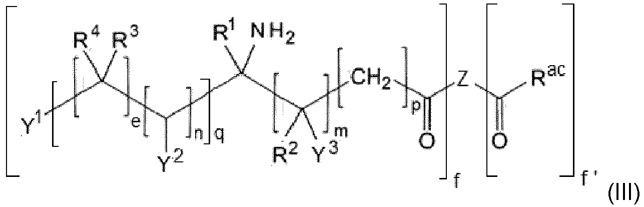 dans laquelle :
- Y¹ représente F¹ ou -NH₂ ou -Ph-XH ou -XH avec X représentant O ou S ou Se et Ph représentant un groupe phényle;
- Y² représente F² ou -NH₂ ou -Ph-XH ou -XH avec X représentant O ou S ou Se et Ph représentant un groupe phényle;
- Y³ représente F³ ou -NH₂ ou -Ph-XH ou -XH avec X représentant O ou S ou Se et Ph représentant un groupe phényle;
à condition qu'au maximum un seul radical parmi les radicaux Y¹, Y² et Y³ représente -NH₂,
à condition qu'au maximum un seul radical parmi les radicaux Y¹, Y² et Y³ représente -Ph-XH ou -XH ;
à condition que quand Y¹ = -NH₂ alors q = 1 ;
à condition que quand Y¹ = -NH₂ ou -XH ou -Ph-XH, alors Y² = F² et Y³ = F³ ;
à condition que quand Y² = -NH₂ ou -XH ou -Ph-XH, alors Y¹ = F¹ et Y₃ = F³ ;
à condition que quand Y³ = -NH₂ ou -XH ou -Ph-XH, alors Y² = F² et Y¹ = F¹ ;
- F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- R¹, R², R³ et R⁴ représente chacun, indépendamment les uns des autres, un radical choisi parmi un atome d'hydrogène, un groupe alkyle linéaire ou ramifié, comprenant de 1 à 20 atomes de carbone, ledit groupe alkyle comprenant éventuellement un ou plusieurs hétéroatomes, un groupe (hétéro)cycloalkyle comprenant de 3 à 20 atomes de carbone, un groupe arylalkyle comprenant de 7 à 20 atomes de carbone ou un groupe (hétéro)aryle comprenant de 4 à 12 atomes de carbone ;
- n est un nombre entier allant de 0 à 28 ;
- p est un nombre entier allant de 0 à 10 ;
- m représente 0 ou 1 ;
- q représente 0 ou 1 ;
- e est un nombre entier allant de 1 à 3 ;
- f est un nombre entier ou non entier allant de 1 à 3, f étant de préférence un nombre entier égal à 1, 2 ou 3 ;
- f' est un nombre entier ou non entier allant de 0 à 2, f' étant de préférence un nombre entier égal à 0, 1 ou 2 ;
- la somme f + f' représente un entier variant de 1 à 3 ;
- Z représente un radical organique monovalent Z^{m}, divalent Z^{d} ou trivalent Z^{t}, ayant une masse moléculaire moyenne en nombre (Mn) allant de 16 à 22 000 g/mol,
- R^{ac} représente un atome d'hydrogène ou un radical hydrocarboné monovalent comprenant de 1 à 60 atomes de carbone, ledit radical pouvant éventuellement comprendre un ou plusieurs hétéroatomes;
- et un composé de formule (IV) suivante : 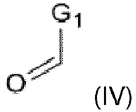 dans laquelle G¹ est tel que défini dans la formule (I) dans l'une quelconque des revendications 1 à 11.

13. Composés de formule (II) selon l'une quelconque des revendications 1 à 12, **caractérisés en ce qu'**il sont obtenus par réaction entre :
- un composé de formule (III') suivante : 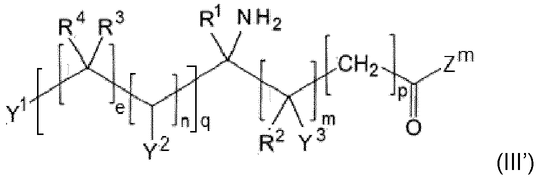 dans laquelle :
- Y¹ représente F¹ ou -Ph-XH ou -XH avec X représentant O ou S ou Se et Ph représentant un groupe phényle;
- Y² représente F² ou -Ph-XH ou -XH avec X représentant O ou S ou Se et Ph représentant un groupe phényle;
- Y³ représente F³ ou -Ph-XH ou -XH avec X représentant O ou S ou Se et Ph représentant un groupe phényle;
à condition qu'au maximum un seul radical parmi les radicaux Y¹, Y² et Y³ représente -Ph-XH ou -XH ;
à condition que quand Y¹ = -XH ou -Ph-XH, alors Y² = F² et Y³ = F³ ;
à condition que quand Y² = -XH ou -Ph-XH, alors Y¹ = F¹ et Y³ = F³ ;
à condition que quand Y³ = -XH ou -Ph-XH, alors Y¹ = F¹ et Y² = F² ;
- F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- R¹, R², R³ et R⁴ représente chacun, indépendamment les uns des autres, un radical choisi parmi un atome d'hydrogène, un groupe alkyle linéaire ou ramifié, comprenant de 1 à 20 atomes de carbone, ledit groupe alkyle comprenant éventuellement un ou plusieurs hétéroatomes, un groupe (hétéro)cycloalkyle comprenant de 3 à 20 atomes de carbone, un groupe arylalkyle comprenant de 7 à 20 atomes de carbone ou un groupe (hétéro)aryle comprenant de 5 à 12 atomes de carbone ;
- n est un nombre entier allant de 0 à 28 ;
- p est un nombre entier allant de 0 à 10 ;
- m représente 0 ou 1 ;
- q représente 0 ou 1 ;
- e est un nombre entier allant de 1 à 3 ;
- Z^{m} représente un radical organique monovalent ayant une masse moléculaire moyenne en nombre (Mn) allant de 16 à 22 000 g/mol;
- et un composé de formule (VIII) suivante : 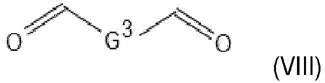 dans laquelle G³ est tel que défini dans la formule (II) ci-dessus dans la revendication 1.

14. Utilisation des composés de formule (X) ou (XI) comme absorbeur d'humidité (« water-scavenger ») notamment dans une composition à base de polyuréthane, ou comme agent durcisseur dans une composition à base de résine époxy : 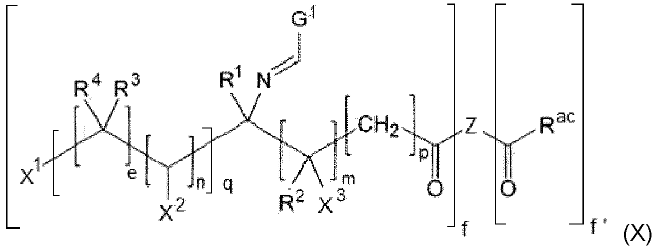 dans laquelle:
- X¹ représente F¹ ou -N=C(H)-G¹ ou -Ph-XH, ou -XH, avec X représentant O ou S ou Se et Ph représentant un groupe phényle;
- X² représente F² ou -N=C(H)-G¹ ou -Ph-XH, ou -XH, avec X représentant O ou S ou Se et Ph représentant un groupe phényle;
- X³ représente F³ ou -N=C(H)-G¹ ou -Ph-XH, ou -XH, avec X représentant O ou S ou Se et Ph représentant un groupe phényle;
à condition qu'au maximum un seul radical parmi les radicaux X¹, X² et X³ représente -N=C(H)-G¹ ;
à condition qu'au maximum un seul radical parmi les radicaux X¹, X² et X³ représente -XH ou -Ph-XH;
à condition que quand X¹ = -N=C(H)-G¹ alors q = 1 ;
à condition que quand X¹ = -N=C(H)-G¹ ou -Ph-XH ou -XH, alors X² = F² et X³ = F³ ;
à condition que quand X² = -N=C(H)-G¹ ou -Ph-XH ou -XH, alors X¹ = F¹ et X³ = F³ ;
à condition que quand X³ = -N=C(H)-G¹ ou -Ph-XH ou -XH, alors X² = F² et X¹ = F¹ ;
- F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ, un groupe -C(O)NH₂, un groupe -SMe, ou un groupe guanidyl de formule -NH-C(=NH)-NH₂,
- R¹, R², R³ et R⁴ représente chacun, indépendamment les uns des autres, un radical choisi parmi un atome d'hydrogène, un groupe alkyle linéaire ou ramifié, comprenant de 1 à 20 atomes de carbone, ledit groupe alkyle comprenant éventuellement un ou plusieurs hétéroatomes, un groupe (hétéro)cycloalkyle comprenant de 3 à 20 atomes de carbone, un groupe arylalkyle comprenant de 7 à 20 atomes de carbone ou un groupe (hétéro)aryle comprenant de 4 à 12 atomes de carbone ;
- n est un nombre entier allant de 0 à 28 ;
- p est un nombre entier allant de 0 à 10 ;
- m représente 0 ou 1 ;
- q représente 0 ou 1 ;
- e est un nombre entier allant de 1 à 3 ;
- f est un nombre entier ou non entier allant de 1 à 3, f étant de préférence un nombre entier égal à 1, 2 ou 3 ;
- f' est un nombre entier ou non entier allant de 0 à 2, f' étant de préférence un nombre entier égal à 0, 1 ou 2 ;
- la somme f + f' représente un entier variant de 1 à 3 ;
- Z représente un radical organique monovalent Z^{m}, divalent Z^{d} ou trivalent Z^{t}, ayant une masse molaire ou une masse moléculaire moyenne en nombre (Mn) allant de 16 à 22 000 g/mol, de préférence de 16 à 12 000 g/mol, de préférence encore de 16 à 8 000 g/mol, encore plus préférentiellement de 16 à 4 000 g/mol,
- R^{ac} représente un atome d'hydrogène ou un radical hydrocarboné monovalent comprenant de 1 à 60 atomes de carbone, ledit radical pouvant éventuellement comprendre un ou plusieurs hétéroatomes ;
- G¹ représente un radical hydrocarboné monovalent de masse molaire ou de masse moléculaire moyenne en nombre (Mn) allant de 15 à 4000 g/mol, de préférence de 60 à 2 000 g/mol, préférentiellement de 60 à 1 000 g/mol et encore plus préférentiellement de 60 à 500 g/mol, ledit radical pouvant éventuellement comprendre un ou plusieurs hétéroatomes; 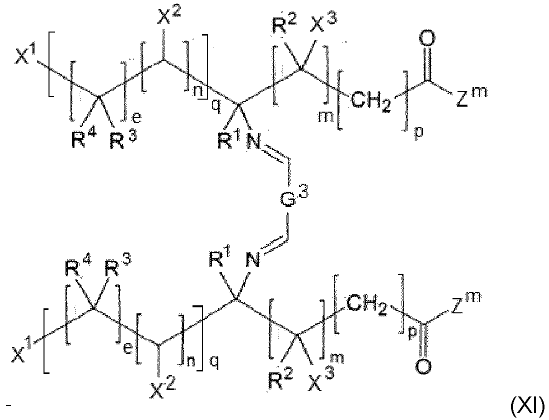
dans laquelle :
- X¹ représente F¹ ou -Ph-XH, ou -XH;
- X² représente F² ou -Ph-XH, ou -XH;
- X³ représente F³ ou -Ph-XH, ou -XH;
- F¹ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- F² représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- F³ représente un radical choisi parmi un atome d'hydrogène, un groupe alkyle linéaire ou ramifié comprenant de 1 à 20 atomes de carbone, un groupe arylalkyle comprenant de 7 à 20 atomes de carbone, un groupe (hétéro)aryle comprenant de 4 à 12 atomes de carbone, ou un groupe (hétéro)cycloalkyle comprenant de 3 à 20 atomes de carbone, un groupe - COOZ^{m}, un groupe -C(O)NH₂, un groupe -SMe, ou un groupe guanidyl de formule -NH-C(=NH)-NH₂,
- X, R¹, R², R³, R⁴, n, q, m, p, e et Z^{m} étant tels que définis pour la formule (I) ci-dessus ;
- G³ représente un radical hydrocarboné monovalent de masse molaire ou de masse moléculaire moyenne en nombre (Mn) allant de 14 à 4 000 g/mol, de préférence de 42 à 2 000 g/mol, préférentiellement de 42 à 1 000 g/mol et encore plus préférentiellement de 42 à 500 g/mol, ledit radical pouvant éventuellement comprendre un ou plusieurs hétéroatomes; à condition qu'au maximum un seul radical parmi les radicaux X¹, X² et X³ représente -XH ou -Ph-XH;
à condition que quand X¹ = -Ph-XH ou -XH, alors X² = F² et X³ = F³ ;
à condition que quand X² = -Ph-XH ou -XH, alors X¹ = F¹ et X³ = F³ ;
à condition que quand X³ = -Ph-XH ou -XH, alors X² = F² et X¹ = F¹.

15. Composition C1 réticulable à l'humidité comprenant :
- au moins un composé de formule (X) ou (XI) telles que définies selon la revendication 14 ; et
- au moins un polymère P comprenant au moins deux fonctions terminales NCO ou au moins une résine époxy.

16. Composition C1 selon la revendication 15 **caractérisée en ce qu'**elle est une composition d'adhésif, de mastic ou de revêtement.
